# EUROPEAN PATENT APPLICATION

(11) **EP 4 303 584 A2**
(43) Date of publication of application: **10.01.2024**
(21) Application number: 23188166.5
(22) Date of filing: 22.07.2011
(51) Int. Cl.: G01N 33/53

(54) **METHODS FOR DETECTING SIGNATURES OF DISEASE OR CONDITIONS IN BODILY FLUIDS**

(30) Priority: 23.07.2010 US 36700610 P
(62) Divisional of application: 11810446.2
(71) Applicant: President and Fellows of Harvard College, Cambridge, MA 02138 (US)
(72) Inventor: KASSIS, Amin I., Chestnut Hill, MA 02467 (US)
(74) Representative: Regimbeau

(57) **Abstract**

This invention provides methods of using cell free bodily fluid and blood cells in the diagnosis, prognosis, or monitoring of diseases or conditions. The invention also relates to methods of using cell free bodily fluid and blood cells to identify markers of diseases or conditions.

## Description

### Field of the Invention

This invention relates generally to methods of using cell free bodily fluid and blood cells in the diagnosis, prognosis, or monitoring of diseases or conditions. The invention also relates to methods of using cell free bodily fluid and blood cells to identify markers of diseases or conditions.

### Background of the Invention

Early diagnosis of a disease often increases the likelihood of successful treatment or cure of such disease. Current diagnostic methods, however, depend largely on population-derived average values obtained from healthy individuals. Personalized diagnostic methods are needed that enable the diagnosis, especially the early diagnosis, of the presence of a disease or a condition in individuals who are not known to have the disease or who have recurrent disease.

Leukocytes begin as pluripotent hematopoietic stem cells in the bone marrow and develop along either the myeloid lineage (monocytes, macrophages, neutrophils, eosinophils, and basophils) or the lymphoid lineage (T and B lymphocytes and natural killer cells). The major function of the myeloid lineage cells (e.g., neutrophils and macrophages) is the phagocytosis of infectious organisms, live unwanted damaged cells, senescent and dead cells (apoptotic and necrotic), as well as the clearing of cellular debris. Phagocytes from healthy animals do not replicate and are diploid, i.e., have a DNA content of 2n. On average, each cell contains <10 ng DNA, <20 ng RNA, and <300 ng of protein. Non-phagocytic cells are also diploid and are not involved in the internalization of dead cells or infectious organisms and have a DNA index of one.

The lifetime of various white blood cell subpopulations varies from a few days (e.g., neutrophils) to several months (e.g., macrophages). Like other cell types, leukocytes age and eventually die. During their aging process, human blood- and tissue-derived phagocytes (e.g., neutrophils) exhibit all the classic markers of programmed cell death (i.e., apoptosis), including caspase activation, pyknotic nuclei, and chromatin fragmentation. These cells also display a number of "eat-me" flags (e.g., phosphatidylserine, sugars) on the extracellular surfaces of their plasma membranes. Consequently, dying and dead cells and subcellular fragments thereof are cleared from tissues and blood by other phagocytic cells.

One object of the present invention is to provide diagnostic methods that can facilitate the detection of a disease or condition-specific markers, e.g., nucleic acids, proteins, carbohydrates, and/or lipids and the like by using cell-free bodily fluids and blood cells. Another object of this invention is to provide methods of identifying a disease or condition-specific markers and further use such markers alone or together with any known markers to diagnose diseases or conditions.

### Summary of the Invention

We have invented new and useful methods for detecting/diagnosing diseases or conditions by using cell-free bodily fluid in combination with non-phagocytic cells or phagocytic cells having a DNA content of2n ( =2n phagocytic cells). In some embodiments, cell-free bodily fluids contain components of diseased cells, such as DNA and/or protein, and serve as surrogates for diseased cells, while non-phagocytic cells or. In other embodiments, cell-free bodily fluids serve as surrogates for diseased cell, while =2n phagocytic cells can serve as control cells.

In one aspect, this invention provides a method for diagnosing or aiding in the diagnosis of a disease or condition in a subject comprising: a) determining a first profile of one or more markers of the disease or condition from a cell-free bodily fluid sample; b) determining a second profile of at least one of the one or more markers from a population of non-phagocytic cells; and c) identifying a difference between the first and second profiles of at least one or more of said markers, wherein the difference is indicative of the presence of said disease or condition in the subject.

In another aspect, this invention provides a method for assessing the risk of developing a disease or condition in a subject comprising: a) determining a first profile of one or more markers of the disease or condition from a cell-free bodily fluid sample; b) determining a second profile of at least one of the one or more markers from a population of non-phagocytic cells; and c) identifying a difference between the first and second profiles of at least one or more of said markers, wherein the difference is indicative of the risk of developing said disease or condition in the subj ect.

In yet another aspect, this invention provides a method for prognosing or aiding in the prognosis of a disease or condition in a subject comprising: a) determining a first profile of one or more markers of the disease or condition from a cell-free bodily fluid sample; b) determining a second profile of at least one of the one or more markers from a population of non-phagocytic cells; and c) identifying a difference between the first and second profiles of at least one or more of said markers, wherein the identified difference is indicative of the prognosis of said disease or condition in the subject.

In yet another aspect, this invention provides a method for assessing the efficacy of a treatment for a disease or condition in a subject comprising: a) determining a first profile of one or more markers of the disease or condition from a cell-free bodily fluid sample from the subject before the treatment; determining a second profile of at least one of the one or more markers from a population of non-phagocytic cells from the subject before the treatment; identifying a first difference between the first and second profiles of at least one or more of said markers; b) determining a third profile of the one or more markers from a cell-free bodily fluid sample from the subject after the treatment; determining a fourth profile of at least one of the one or more markers from a population of non-phagocytic cells from the subject after the treatment; identifying a second difference between the third and fourth profiles of at least one or more of said markers; and c) identifying a difference between the first difference and the second difference, wherein the identified difference is indicative of the efficacy of the treatment for said disease or condition in the subject.

In yet another aspect, this invention provides a method for monitoring the progression or regression of a disease or condition in a subject comprising: a) determining a first profile of one or more markers of the disease or condition from a cell-free bodily fluid sample from the subject at a first time point; determining a second profile of at least one of the one or more markers from a population of non-phagocytic cells from the subject at the first time point; identifying a first difference between the first and second profiles of at least one or more of said markers; b) determining a third profile of the one or more markers from a cell-free bodily fluid sample from the subject at a second time point; determining a fourth profile of at least one of the one or more markers from a population of non-phagocytic cells from the subject at the second time point; identifying a second difference between the third and fourth profiles of at least one or more of said markers; and c) identifying a difference between the first difference and the second difference, wherein the identified difference is indicative of the progression or regression of said disease or condition in the subject.

In yet another aspect, this invention provides a method for identifying a compound capable of ameliorating or treating a disease or condition in a subject comprising: a) determining a first profile of one or more markers of the disease or condition from a cell-free bodily fluid sample from the subject before administering the compound to the subject; determining a second profile of at least one of the one or more markers from a population of non-phagocytic cells from the subject before administering the compound to the subject; identifying a first difference between the first and second profiles of at least one or more of said markers; b) determining a third profile of the one or more markers from a cell-free bodily fluid sample from the subject after the administration of the compound; determining a fourth profile of at least one of the one or more markers from a population of non-phagocytic cells from the subject after the administration of the compound; identifying a second difference between the third and fourth profiles of at least one or more of said markers; and c)identifying a difference between the first difference and the second difference, wherein the identified difference indicates that the compound is capable of ameliorating or treating said disease or condition in the subject.

In yet another aspect, this invention provides a method for diagnosing or aiding in the diagnosis of a disease or condition in a subject comprising: a) determining a first profile of one or more markers of the disease or condition from a cell-free bodily fluid sample; b) determining a second profile of at least one of the one or more markers from a cell-free bodily fluid sample; and c) identifying a difference between the first and second profiles of at least one or more of said markers, wherein the difference is indicative of the presence of said disease or condition in the subject.

In yet another aspect, this invention provides a method for assessing the risk of developing a disease or condition in a subject comprising: a) determining a first profile of one or more markers of the disease or condition from a cell-free bodily fluid sample; b) determining a second profile of at least one of the one or more markers from a population of =2n phagocytic cells; and c) identifying a difference between the first and second profiles of at least one or more of said markers, wherein the difference is indicative of the risk of developing said disease or condition in the subject.

In yet another aspect, this invention provides a method for prognosing or aiding in the prognosis of a disease or condition in a subject comprising: a) determining a first profile of one or more markers of the disease or condition from a cell-free bodily fluid sample; b) determining a second profile of at least one of the one or more markers from a population of=2n phagocytic cells; and c) identifying a difference between the first and second profiles of at least one or more of said markers, wherein the difference is indicative of the prognosis of said disease or condition in the subject.

In yet another aspect, this invention provides a method for assessing the efficacy of a treatment for a disease or condition in a subject comprising: a) determining a first profile of one or more markers of the disease or condition from a cell-free bodily fluid sample from the subject before the treatment; determining a second profile of at least one of the one or more markers from a population of =2n phagocytic cells from the subject before the treatment; identifying a first difference between the first and second profiles of at least one or more of said markers; b) determining a third profile of the one or more markers from a cell-free bodily fluid sample from the subject after the treatment; determining a fourth profile of at least one of the one or more markers from a population of =2n phagocytic cells from the subject after the treatment; identifying a second difference between the third and fourth profiles of at least one or more of said markers; and c) identifying a difference between the first difference and the second difference, wherein the identified difference is indicative of the efficacy of the treatment for said disease or condition in the subject.

In yet another aspect, this invention provides a method for monitoring the progression or regression of a disease or condition in a subject comprising: a) determining a first profile of one or more markers of the disease or condition from a cell-free bodily fluid sample from the subject at a first time point; determining a second profile of at least one of the one or more markers from a population of =2n phagocytic cells from the subject at the first time point; identifying a first difference between the first and second profiles of at least one or more of said markers; b) determining a third profile of the one or more markers from a cell-free bodily fluid sample from the subject at a second time point; determining a fourth profile of at least one of the one or more markers from a population of =2n phagocytic cells from the subject at the second time point; identifying a second difference between the third and fourth profiles of at least one or more of said markers; and c) identifying a difference between the first difference and the second difference, wherein the identified difference is indicative of the progression or regression of said disease or condition in the subject.

In yet another aspect, this invention provides a method for identifying a compound capable of ameliorating or treating a disease or condition in a subject comprising: a) determining a first profile of one or more markers of the disease or condition from a cell-free bodily fluid sample from the subject before administering the compound to the subject; determining a second profile of at least one of the one or more markers from a population of =2n phagocytic cells from the subject before administering the compound to the subject; identifying a first difference between the first and second profiles of at least one or more of said markers; b) determining a third profile of the one or more markers from a cell-free bodily fluid sample from the subject after the administration of the compound; determining a fourth profile of at least one of the one or more markers from a population of =2n phagocytic cells from the subject after the administration of the compound; identifying a second difference between the third and fourth profiles of at least one or more of said markers; c) identifying a difference between the first difference and the second difference, wherein the identified difference indicates that the compound is capable of ameliorating or treating said disease or condition in the subject.

In yet another aspect, this invention provides a method for identifying one or more markers for a disease or condition comprising: a) determining a first profile of analytes from a cell-free bodily fluid sample from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from a cell-free bodily fluid sample from a control subject not having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. Optionally, this method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; determining a second profile of analytes from a cell-free bodily fluid sample from a control subject not having said disease or condition; identifying a first set of differences between the first and second profiles, wherein the first set of differences is specific to the first profile relative to the second profile; b) determining a third profile of analytes from non-phagocytic cells from the subject having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; obtaining a second profile of analytes from a cell-free bodily fluid sample from a control subject not having said disease or condition by data mining; identifying a first set of differences between the first and second profiles, wherein the first set of differences is specific to the first profile relative to the second profile; b) determining a third profile of analytes from non-phagocytic cells from the subject having said disease or condition; obtaining a fourth profile of analytes from non-phagocytic cells from a control subject not having said disease or condition by data mining; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition by data mining; obtaining a sixth profile of analytes from cells or tissues not affected by said disease or condition by data mining; identifying a third set of differences between the fifth and sixth profiles, wherein the third set of differences is specific to the fifth profile relative to the sixth profile; and e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from cells or tissues affected by said disease or condition from the subject having said disease or condition; determining a fourth profile of analytes from cells or tissues not affected by said disease or condition from the subject having said disease or condition; identifying a second set of differences between the third and fourth profiles, wherein the second set of differences is specific to the third profile relative to the fourth profile; c) identifying one or more analytes present in both the first set of differences and the second set of differences, the identified analytes being markers of said disease or condition. And optionally, the method further comprises d) determining a fifth profile of analytes from a cell-free bodily fluid sample from a control subject not having said disease or condition; identifying a third set of differences between the first and fifth profiles, wherein the third set of differences is specific to the first profile relative to the fifth profile; e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; determining a second profile of analytes from =2n phagocytic cells from the subject having said disease or condition; identifying a first set of differences between the first and second profiles, wherein the first set of differences is specific to the first profile relative to the second profile; b) determining a third profile of analytes from a cell-free bodily fluid sample from a control subject not having said disease or condition; determining a fourth profile of analytes from =2n phagocytic cells from the control subject not having said disease or condition; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises: d) obtaining a fifth profile of analytes from cells or tissues affected by said disease or condition from the subject having said disease or condition; obtaining a sixth profile of analytes from cells or tissues not affected by said disease or condition from the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In some embodiments, the markers or the analytes are nucleic acids (e.g., nucleotides, oligonucleotides, DNAs, RNAs, or DNA-RNA hybrids), proteins (e.g., , amino acids, peptides, enzymes, antigens, antibodies, cytokines, lipoproteins, glycoproteins, or hormones), lipids (e.g., fatty acids, phosphatides, cholesterol), carbohydrates (e.g., monosaccharides, disaccharides, polysaccharides), metabolites (e.g., vitamins, primary metabolites, secondary metabolites), or combinations thereof.

In some embodiments, the profile is a nucleic acid profile (e.g., genotypic profile, a single nucleotide polymorphism profile, a gene mutation profile, a gene copy number profile, a DNA methylation profile, a DNA acetylation profile, a chromosome dosage profile, a gene expression profile), a protein profile (e.g., protein expression, protein activation), a lipid profile, a carbohydrate profile, a metabolite profile, or a combination thereof. In some embodiments, the profile is determined by a qualitative assay, a quantitative assay, or a combination thereof.

In some embodiments, at least one of the one or more markers is upregulated or activated in the cell-free bodily fluids compared to the non-phagocytic cells. In some embodiments, at least one of the one or more markers is down-regulated or inhibited in the cell-free bodily fluid sample compared to the non-phagocytic cells. In some embodiments, at least one of the one or more markers is up-regulated or activated in the cell-free bodily fluids compared to the =2n phagocytic cells. In some embodiments, at least one of the one or more markers is down-regulated or inhibited in the cell-free bodily fluids compared to the =2n phagocytic cells.

In some embodiments, the first profile, the second profile, the third profile, the fourth profile, the fifth profile, or the sixth profile comprises the absence of at least one of the one or more markers.

In some embodiments, the difference is at least 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference.

In some embodiments, the methods of this invention also comprise lysing the =2n phagocytic cells and the non-phagocytic cells; and also extracting the cellular contents from those cells. In some embodiments, the methods of this invention comprise extracting the cellular contents from cell-free bodily fluids. In some embodiments, the cell-free bodily fluids comprise viable diseased cells, dead diseased cells, apoptotic diseased cells, circulating tumor cells, infectious agents, fetal cells, trophoblasts, or fragments thereof.

In some embodiments, at least one of the one or more markers of the disease or condition is present in the cellular contents of the cell-free bodily fluids. In some embodiments, the one or more markers of said disease or condition are not present in the cellular contents of the non-phagocytic cells or the =2n phagocytic cells.

In some embodiments, the methods of this invention also comprise comparing the identified difference of c) to a repository of one or more known markers of said disease or condition (e.g., data obtained by data mining).

In some embodiments, the phagocytic cells are professional phagocytic cells (e.g., neutrophils, macrophages, monocytes, dendritic cells, foam cells, mast cells, eosinophils), non-professional phagocytic cells (e.g., epithelial cells, endothelial cells, fibroblasts, mesenchymal cells), or mixtures thereof. In some embodiments, the non-phagocytic cells are T cells, B cells, null cells, basophils, or mixtures thereof.

In some embodiments, the phagocytic cells (e.g., =2n phagocytic cells) and the non-phagocytic cells are isolated from a bodily fluid sample (e.g., blood, urine), tissues, or cells (e.g., white blood cells, fetal cells) of the subject.

In some embodiments, a standard/know cell separation/isolation/purification technique, such as antibody, flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platforms, or a combination thereof, is used to isolate phagocytic cells (e.g., =2n phagocytic cells) and non-phagocytic cells from bodily fluids, tissues or cells, or to separate >2n phagocytic cells from =2n phagocytic cells.

Also provided by this invention are markers that can be used in the methods of this invention and that can be identified by the methods of this invention.

The present invention also relates to the following items.
Item 1. A method for diagnosing or aiding in the diagnosis of a disease or condition, or assessing the risk of developing the disease or condition, or prognosing or aiding in the prognosis of the disease or condition, in a subject comprising:
   a) determining a first profile of one or more markers of the disease or condition from a cell-free bodily fluid sample;
   b) determining a second profile of at least one of the one or more markers from a population of phagocytic cells having a DNA content of 2n (=2n phagocytic cells) or a population of non-phagocytic cells; and
   c) identifying a difference between the first and second profiles of at least one or more of said markers, wherein the difference is indicative of the presence of said disease or condition, or the risk of developing said disease or condition, or the prognosis of said disease or condition, in the subject.
Item 2. A method for assessing the efficacy of a treatment for a disease or condition, or monitoring the progression or regression of the disease or condition, or identifying a compound capable of ameliorating or treating the disease or condition, in a subject comprising:
   a) determining a first profile of one or more markers of the disease or condition from a cell-free bodily fluid sample from the subject at a first time point; determining a second profile of at least one of the one or more markers from a population of =2n phagocytic cells or a population of non-phagocytic cells from the subject at the first time point; identifying a first difference between the first and second profiles of at least one or more of said markers;
   b) determining a third profile of the one or , or at a second time point, or after administration of the compound, respectively more markers from a cell-free bodily fluid sample from the subject at a second time point; determining a fourth profile of at least one of the one or more markers from a population of =2n phagocytic cells or a population of non-phagocytic cells from the subject at the second time point; identifying a second difference between the third and fourth profiles of at least one or more of said markers; and
   c) identifying a difference between the first difference and the second difference, wherein the identified difference is indicative of the efficacy of the treatment for said disease or condition, or the progression or regression of said disease or condition, or indicates that the compound is capable of ameliorating or treating said disease or condition, in the subject.
Item 3. A method for diagnosing or aiding in the diagnosis of a disease or condition in a subject comprising:
   a) determining a first profile of one or more markers of the disease or condition from a cell-free bodily fluid sample;
   b) determining a second profile of at least one of the one or more markers from a population of non-phagocytic cells; and
   c) identifying a difference between the first and second profiles of at least one or more of said markers, wherein the difference is indicative of the presence of said disease or condition in the subject.
Item 4. A method for assessing the risk of developing a disease or condition in a subject comprising:
   a) determining a first profile of one or more markers of the disease or condition from a cell-free bodily fluid sample;
   b) determining a second profile of at least one of the one or more markers from a population of non-phagocytic cells; and
   c) identifying a difference between the first and second profiles of at least one or more of said markers, wherein the difference is indicative of the risk of developing said disease or condition in the subject.
Item 5. A method for prognosing or aiding in the prognosis of a disease or condition in a subject comprising:
   a) determining a first profile of one or more markers of the disease or condition from a cell-free bodily fluid sample;
   b) determining a second profile of at least one of the one or more markers from a population of non-phagocytic cells; and
   c) identifying a difference between the first and second profiles of at least one or more of said markers, wherein the identified difference is indicative of the prognosis of said disease or condition in the subject.
Item 6. A method for assessing the efficacy of a treatment for a disease or condition in a subject comprising:
   a) determining a first profile of one or more markers of the disease or condition from a cell-free bodily fluid sample from the subject before the treatment; determining a second profile of at least one of the one or more markers from a population of non-phagocytic cells from the subject before the treatment; identifying a first difference between the first and second profiles of at least one or more of said markers;
   b) determining a third profile of the one or more markers from a cell-free bodily fluid sample from the subject after the treatment; determining a fourth profile of at least one of the one or more markers from a population of non-phagocytic cells from the subject after the treatment; identifying a second difference between the third and fourth profiles of at least one or more of said markers; and
   c) identifying a difference between the first difference and the second difference, wherein the identified difference is indicative of the efficacy of the treatment for said disease or condition in the subject.
Item 7. A method for monitoring the progression or regression of a disease or condition in a subject comprising:
   a) determining a first profile of one or more markers of the disease or condition from a cell-free bodily fluid sample from the subject at a first time point; determining a second profile of at least one of the one or more markers from a population of non-phagocytic cells from the subject at the first time point; identifying a first difference between the first and second profiles of at least one or more of said markers;
   b) determining a third profile of the one or more markers from a cell-free bodily fluid sample from the subject at a second time point; determining a fourth profile of at least one of the one or more markers from a population of non-phagocytic cells from the subject at the second time point; identifying a second difference between the third and fourth profiles of at least one or more of said markers; and
   c) identifying a difference between the first difference and the second difference, wherein the identified difference is indicative of the progression or regression of said disease or condition in the subject.
Item 8. A method for identifying a compound capable of ameliorating or treating a disease or condition in a subject comprising:
   a) determining a first profile of one or more markers of the disease or condition from a cell-free bodily fluid sample from the subject before administering the compound to the subject; determining a second profile of at least one of the one or more markers from a population of non-phagocytic cells from the subject before administering the compound to the subject; identifying a first difference between the first and second profiles of at least one or more of said markers;
   b) determining a third profile of the one or more markers from a cell-free bodily fluid sample from the subject after the administration of the compound; determining a fourth profile of at least one of the one or more markers from a population of non-phagocytic cells from the subject after the administration of the compound; identifying a second difference between the third and fourth profiles of at least one or more of said markers; and
   c) identifying a difference between the first difference and the second difference, wherein the identified difference indicates that the compound is capable of ameliorating or treating said disease or condition in the subject.
Item 9. A method for diagnosing or aiding in the diagnosis of a disease or condition in a subject comprising:
   a) determining a first profile of one or more markers of the disease or condition from a cell-free bodily fluid sample;
   b) determining a second profile of at least one of the one or more markers from a population of phagocytic cells having a DNA content of 2n (=2n phagocytic cells); and
   c) identifying a difference between the first and second profiles of at least one or more of said markers, wherein the difference is indicative of the presence of said disease or condition in the subject.
Item 10. A method for assessing the risk of developing a disease or condition in a subject comprising:
   a) determining a first profile of one or more markers of the disease or condition from a cell-free bodily fluid sample;
   b) determining a second profile of at least one of the one or more markers from a population of =2n phagocytic cells; and
   c) identifying a difference between the first and second profiles of at least one or more of said markers, wherein the difference is indicative of the risk of developing said disease or condition in the subject.
Item 11. A method for prognosing or aiding in the prognosis of a disease or condition in a subject comprising:
   a) determining a first profile of one or more markers of the disease or condition from a cell-free bodily fluid sample;
   b) determining a second profile of at least one of the one or more markers from a population of =2n phagocytic cells; and
   c) identifying a difference between the first and second profiles of at least one or more of said markers, wherein the difference is indicative of the prognosis of said disease or condition in the subject.
Item 12. A method for assessing the efficacy of a treatment for a disease or condition in a subject comprising:
   a) determining a first profile of one or more markers of the disease or condition from a cell-free bodily fluid sample from the subject before the treatment; determining a second profile of at least one of the one or more markers from a population of =2n phagocytic cells from the subject before the treatment; identifying a first difference between the first and second profiles of at least one or more of said markers;
   b) determining a third profile of the one or more markers from a cell-free bodily fluid sample from the subject after the treatment; determining a fourth profile of at least one of the one or more markers from a population of =2n phagocytic cells from the subject after the treatment; identifying a second difference between the third and fourth profiles of at least one or more of said markers; and
   c) identifying a difference between the first difference and the second difference, wherein the identified difference is indicative of the efficacy of the treatment for said disease or condition in the subject.
Item 13. A method for monitoring the progression or regression of a disease or condition in a subject comprising:
   a) determining a first profile of one or more markers of the disease or condition from a cell-free bodily fluid sample from the subject at a first time point; determining a second profile of at least one of the one or more markers from a population of =2n phagocytic cells from the subject at the first time point; identifying a first difference between the first and second profiles of at least one or more of said markers;
   b) determining a third profile of the one or more markers from a cell-free bodily fluid sample from the subject at a second time point; determining a fourth profile of at least one of the one or more markers from a population of =2n phagocytic cells from the subject at the second time point; identifying a second difference between the third and fourth profiles of at least one or more of said markers; and
   c) identifying a difference between the first difference and the second difference, wherein the identified difference is indicative of the progression or regression of said disease or condition in the subject.
Item 14. A method for identifying a compound capable of ameliorating or treating a disease or condition in a subject comprising:
   a) determining a first profile of one or more markers of the disease or condition from a cell-free bodily fluid sample from the subject before administering the compound to the subject; determining a second profile of at least one of the one or more markers from a population of =2n phagocytic cells from the subject before administering the compound to the subject; identifying a first difference between the first and second profiles of at least one or more of said markers;
   b) determining a third profile of the one or more markers from a cell-free bodily fluid sample from the subject after the administration of the compound; determining a fourth profile of at least one of the one or more markers from a population of =2n phagocytic cells from the subject after the administration of the compound; identifying a second difference between the third and fourth profiles of at least one or more of said markers;
   c) identifying a difference between the first difference and the second difference, wherein the identified difference indicates that the compound is capable of ameliorating or treating said disease or condition in the subject.
Item 15. The method of any one of the items 3-8, wherein at least one of the one or more markers is up-regulated or activated in the cell-free bodily fluid sample compared to the non-phagocytic cells.
Item 16. The method of any one of the items 3-8, wherein at least one of the one or more markers is down-regulated or inhibited in the cell-free bodily fluid sample compared to the non-phagocytic cells.
Item 17. The method of any one of the items 9-14, wherein at least one of the one or more markers is up-regulated or activated in the cell-free bodily fluid sample compared to the =2n phagocytic cells.
Item 18. The method of any one of the items 9-14, wherein at least one of the one or more markers is down-regulated or inhibited in the cell-free bodily fluid sample compared to the =2n phagocytic cells.
Item 19. The method of any one of the items 3-14, wherein the first profile or the second profile comprises the absence of at least one of the one or more markers of said disease or condition.
Item 20. The method of any one of the items 6-8 and 12-14, wherein the third profile or the fourth profile comprises the absence of at least one of the one or more markers of said disease or condition.
Item 21. The method of any one of the items 3-14, further comprising extracting the one or more markers from the cell-free bodily fluid sample.
Item 22. The method of any one of the items 3-8, further comprising lysing the the non-phagocytic cells before a).
Item 23. The method of any one of the items 3-8 and 21, further comprising extracting the cellular contents from the non-phagocytic cells before a).
Item 24. The method of any one of the items 3-14, wherein the cell-free bodily fluid sample comprise viable diseased cells, dead diseased cells, apoptotic diseased cells, circulating tumor cells, infectious agents, fetal cells, trophoblasts, or fragments thereof.
Item 25. The method of any one of the items 3-14, wherein at least one of the one or more markers of said disease or condition is present in the cell-free bodily fluid sample.
Item 26. The method of item 23, wherein the one or more markers of said disease or condition are not present in the cellular contents of the non-phagocytic cells.
Item 27. The method of any one of the items 9-14, further comprising lysing the =2n phagocytic cells before a).
Item 28. The method of any one of the items 9-14 and 27, further comprising extracting cellular contents from the =2n phagocytic cells before a).
Item 29. The method of item 28, wherein the one or more markers of said disease or condition are not present in the cellular contents of the =2n phagocytic cells.
Item 30. The method of any one of the items 3-14, further comprising comparing the identified difference of c) to a repository of one or more known markers of said disease or condition.
Item 31. The method of item 30, wherein the repository is obtained by data mining.
Item 32. The method of any one of the items 9-14, wherein the phagocytic cells are professional phagocytic cells, non-professional phagocytic cells, or mixtures thereof.
Item 33. The method of item 32, wherein the professional phagocytic cells are neutrophils, macrophages, monocytes, dendritic cells, foam cells, mast cells, eosinophils, or mixtures thereof.
Item 34. The method of item 32, wherein the non-professional phagocytic cells are epithelial cells, endothelial cells, fibroblasts, mesenchymal cells, or mixtures thereof.
Item 35. The method of any one of the items 3-8, wherein the non-phagocytic cells are T cells, B cells, null cells, basophils, or mixtures thereof.
Item 36. The method of any one of the items 3-14, wherein the cell-free bodily fluid sample is separated from a bodily fluid sample.
Item 37. The method of item 36, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid.
Item 38. The method of item 36, wherein the cell-free bodily fluid sample is separated by filtration, centrifugation, flow cytometry, fluorescence activated cell sorting, gradient-based centrifugation, elution, microfluidics, magnetic separation technique, fluorescent-magnetic separation technique, nanostructure, quantum dots, high throughput microscope-based platforms, or a combination thereof.
Item 39. The method of item 36, wherein the cell-free bodily fluid sample is separated by using a substance present in the sample.
Item 40. The method of item 39, wherein the substance is a product of a marker of said disease or condition.
Item 41. The method of item 36, wherein the cell-free bodily fluid sample is plasma or serum.
Item 42. The method of any one of the items 3-8, wherein the non-phagocytic cells are isolated from a bodily fluid sample, tissues, or cells of the subject.
Item 43. The method of item 42, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid.
Item 44. The method of item 42, wherein the cells are white blood cells.
Item 45. The method of any one of the items 42-44, wherein the non-phagocytic cells are isolated using antibodies.
Item 46. The method of any one of the items 42-44, wherein the non-phagocytic cells are isolated by flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platforms, or a combination thereof.
Item 47. The method of any one of the items 9-14, wherein the =2n phagocytic cells are isolated from a bodily fluid sample, tissues, or cells of the subject.
Item 48. The method of any one of the items 9-14, wherein the =2n phagocytic cells are isolated from a population of phagocytic cells.
Item 49. The method of item 48, wherein the =2n phagocytic cells are isolated from the population of phagocytic cells by flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, or a combination thereof.
Item 50. The method of item 48, wherein the population of phagocytic cells is isolated from a bodily fluid sample, tissues, or cells of the subject.
Item 51. The method of item 47 or 50, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid.
Item 52. The method of item 47 or 50, wherein the cells are white blood cells.
Item 53. The method of any one of the items 48 and 50-52, wherein the population of phagocytic cells is isolated using antibodies.
Item 54. The method of any one of the items 48 and 50-52, wherein the population of phagocytic cells is isolated by flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, or a combination thereof.
Item 55. The method of any one of the items 3-14, wherein the one or more markers are nucleic acids, proteins, lipids, carbohydrates, metabolites, or combinations thereof.
Item 56. The method of item 54, wherein the nucleic acids are nucleotides, oligonucleotides, DNAs, RNAs, or DNA-RNA hybrids.
Item 57. The method of item 56, wherein the DNAs are double-stranded DNAs, single-stranded DNAs, multi-stranded DNAs, complementary DNAs, genomic DNAs, or non-coding DNAs.
Item 58. The method of item 56, wherein the RNAs are messenger RNAs (mRNAs), microRNAs (miRNAs), small nucleolar RNAs (snoRNAs), ribosomal RNAs (rRNAs), transfer RNAs (tRNAs), small interfering RNAs (siRNAs), heterogeneous nuclear RNAs (hnRNAs), or small hairpin RNAs (shRNAs).
Item 59. The method of item 54, wherein the proteins are amino acids, peptides, enzymes, antigens, antibodies, cytokines, lipoproteins, glycoproteins, or hormones.
Item 60. The method of item 54, wherein the lipids are fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lysosphingomyelin,, gangliosides, plasmalogen, sulfatide, low density lipoproteins (LDLs), very low density lipoproteins (VLDLs), high density lipoproteins (HDLs), sphingoid base-1-phosphates, or derivatives thereof.
Item 61. The method of item 54, wherein the carbohydrates are monosaccharides, disaccharides, polysaccharides, oligosaccharides, or derivatives thereof.
Item 62. The method of item 54, wherein the metabolites are primary metabolites, secondary metabolites, organic metabolites, inorganic metabolites, prostaglandins, hydroxyeicosatetraenoic acids, hydroxyoctadecadienoic acids, steroids, bile acids, vitamins, or derivatives thereof.
Item 63. The method of any one of the items 3-14, wherein the profile is a nucleic acid profile, a protein profile, a lipid profile, a carbohydrate profile, a metabolite profile, or a combination thereof.
Item 64. The method of item 63, wherein the profile is determined by a qualitative assay, a quantitative assay, or a combination thereof.
Item 65. The method of item 64, wherein the quantitative assay uses sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{®} sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.
Item 66. The method of item 63, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof. Item 67. The method of item 66, wherein the nucleic acid profile is determined by polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MI,PA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylationsensitive restriction enzyme analysis, bisulfite-driven conversion of non-methylated cytosine to uracil, methyl-binding PCR analysis, or a combination thereof.
Item 68. The method of item 66, wherein the nucleic acid profile is determined by a sequencing technique selected from the group consisting of direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{®} sequencing, MS-PET sequencing, mass spectrometry, and a combination thereof.
Item 69. The method of item 63, wherein the protein profile is a protein expression profile, a protein activation profile, or a combination thereof.
Item 70. The method of item 69, wherein the protein profile is determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof. Item 71. The method of item 69, wherein the protein activation profile comprises determining a phosphorylation state, an ubiquitination state, a myristoylation state, a conformational state, or a combination thereof of the one or more markers.
Item 72. The method of item 63, wherein the lipid profile is determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionizationtime of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.
Item 73. The method of item 63, wherein the carbohydrate profile is determined by chromatography, liquid chromatography, size exclusion chromatography, high performance anion exchange chromatography with pulsed amperometric detection (HPAEC-PAD), liquid chromatography, gas chromatography, fluorescent assay, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionizationtime of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassay, microfluidic chip-based assay, detection of fluorescence, detection of chemiluminescence, or a combination thereof.
Item 74. The method of any one the items 3-14, wherein the subject has at least two diseases or conditions.
Item 75. The method of item 74, wherein the subject has at least one prenatal or pregnancy-related disease or condition.
Item 76. The method of item 74, wherein the subject has at least one disease or condition that is not a disease or condition.
Item 77. The method of any one the items 3-14, wherein the subject is a mammal. Item 78. The method of item 77, wherein the subject is a human.
Item 79. The method of any one the items 3-14, wherein the disease or condition is a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a mitochondrial disorder, a respiratory-gastrointestinal tract disease or condition, a reproductive disease or condition, an ophthalmic disease or condition, a musculo-skeletal disease or condition, or a dermal disease or condition.
Item 80. The method of any one the items 3-14, wherein the difference is greater than a 1-fold difference.
Item 81. The method of item 80, wherein the difference is at least 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference.
Item 82. A method for identifying one or more markers for a disease or condition comprising:
   a) determining a first profile of analytes from a bodily fluid sample from a subject having said disease or condition; determining a second profile of analytes from non-phagocytic cells from the subject having said disease or condition; identifying a first set of differences between the first and second profiles, wherein the first set of differences is specific to the first profile relative to the second profile;
   b) determining a third profile of analytes from a bodily fluid sample from a control subject not having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile;
   c) identifying one or more analytes specific to the first set of differences relative to the second set of differences, the identified analytes being markers of said disease or condition.
Item 83. The method of item 82, further comprising:
   d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and
   e) identifying at least one of the one or more markers of c) present in the third set of differences.
Item 84. A method for identifying one or more markers of a disease or condition comprising:
   a) determining a first profile of analytes from a bodily fluid sample from a subject having said disease or condition; determining a second profile of analytes from a bodily fluid sample from a control subject not having said disease or condition; identifying a first set of differences between the first and second profiles, wherein the first set of differences is specific to the first profile relative to the second profile;
   b) determining a third profile of analytes from non-phagocytic cells from the subject having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile;
   c) identifying one or more analytes specific to the first set of differences relative to the second set of differences, the identified analytes being markers of said disease or condition.
Item 85. The method of item 84, further comprising:
   d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and
   e) identifying at least one of the one or more markers of c) present in the third set of differences.
Item 86. A method for identifying one or more markers of a disease or condition comprising:
   a) determining a first profile of analytes from a bodily fluid sample from a subject having said disease or condition; obtaining a second profile of analytes from a bodily fluid sample from a control subject not having said disease or condition by data mining; identifying a first set of differences between the first and second profiles, wherein the first set of differences is specific to the first profile relative to the second profile;
   b) determining a third profile of analytes from non-phagocytic cells from the subject having said disease or condition; obtaining a fourth profile of analytes from non-phagocytic cells from a control subject not having said disease or condition by data mining; identifying a second set of differences between the third and fourth profiles, wherein the second set of differences is specific to the third profile relative to the fourth profile; and
   c) identifying one or more analytes specific to the first set of differences relative to the second set of differences, the identified analytes being markers of said disease or condition.
Item 87. The method of item 86, further comprising:
   d) obtaining a fifth profile of analytes from cells or tissues affected by said disease or condition by data mining; obtaining a sixth profile of analytes from cells or tissues not affected by said disease or condition by data mining; identifying a third set of differences between the fifth and sixth profiles, wherein the third set of differences is specific to the fifth profile relative to the sixth profile; and
   e) identifying at least one of the one or more markers of c) present in the third set of differences.
Item 88. A method for identifying one or more markers of a disease or condition comprising:
   a) determining a first profile of analytes from a bodily fluid sample from a subject having said disease or condition; determining a second profile of analytes from non-phagocytic cells from the subject having said disease or condition; identifying a first set of differences between the first and second profiles, wherein the first set of differences is specific to the first profile relative to the second profile;
   b) determining a third profile of analytes from cells or tissues affected by said disease or condition from the subject having said disease or condition; determining a fourth profile of analytes from cells or tissues not affected by said disease or condition from the subject having said disease or condition; identifying a second set of differences between the third and fourth profiles, wherein the second set of differences is specific to the third profile relative to the fourth profile;
   c) identifying one or more analytes present in both the first set of differences and the second set of differences, the identified analytes being markers of said disease or condition.
Item 89. The method of item 88, further comprising:
   d) determining a fifth profile of analytes from a bodily fluid sample from a control subject not having said disease or condition; identifying a third set of differences between the first and fifth profiles, wherein the third set of differences is specific to the first profile relative to the fifth profile;
   e) identifying at least one of the one or more markers of c) present in the third set of differences.
Item 90. A method for identifying one or more markers of a disease or condition comprising:
   a) determining a first profile of analytes from a bodily fluid sample from a subject having said disease or condition; determining a second profile of analytes from =2n phagocytic cells from the subject having said disease or condition; identifying a first set of differences between the first and second profiles, wherein the first set of differences is specific to the first profile relative to the second profile;
   b) determining a third profile of analytes from a bodily fluid sample from a control subject not having said disease or condition; determining a fourth profile of analytes from =2n phagocytic cells from the control subject not having said disease or condition; identifying a second set of differences between the third and fourth profiles, wherein the second set of differences is specific to the third profile relative to the fourth profile; and
   c) identifying one or more analytes specific to the first set of differences relative to the second set of differences, the identified analytes being markers of said disease or condition.
Item 91. The method of item 90, further comprising:
   d) obtaining a fifth profile of analytes from cells or tissues affected by said disease or condition from the subject having said disease or condition; obtaining a sixth profile of analytes from cells or tissues not affected by said disease or condition from the subject having said disease or condition; identifying a third set of differences between the fifth and sixth profiles, wherein the third set of differences is specific to the fifth profile relative to the sixth profile; and
   e) identifying at least one of the one or more markers of c) present in the third set of differences.
Item 92. The method of any one of the items 82-91, further comprising extracting the one or more markers from the cell-free bodily fluid sample.
Item 93. The method of any one of the items 82-89, further comprising lysing the non-phagocytic cells before a).
Item 94. The method of any one of the items 82-89 and 93, further comprising extracting the cellular contents from the non-phagocytic cells before a).
Item 95. The method of any one of the items 82-91, wherein the cell-free bodily fluid sample comprise viable diseased cells, dead diseased cells, apoptotic diseased cells, circulating tumor cells, infectious agents, fetal cells, trophoblasts, or fragments thereof.
Item 96. The method of any one of the items 82-91, wherein at least one of the one or more markers of said disease or condition is present in the cell-free bodily fluid sample.
Item 97. The method of item 94, wherein the one or more markers of said disease or condition are not present in the cellular contents of the non-phagocytic cells.
Item 98. The method of any one of the items 90-91, further comprising lysing the =2n phagocytic cells before a).
Item 99. The method of any one of the items 90-91 and 98, further comprising extracting cellular contents from the =2n phagocytic cells before a).
Item 100. The method of item 99, wherein the one or more markers of said disease or condition are not present in the cellular contents of the =2n phagocytic cells. Item 101. The method of any one of the items 82-91, further comprising comparing the identified difference of c) to a repository of one or more known markers of said disease or condition.
Item 102. The method of item 101, wherein the repository is obtained by data mining.
Item 103. The method of any one of the items 90-91, wherein the phagocytic cells are professional phagocytic cells, non-professional phagocytic cells, or mixtures thereof.
Item 104. The method of item 111, wherein the professional phagocytic cells are neutrophils, macrophages, monocytes, dendritic cells, foam cells, mast cells, eosinophils, or mixtures thereof.
Item 105. The method of item 111, wherein the non-professional phagocytic cells are epithelial cells, endothelial cells, fibroblasts, mesenchymal cells, or mixtures thereof.
Item 106. The method of any one of the items 82-89, wherein the non-phagocytic cells are T cells, B cells, null cells, basophils, or mixtures thereof.
Item 107. The method of any one of the items 90-91, wherein the cell-free bodily fluid sample is separated from a bodily fluid sample.
Item 108. The method of item 107, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid.
Item 109. The method of item 107, wherein the cell-free bodily fluid sample is separated by filtration, centrifugation, flow cytometry, fluorescence activated cell sorting, gradient-based centrifugation, elution, microfluidics, magnetic separation technique, fluorescent-magnetic separation technique, nanostructure, quantum dots, high throughput microscope-based platforms, or a combination thereof.
Item 110. The method of item 107, wherein the cell-free bodily fluid sample is separated by using a substance present in the sample.
Item 111. The method of any one of the items 82-91, wherein the one or more markers are nucleic acids, proteins, lipids, carbohydrates, metabolites, or combinations thereof.
Item 112. The method of any one of the items 82-91, wherein the analytes are nucleic acids, proteins, lipids, carbohydrates, metabolites, or combinations thereof.
Item 113. The method of item 111 or 112, wherein the nucleic acids are nucleotides, oligonucleotides, DNAs, RNAs, or DNA-RNA hybrids.
Item 114. The method of item 113, wherein the DNAs are double-stranded DNAs, single-stranded DNAs, multi-stranded DNAs, complementary DNAs, genomic DNAs or non-coding DNAs.
Item 115. The method of item 113, wherein the RNAs are messenger RNAs (mRNAs), microRNAs (miRNAs), small nucleolar RNAs (snoRNAs), ribosomal RNAs (rRNAs), transfer RNAs (tRNAs), small interfering RNAs (siRNAs), heterogeneous nuclear RNAs (hnRNAs), or small hairpin RNAs (shRNAs).
Item 116. The method of item 111 or 112, wherein the proteins are amino acids, peptides, enzymes, antigens, antibodies, cytokines, lipoproteins, glycoproteins, or hormones.
Item 117. The method of item 111 or 112, wherein the lipids are fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lysosphingomyelin, gangliosides, plasmalogen, sulfatide, low density lipoproteins (LDLs), very low density lipoproteins (VLDLs), high density lipoproteins (HDLs), sphingoid base-1-phosphates or derivatives thereof.
Item 118. The method of item 111 or 112, wherein the carbohydrates are monosaccharides, disaccharides, polysaccharides, oligosaccharides, or derivatives thereof.
Item 119. The method of item 111 or 112, wherein the metabolites are primary metabolites, secondary metabolites, organic metabolites, inorganic metabolites, prostaglandins, hydroxyeicosatetraenoic acids, hydroxyoctadecadienoic acids, steroids, bile acids, vitamins, or derivatives thereof.
Item 120. The method of any one of the items 82-91, wherein the profile is a nucleic acid profile, a protein profile, a lipid profile, a carbohydrate profile, a metabolite profile, or a combination thereof.
Item 121. The method of item 120, wherein the profile is determined by a qualitative assay, a quantitative assay, or a combination thereof.
Item 122. The method of item 121, wherein the quantitative assay uses sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{®} sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.
Item 123. The method of item 120, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof. Item 124. The method of item 123, wherein the nucleic acid profile is determined by polymerase chain reaction (PCR) analysis, sequencing analysis, electrophoretic analysis, restriction fragment length polymorphism (RFLP) analysis, Northern blot analysis, reverse-transcriptase-PCR analysis (RT-PCR), quantitative PCR, quantitative RT-PCR, allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylationsensitive restriction enzyme analysis, surface plasmon resonance, bisulfite-driven conversion of non-methylated cytosine to uracil, methyl-binding PCR analysis, or a combination thereof.
Item 125. The method of item 123, wherein the nucleic acid profile is determined by a sequencing technique selected from direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{®} sequencing, MS-PET sequencing, mass spectrometry, and a combination thereof.
Item 126. The method of item 120, wherein the protein profile is a protein expression profile, a protein activation profile, or a combination thereof.
Item 127. The method of item 126, wherein the protein activation profile comprises determining a phosphorylation state, an ubiquitination state, a myristoylation state, or a conformational state of the one or more markers.
Item 128. The method of item 126, wherein the protein profile is determined by an immunohistochemistry assay, an enzyme-linked immunosorbent assay (ELISA), chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, surface plasmon resonance, microfluidic chip-based assays, Western blotting assay, or a combination thereof.
Item 129. The method of item 126, wherein the lipid profile is determined by chromatography, liquid chromatography, size exclusion chromatography, high performance liquid chromatography (HPLC), gas chromatography, mass spectrometry, matrix assisted laser desorption/ionization-time of flight (MALDI-TOF) mass spectrometry, tandem mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assays, detection of fluorescence, detection of chemiluminescence, or a combination thereof.
Item 130. The method of item 126, wherein the carbohydrate profile is determined by chromatography, liquid chromatography, size exclusion chromatography, high performance anion exchange chromatography with pulsed amperometric detection (HPAEC-PAD), liquid chromatography, gas chromatography, fluorescent assay, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assays, detection of fluorescence, detection of chemiluminescence, or a combination thereof.
Item 131. The method of any one the items 82-91, wherein the subject is a mammal.
Item 132. The method of item 131, where in the subject is a human.
Item 133. The method of any one the items 82-91, wherein the disease or condition is a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a mitochondrial disorder, a respiratory-gastrointestinal tract disease or condition, a reproductive disease or condition, an ophthalmic disease or condition, a musculo-skeletal disease or condition, or a dermal disease or condition.
Item 134. The method of any one the items 82-91, wherein the difference is greater than a 1-fold difference.
Item 135. The method of item 134, wherein the difference is at least 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference.
Item 136. The method of item 3 or 9, further comprising determining at least one diagnostic parameter of said disease or condition.
Item 137. The method of item 136, wherein the diagnostic parameter is determined by physical inspection, visual inspection, biopsy, scanning, histology, radiology, imaging, ultrasound, use of a commercial kit, genetic testing, immunological testing, analysis of bodily fluids, or monitoring neural activity.
Item 138. The method of any one of the items 3-14, wherein the one or more markers comprise at least one gene selected from the group consisting of AKT2, BAK1, EGFR, ERBB2, ETS2, FOS, JUN, MAP2K1, MMP2, PDGFB, RB1, SERPINB2, SNCG, and SPP1.
Item 139. The method of any one of the items 3-14, wherein the one or more markers comprise at least one gene selected from the group consisting of AKT1, AKT2, BAK2, CDC25A, E2F1, EGFR, ERBB2, FOS, JUN, MAP2K1, MMP2, NFKB1, PDGFB, PIK3R1, PNN, RB1, SERPINB2, SERPINB5, SNCG, SPP1, TERT, TIMP3, and TP53.
Item 140. The method of any one of the items 3-14, wherein the one or more markers comprise at least one gene selected from the group consisting of CASP8, CASP9, COL18A1, ETS2, HTATIP2, MMP9, SRC, and TWIST1.
Item 141. The method of any one of the items 3-14, wherein the one or more markers comprise at least one gene selected from the group consisting of AKT1, APAF1, ATM, CDC25A, CDKN1A, ETS2, FOS, IL8, ITGA4, ITGA6, ITGAV, JUN, MAP2K1, NFKBIA, PLAU, PLAUR, RAF1, SERPINB2, SYK, TIMP1, TNF, TNFRSF10B, and TNFRSF1A.
Item 142. The method of any one of the items 3-14, wherein the one or more markers comprise at least one gene selected from the group consisting of ACP2, AK2, AKT3, ARL5B, ATP2B3, BGN, BRAF, BTG2, CAMKK2, CAPG, CAPN12, CPLX2, DENNDSA, DNA2, FAM104A, FNIP1, GFRA4, GLUD1, GNAQ, GP1BB, HNRPLL, HOXA2, HPS3, INPP4A, ITGAV, KLHL23, LANCL2, LYPD6, MAPKAPK3, MEF2A (includes, EG:4205), MEF2C, NVL, PCYT1A, PGLYRP4, PLOD1, PPP1CB, PRKAB2, PROS1, PTPRE, RASA4 (includes,EG:10156), RBMS2, RBPJ, STATSB, THBS1, TRIB1, TRIM2, TSPAN6, and ZDHHC21.
Item 143. The method of any one of the items 3-14, wherein the one or more markers comprise at least one gene selected from the group consisting of B4GALT5, BOP1, CCL2, CCL3, CCL3L1, CCRL2, CD83, CLEC4G, CLIC4, CTSC, CTSO, CXCL10, FCGR3A, FPR3, HBA1, HBB, LRMP, MAP1LC3B2, MS4A4A, MSR1, MYADML, NID1, PF4, PION, RNF217, SAMD9L, SERPING1, and SPARC.
Item 144. The method of any one of the items 3-14, wherein the one or more markers comprise at least one gene selected from the group consisting of ACOT9, AMPD2, ARHGAP15, BATF2, C3AR1, C5orf41, CCL3, CCL3L1, CD63, CHST11, CHSY1, CLEC4G, CTSZ, CXorf21, CYTH4, CYTIP, DLEU2, DNAJA1, DOCK8, DTX3L, DUSP6, EPSTI1, ERF, F2RL1, FYB, GABRB2, GBPS, GLRX, GNB4, ICAM1, IFI35, IFIH1, IFNAR2, IL1R1, IRF1, ITGA5, LAP3, LAPTMS, LCP2, MAP1LC3B, MAP1LC3B2, MICAL2, MT1DP, MT1JP, MT1M, MT2A, MYADML, NEK6, NINJ2, NNMT, NT5C3L, NUB1, PDE4B, PLOD1, PML, PRKCB, PSMB9, RCN3, RGS4, RNASE6, RTP4, SAMD9L, SEL1L, SERPING1, SETX, SIGLEC10, SKIL, SLC7A7, SNORA21, SP100, SP110, SP140, SSFA2, STAT2, STK17B, STK3, TDRD7, TMCC1, TMPRSS11E2, TNFRSF1B, TPM1, TRIM21,TXNDC4, UBE2L6, UBE2W, USP18, VAV1, WARS, WIPF1, and WIPI1.
Item 145. The method of any one of the items 3-14, wherein the one or more markers comprise at least one gene selected from the group consisting of ADAR, ADM, ALAS1, ANKRD22, ARHGAP27, B3GNT5, BCL10, C12orf35, C15orf29, C2orf59, CD177, CEACAM1, CPEB2, DDX58, F2RL1, GDPD3, GNAI3, HIST2H3A, HIST2H3D, HIST2H4A, HMGCR, HSPA6, HSPC159, IL4R, IMPA2, KPNB1, KREMEN1, KRT23, LDLR, LOC100130904, LTB4R, MAEA, MARK2, MBOAT2, MPZL3, N4BP1, NBEAL2, NMI, NPEPPS, PARP14, PGM2, PPIF, PXN, RALBP1, ROD1, RPS6KA1, S100P, SERTAD2, SLC9A1, SLPI, SP110, SPINT1, ST14, TBC1D3, TNFRSF9, TRIM21, UPP1, VPS24, ZBTB34, and ZNF256.
Item 146. The method of any one of the items 3-14, wherein the one or more markers comprise at least one or more of the markers identified by the methods of any one the items 82-91.
Item 147. A kit comprising a plurality of marker detection agents that detect at least one or more of the markers identified by the methods of any one of the items 82-91.
Item 148. A method of treating or preventing a disease or condition in a subject comprising administering to said subject an agent that modulates the activity or expression of at least one or more of the markers identified by the methods of any one the items 82-91.

### Brief Description of the Drawings

The foregoing and other features and advantages of the present invention will be more fully understood from the following detailed description of illustrative embodiments taken in conjunction with the accompanying drawings in which:

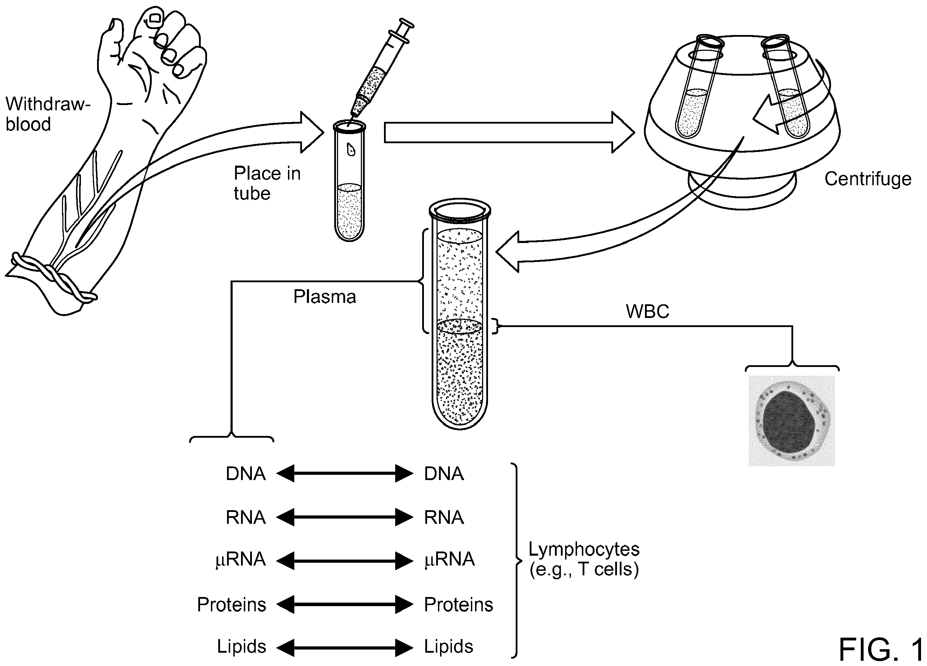

Figure 1 schematically depicts a proposed method leading to identification of disease-/condition-specific DNA, RNA, protein, and/or lipid signatures within the plasma following comparisons and subtraction of patientspecific intrinsic signatures obtained from the DNA, RNA, proteins, and/or lipids isolated from non-phagocytic lymphocytes.

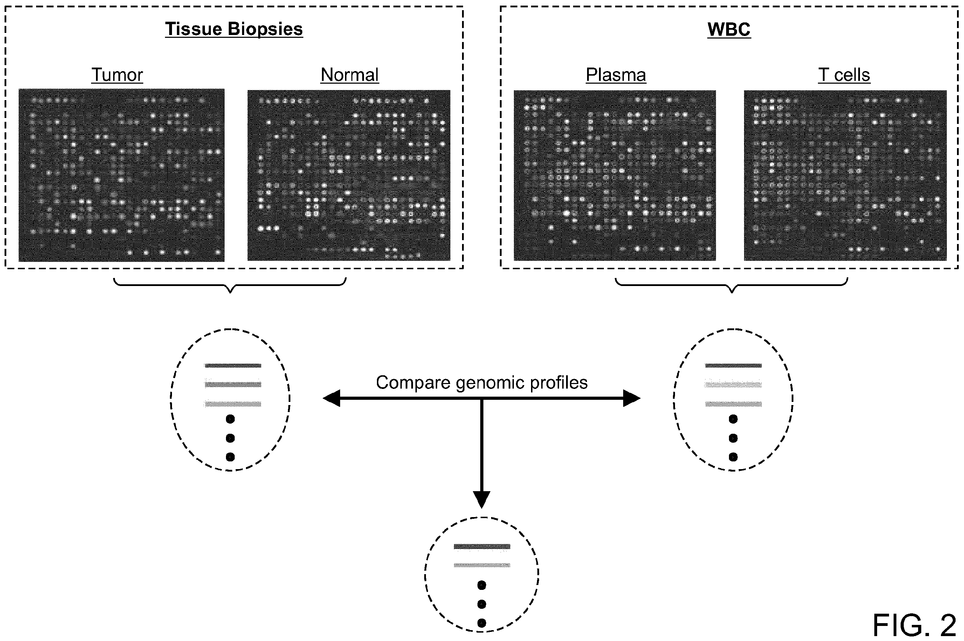

Figure 2 schematically depicts an analytical method used in the identification of tumor-specific signatures expressed in plasma of a cancer patient.

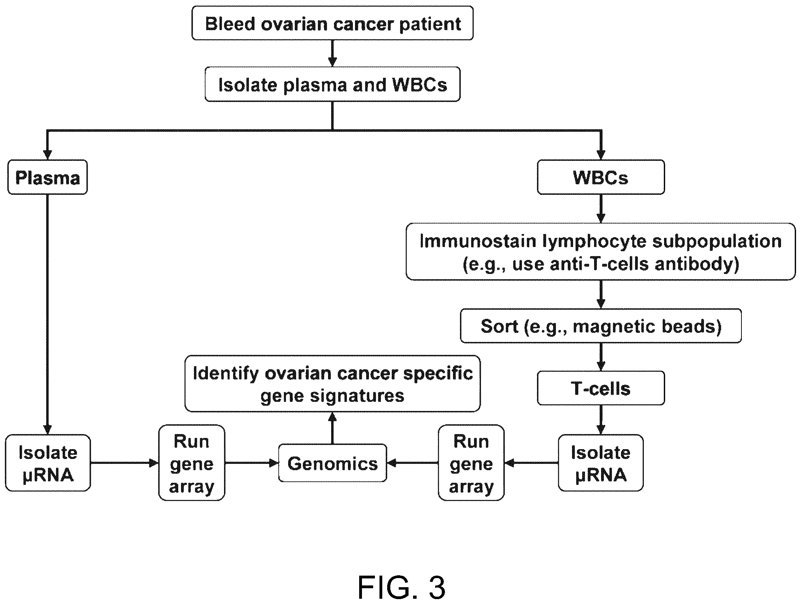

Figure 3 schematically depicts a general flowchart of one embodiment of a method of the invention.

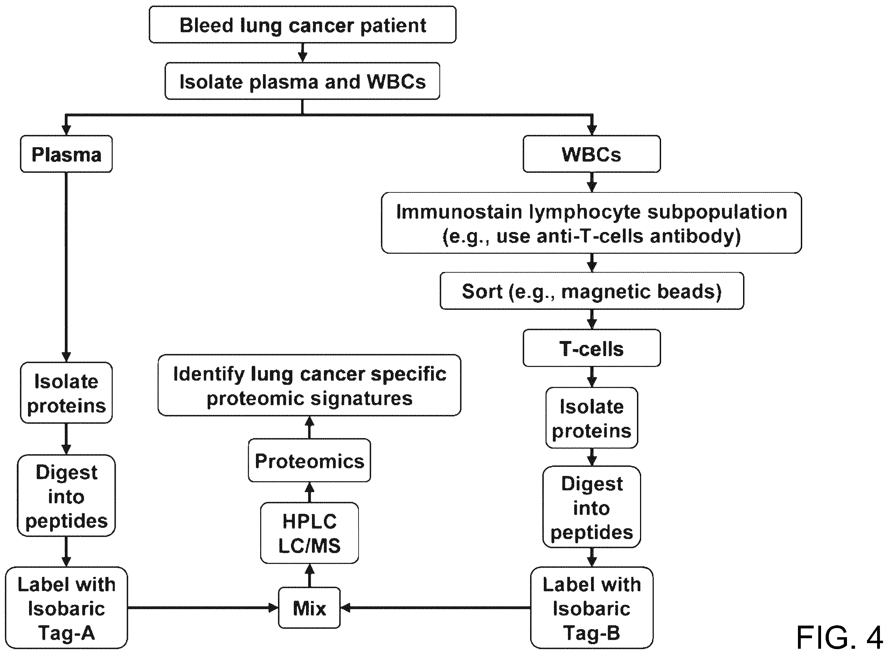

Figure 4 schematically depicts a general flowchart of another embodiment of a method of the invention.

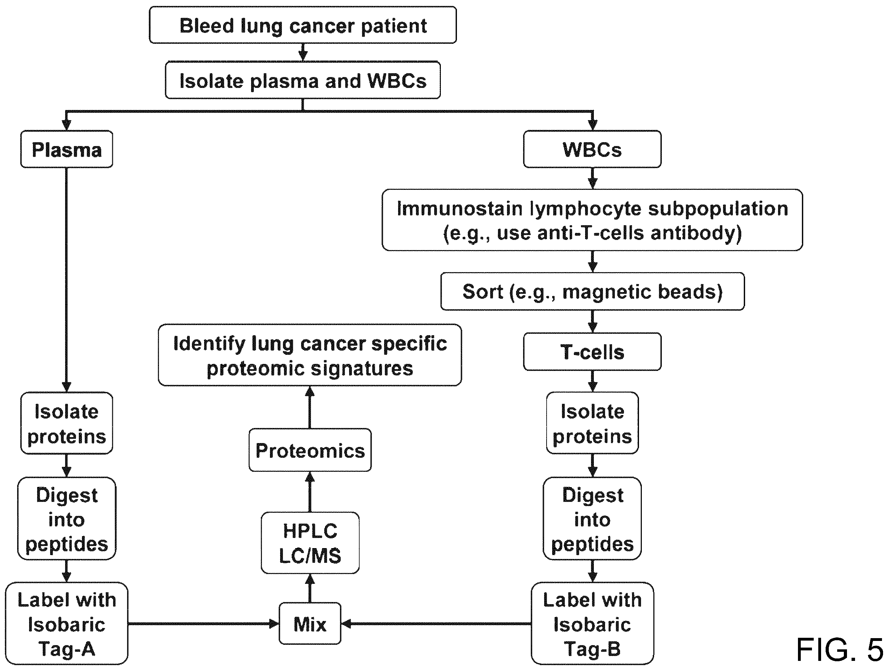

Figure 5 schematically depicts a general flowchart of yet another embodiment of a method of the invention.

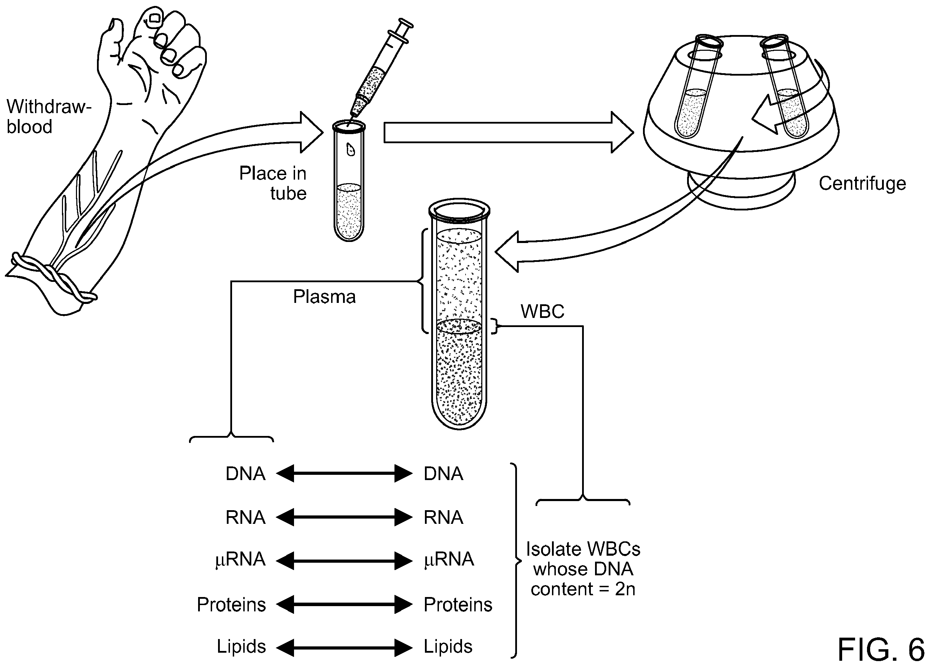

Figure 6 schematically depicts a proposed pathway leading to identification of disease-/ condition-specific DNA, RNA, protein and lipid signatures within the plasma following comparisons and subtraction of patientspecific intrinsic signatures obtained from the DNA, RNA, proteins, and/or lipids isolated from WBCs with a DNA content of 2n.

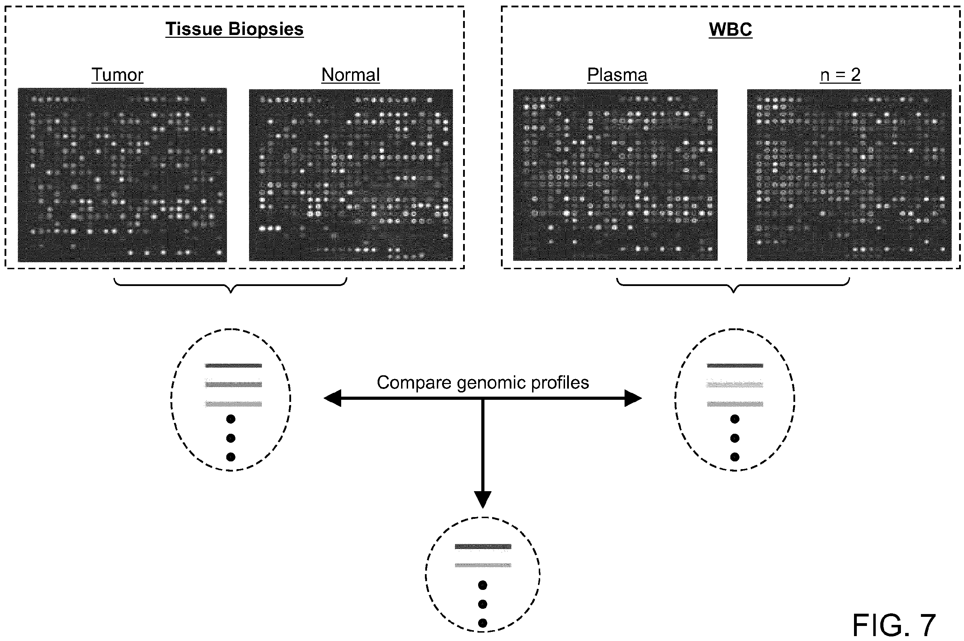

Figure 7 schematically depicts analytical approaches used in the identification of tumor-specific signatures in a cancer patient.

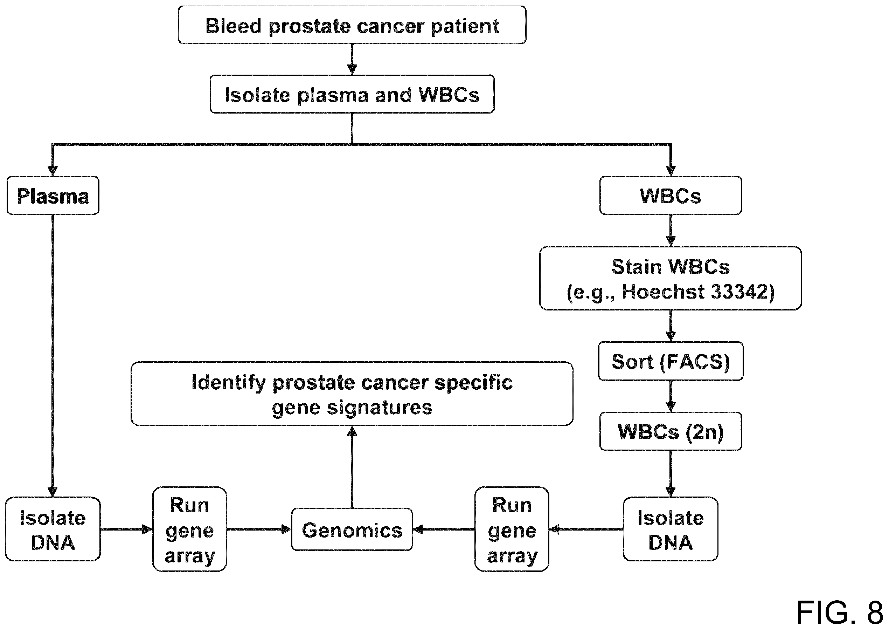

Figure 8 schematically depicts a general flowchart of another embodiment of a method of the invention.

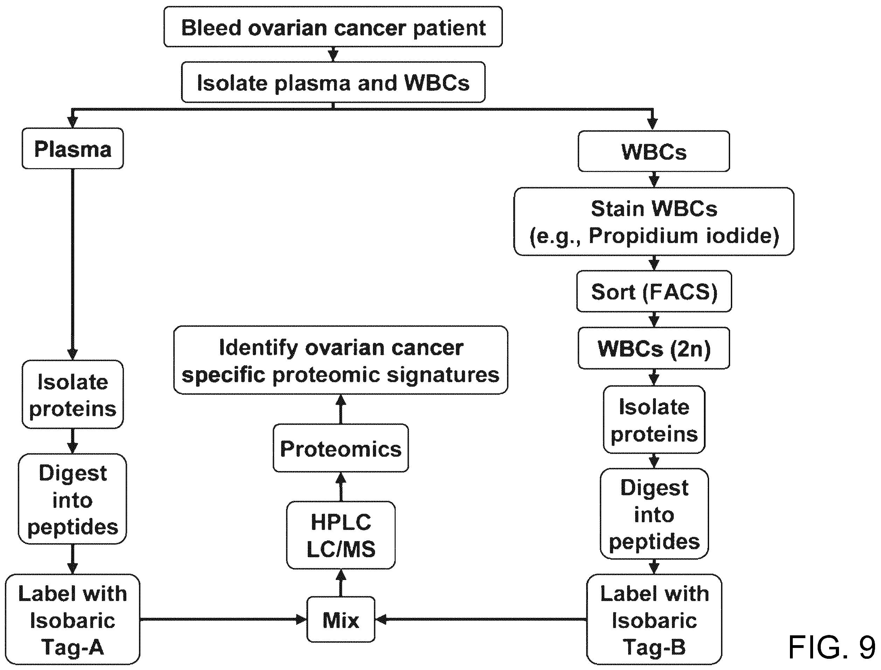

Figure 9 schematically depicts a general flowchart of another one embodiment of a method of the invention.

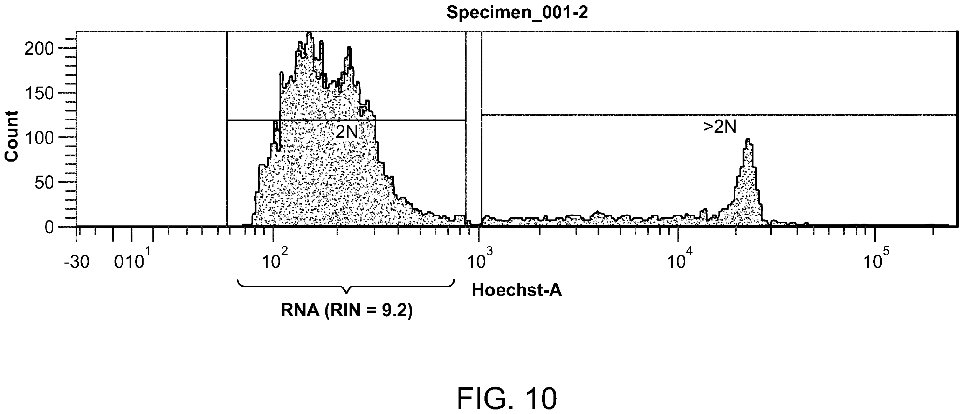

Figure 10 shows a FACS profile of human WBCs previously stained with Hoechst 33342 demonstrating the isolation of diploid WBCs as well as the results of the high quality of RNA extracted from these cells (RIN = 9.2).

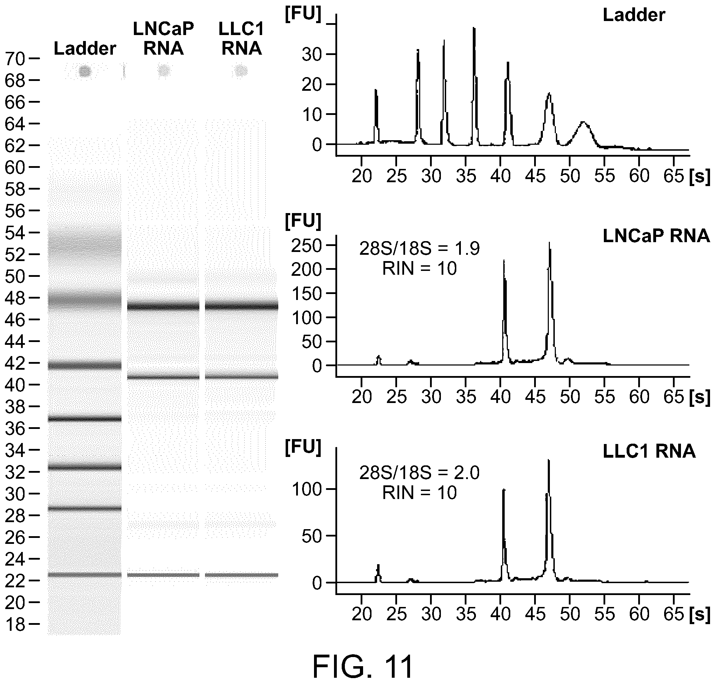

Figure 11 depicts gel electrophoresis analysis of total RNA isolated from LNCaP and LLC1 cells.

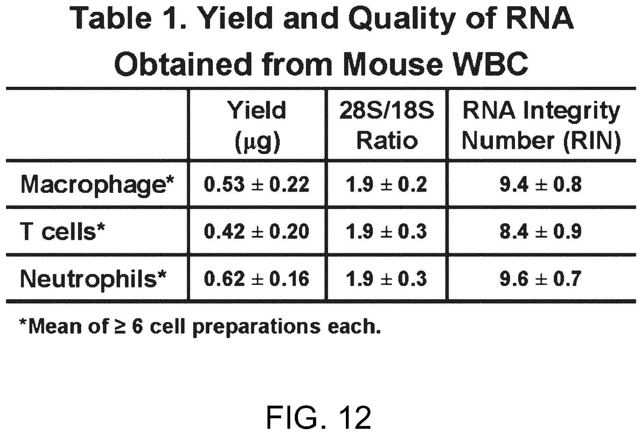

Figure 12 lists the yield and quality of RNA obtained from mouse white blood cells (WBCs).

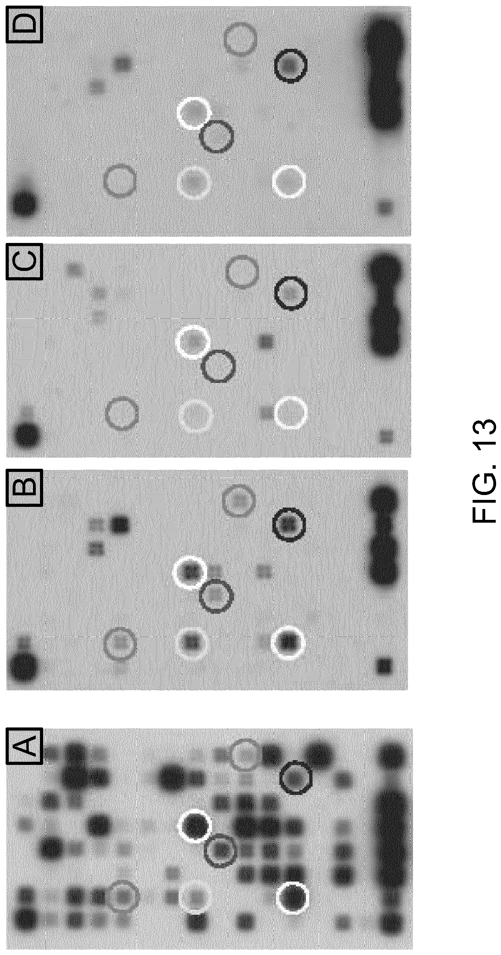

Figures 13A-13D depict arrays showing seven up-regulated (≥2 fold), cancer related genes detected in neutrophils from LNCaP (human prostate cancer) tumor-bearing nude mice. (A) LNCaP tumor. (B) Neutrophils obtained from nude mice bearing LNCaP tumors (NT). (C) T cells obtained from nude mice bearing LNCaP tumors (TT). (D) Neutrophils obtained from non-tumor-bearing nude mice (NN). Circled signatures expressed in tumor cells (A) and in neutrophils from tumor-bearing mice (B), and minimally expressed in neutrophils from non-tumor-bearing mice (D), and in non-phagocytic T cells (C). Expression in NT was ≥2-fold than that in NN and TT.

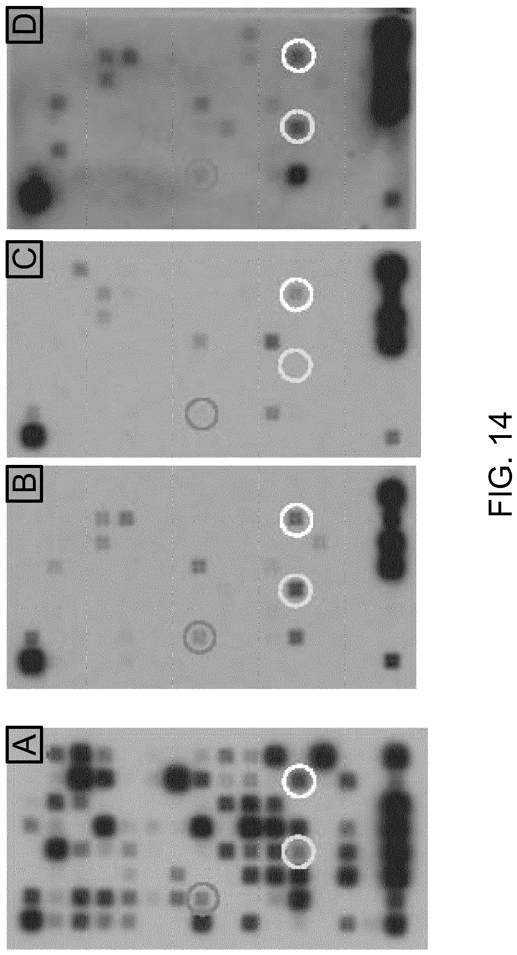

Figures 14A-14D depict arrays showing three up-regulated, cancer related genes detected in macrophages from LNCaP (human prostate cancer) tumor-bearing nude mice. (A) LNCaP tumor. (B) macrophages obtained from nude mice bearing LNCaP tumors (MT). (C) T cells obtained from nude mice bearing LNCaP tumors (TT). (D) macrophages obtained from non-tumor-bearing nude mice (MN). Circled signatures expressed in tumor cells (A) and in macrophages from tumor-bearing mice (B), and minimally expressed in macrophages from non-tumor-bearing mice (D), and in non-phagocytic T cells (C). Expression in MT was ≥2-fold than that in MN and TT.

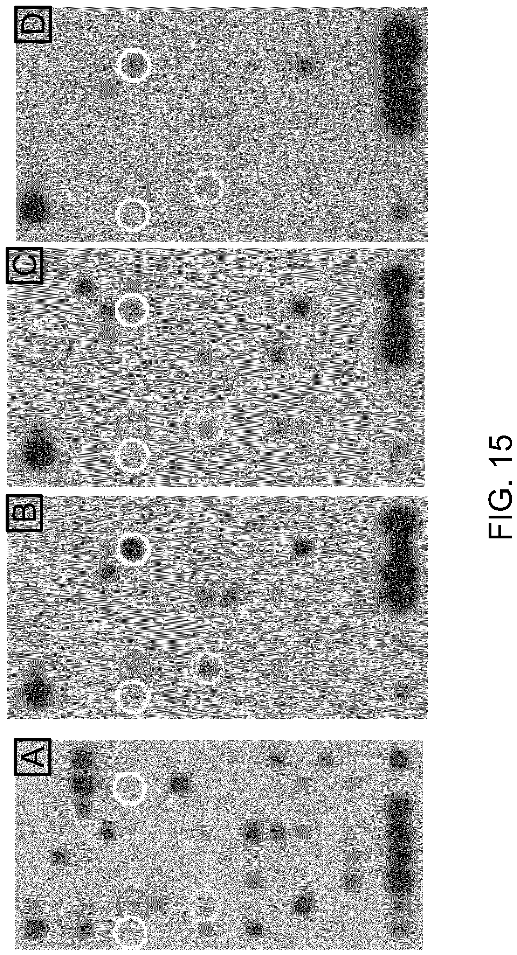

Figures 15A-15D depict arrays showing four up-regulated (≥2 fold), cancer related genes detected in neutrophils from LS174T (human colon cancer) tumor-bearing nude mice. (A) LS174T tumor. (B) Neutrophils obtained from nude mice bearing LS174T tumors (NT). (C) T cells obtained from nude mice bearing LS174T tumors (TT). (D) Neutrophils obtained from non-tumor-bearing nude mice (NN). Circled signatures expressed in tumor cells (A) and in neutrophils from tumor-bearing mice (B), and minimally expressed in neutrophils from non-tumor-bearing mice (D), and in non-phagocytic T cells (C). Expression was NT is ≥2-fold than that in NN and TT.

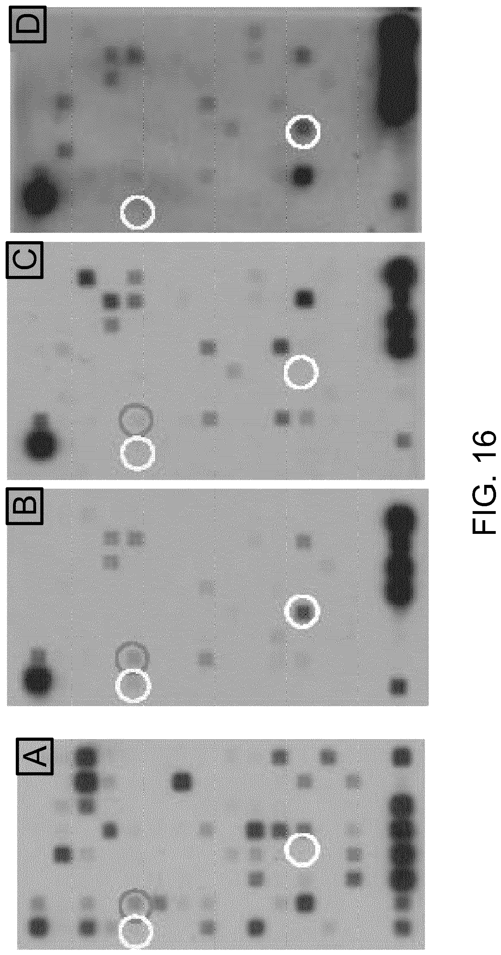

Figures 16A-16D depict arrays showing three up-regulated (≥2 fold), cancer related genes detected in macrophages from LS174T (human colon cancer) tumor-bearing nude mice. (A) LS174T tumor. (B) Macrophages obtained from nude mice bearing LS174T tumors (MT). (C) T cells obtained from nude mice bearing LS174T tumors (TT). (D) Macrophages obtained from non-tumor-bearing nude mice (MN). Circled signatures expressed in tumor cells (A) and in macrophages from tumor-bearing mice (B), and minimally expressed in macrophages from non-tumor-bearing mice (D), and in non-phagocytic T cells (C). Expression in MT is ≥2-fold than that in MN and TT.

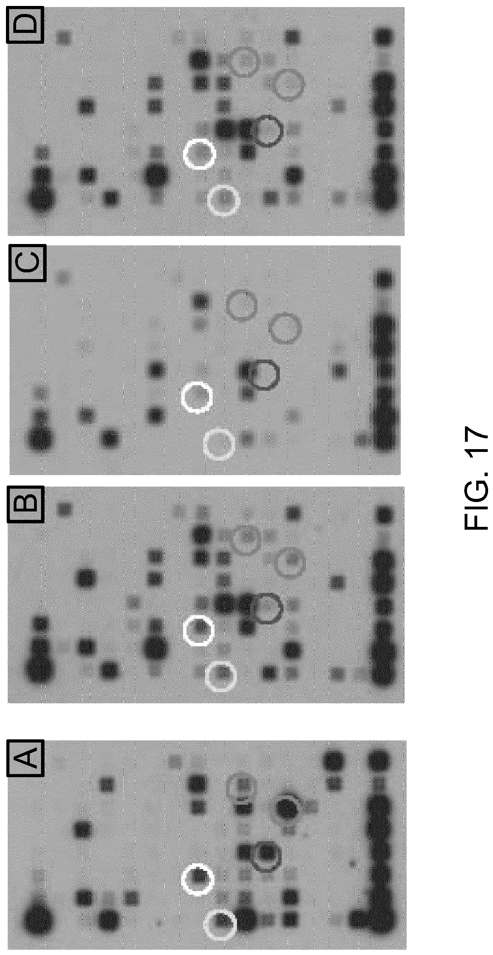

Figures 17A-17D depict arrays showing five up-regulated (≥2 fold), cancer related genes detected in neutrophils from LLC1 (mouse metastatic lung cancer) tumor-bearing C57/B1 mice. (A) LLC1 tumor. (B) Neutrophils obtained from C57/Bl mice bearing LLC1 tumors (NT). (C) T cells obtained from C57/Bl mice bearing LLC1 tumors (TT). (D) Neutrophils obtained from non-tumor-bearing C57/Bl mice (NN). Circled signatures expressed in tumor cells (A) and in neutrophils from tumor-bearing mice (B), and minimally expressed in neutrophils from non-tumor-bearing mice (D), and in non-phagocytic T cells (C). Expression in NT was ≥2-fold than that in NN and TT.

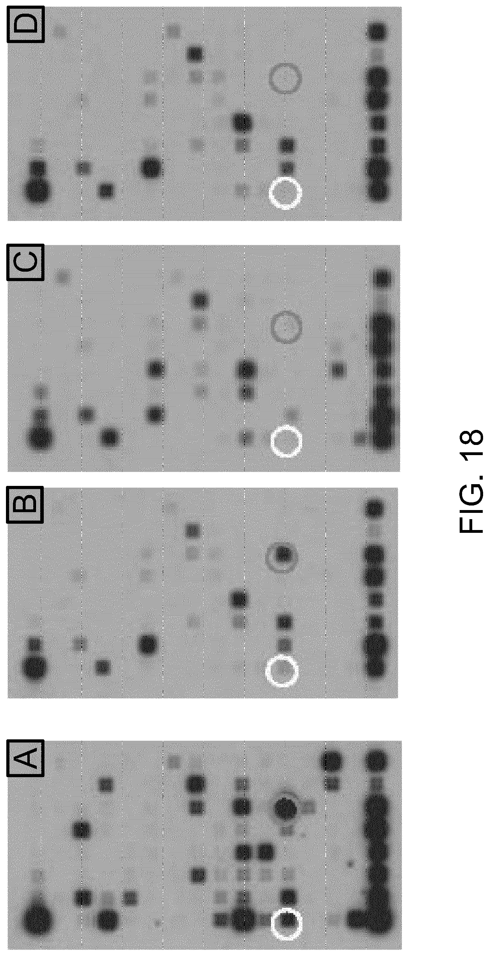

Figures 18A-18D depict arrays showing two up-regulated (≥2 fold), cancer related genes detected in macrophages from LLC1 (mouse metastatic lung cancer) tumor-bearing C57/B1 mice. (A) LLC1 tumor. (B) Macrophages obtained from C57/Bl mice bearing LLC1 tumors (MT). (C) T cells obtained from C57/Bl mice bearing LLC1 tumors (TT). (D) Macrophages obtained from non-tumor-bearing C57/B1 mice (MN). Circled signatures expressed in tumor cells (A) and in neutrophils from tumor-bearing mice (B), and minimally expressed in neutrophils from non-tumor-bearing mice (D), and in non-phagocytic T cells (C). Expression in MT was ≥2-fold than that in MN and TT.

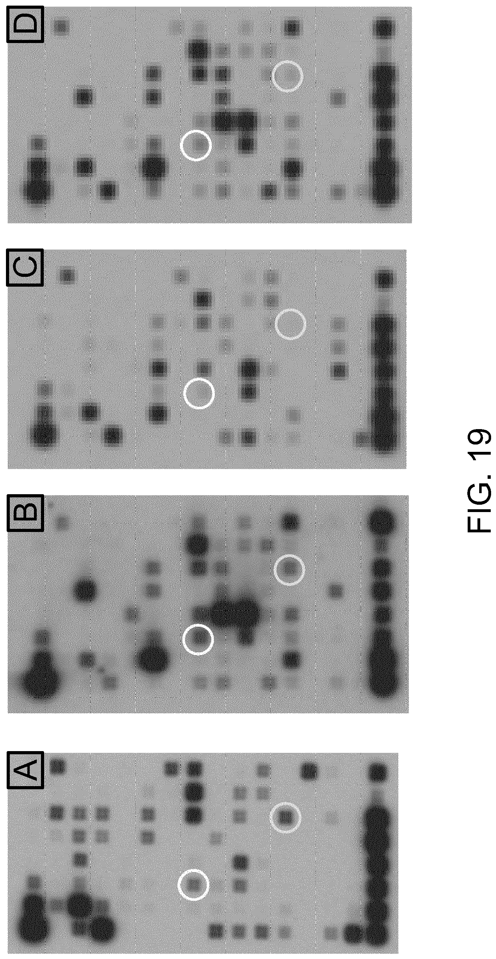

Figure 19A-19D depict arrays showing two up-regulated (≥2 fold), cancer related genes detected in neutrophils from B16F10 (mouse metastatic melanoma) tumor bearing C57/B1 mice. (A) B16F10 tumor. (B) Neutrophils obtained from C57/Bl mice bearing B16F10 tumors (NT). (C) T cells obtained from C57/B1 mice-bearing B16F10 tumors (TT). (D) Neutrophils obtained from non-tumor-bearing C57/Bl mice (NN). Circled signatures expressed in tumor cells (A) and in neutrophils from tumor-bearing mice (B), and minimally expressed in neutrophils from non-tumor-bearing mice (D), and in non-phagocytic T cells (C). Expression in NT was ≥2-fold than that in NN and TT.

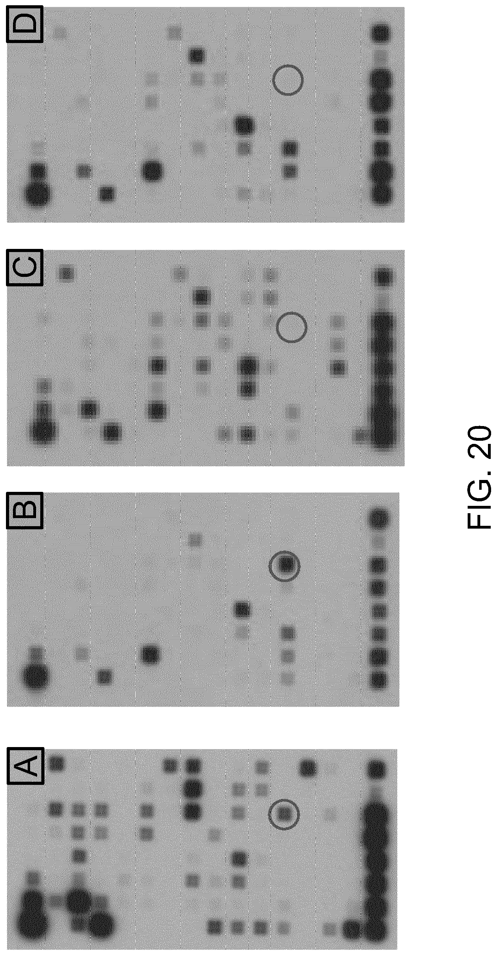

Figure 20A-20D depict arrays showing one up-regulated (≥2 fold), cancer related genes detected in macrophages from B16F10 (mouse metastatic melanoma) tumor-bearing C57/B1 mice. (A) B16F10 tumor. (B) Macrophages obtained from C57/Bl mice bearing B16F10 tumors (MT). (C) T cells obtained from C57/Bl mice bearing B16F10 tumors (TT). (D: Macrophages obtained from non-tumor-bearing C57/Bl mice (MN). Circled signatures expressed in tumor cells (A) and in macrophages from tumor-bearing mice (B), and minimally expressed in macrophages from non-tumor-bearing mice (D), and in non-phagocytic T cells (C). Expression in MT was ≥2-fold than that in MN and TT.

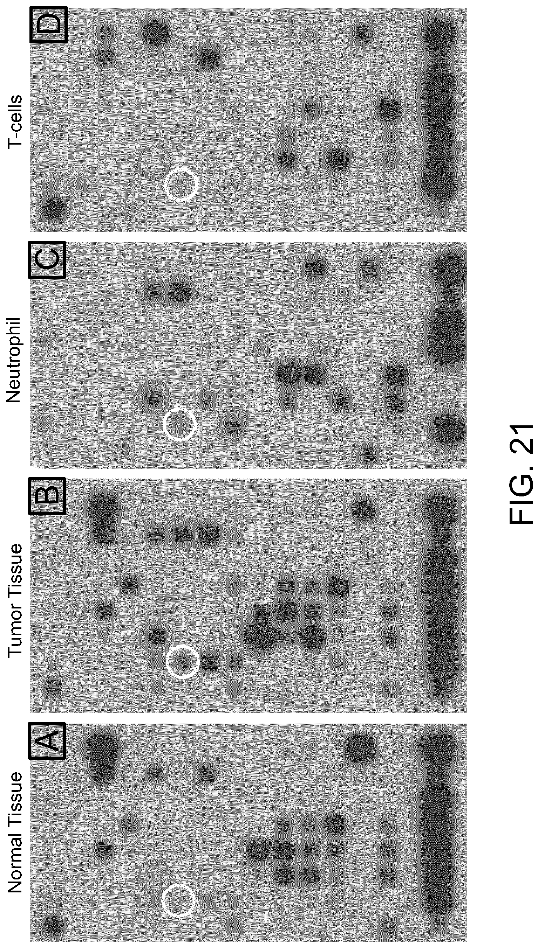

Figure 21A-21D depict arrays showing five up-regulated (≥2 fold), cancer related genes detected in neutrophils from patient with head and neck cancer (squamous cell carcinoma). (A) Normal tissue (skin) biopsy. (B) Tumor tissue biopsy. (C) Neutrophils obtained from patient blood (NT). (D) T cells obtained from patient blood (TT). Circled signatures expressed in tumor cells (B) and in neutrophils from patient blood (C), and minimally expressed or not expressed in normal skin (A) or non-phagocytic T cells (D). Expression in NT was ≥2-fold than that in TT and skin.
Figure 22A-22D depict arrays showing 23 up-regulated (≥2 fold), cancer related genes detected in macrophages from patient with ovarian cancer (adenocarcinoma). (A) Macrophages obtained from patient blood (MT). (B) T cells obtained from patient blood (TT). Circled signatures expressed in macrophages from patient (A) and minimally expressed in non-phagocytic T cells (B). Expression in MT was ≥2-fold than that in TT.

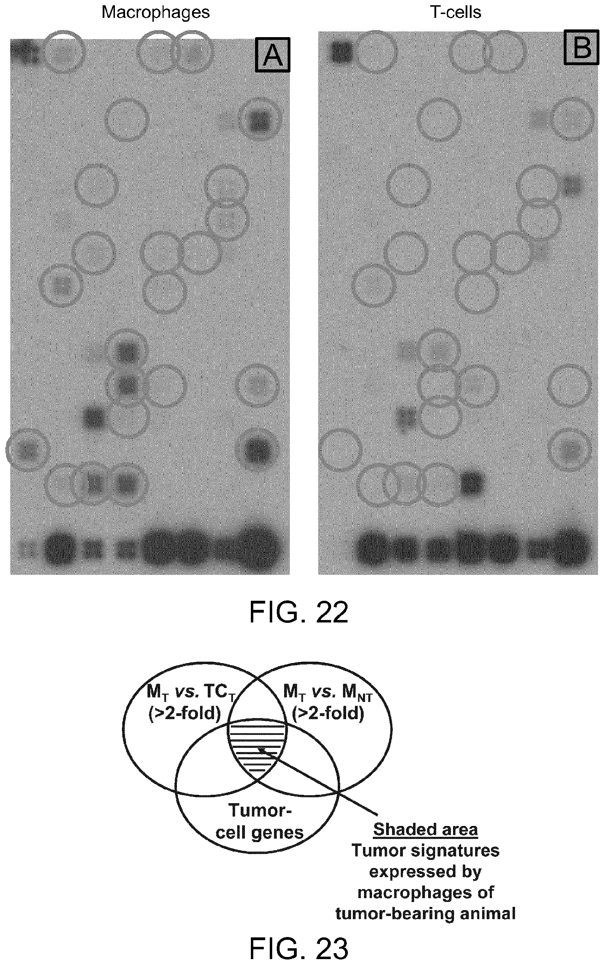

Figure 23 depicts a method used to identify tumor signatures in phagocytic cells. In this example, expression intensities of cancer associated genes in macrophages from tumor-bearing animals (MT) were quantified compared to those from T cells from the same animals (TT) and those overexpressed by >2-fold identified. Next, the intensities of all expressed genes in MT were quantified and compared to those in macrophages obtained from non-tumor bearing animals (MNT) and the genes overexpressed >2-fold were identified. The genes common to both lists were selected and compared to those expressed by the same tumor (shaded area).

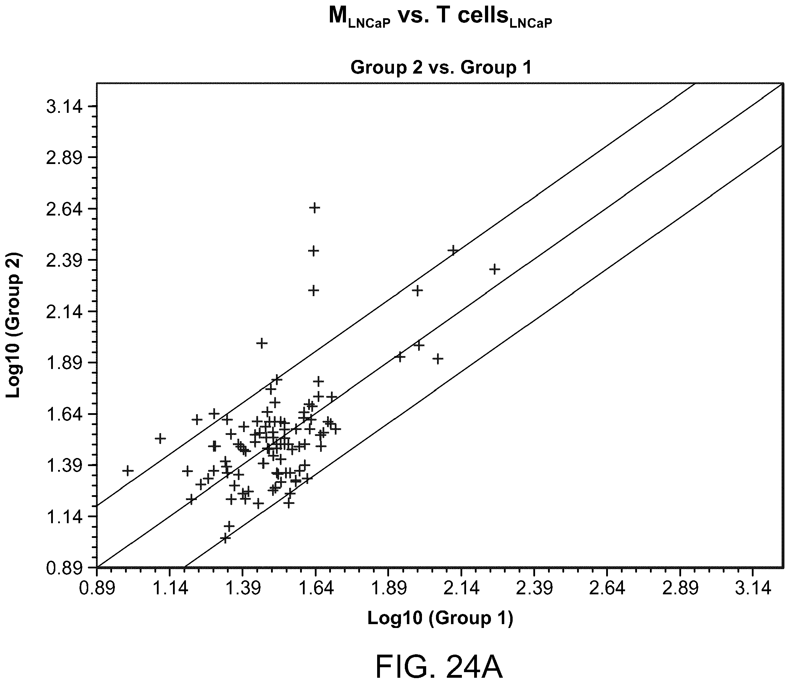

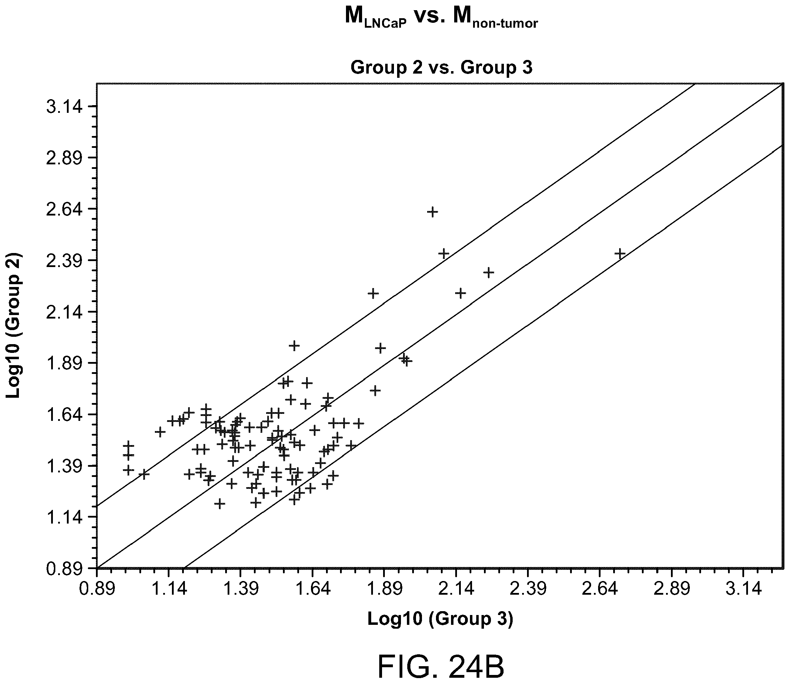

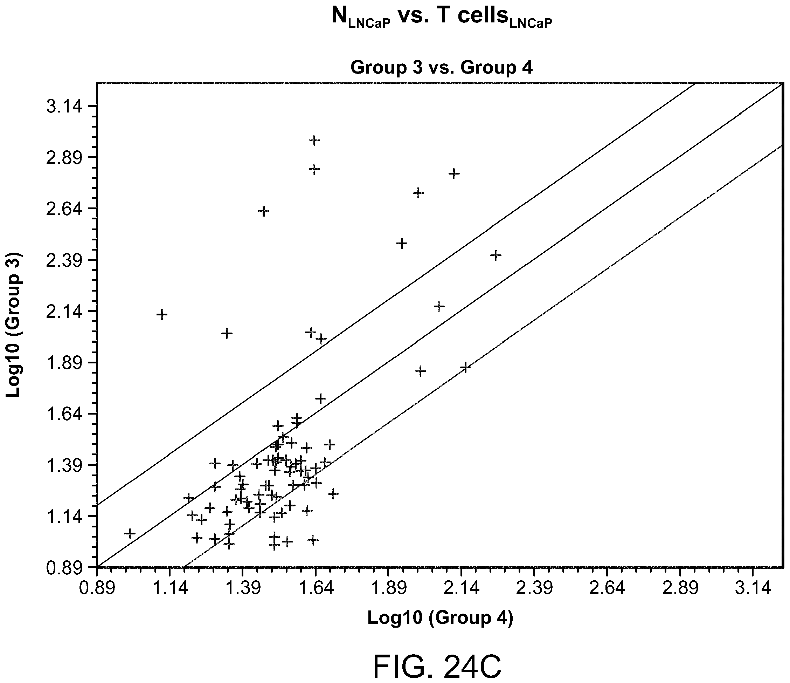

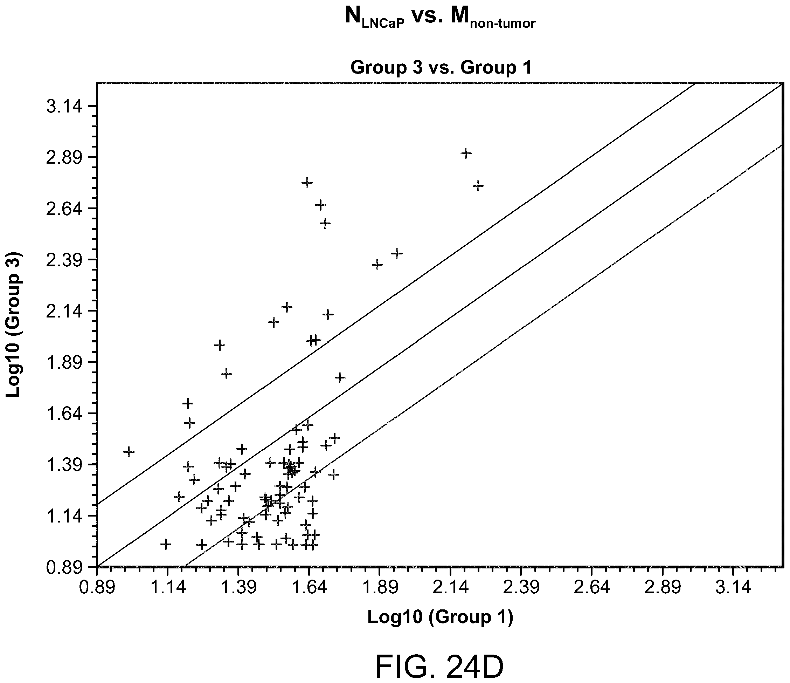

Figures 24A-24B depict gene expression intensity comparisons in (A) macrophages obtained from nude mice bearing LNCaP human prostate tumors (MLNCaP) and T cells from the same animals (T cellsLNCaP), (B) MLNCaP and macrophages obtained from non-tumor-bearing mice (Mnon-tumor), (C) neutrophils obtained from nude mice bearing LNCaP human prostate tumors (NLNCaP) and T cells from the same animals (T cellsLNCaP), and (D) NLNCaP and macrophages obtained from non-tumor-bearing mice (Nnon-tumor). Genes in red were overexpressed >2 fold; those in green were under-expressed >2 fold.

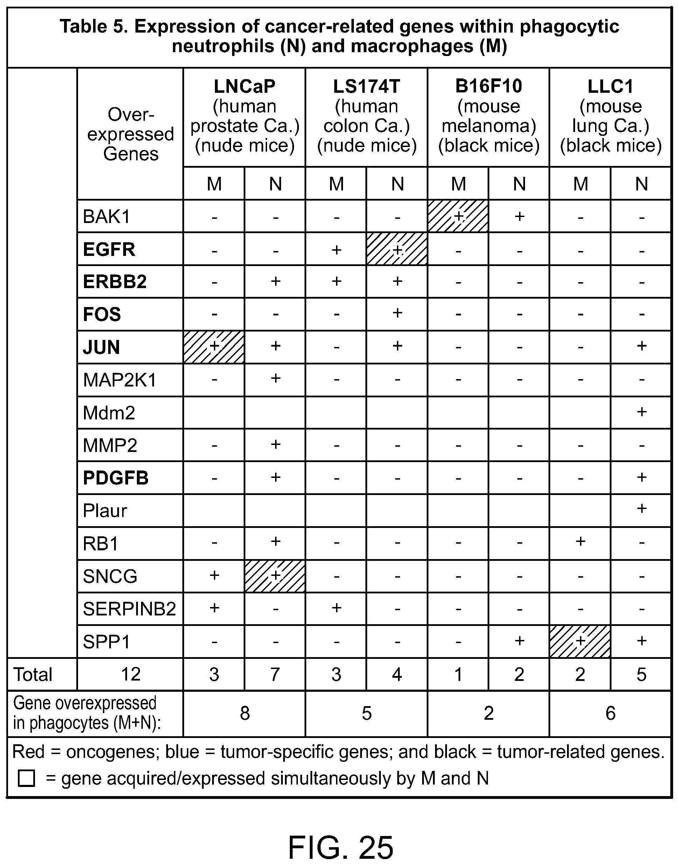

Figure 25 lists expression of cancer-related genes within phagocytic neutrophils (N) and macrophages (M).

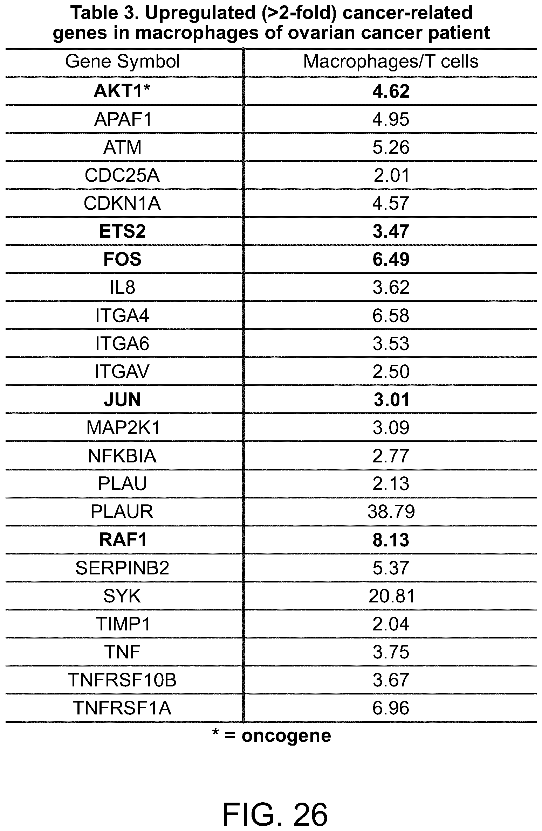

Figure 26 lists cancer-related genes upregulated (>2-fold) in phagocytic macrophages of a patient with ovarian cancer in comparison to non-phagocytic T cells.

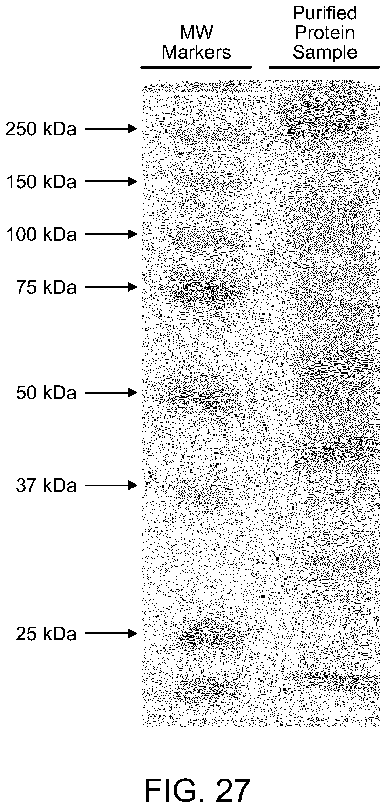

Figure 27 depicts SDS gel (10%) electrophoresis of protein sample (5.9 µg) obtained from mouse WBC.

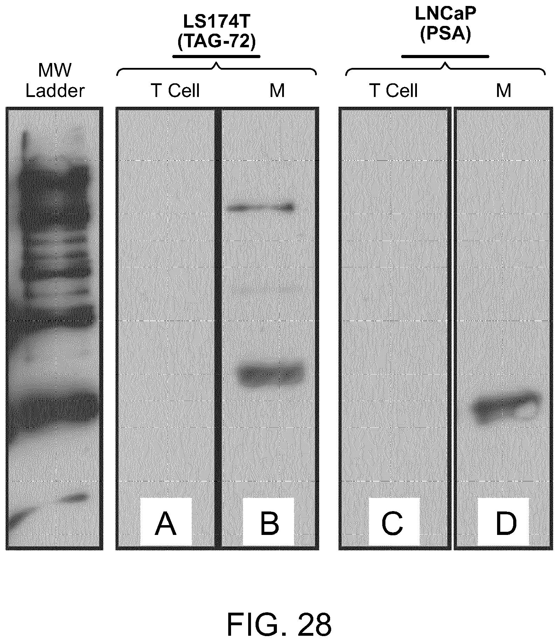

Figure 28 depicts Western blot analysis of TAG-72 and PSA expression in T cells and monocytes/macrophages (M/M) obtained from tumor-bearing mice, illustrating the presence of signatures in phagocytic cells only.

### Detailed Description of the Invention

Unless otherwise defined herein, scientific and technical terms used in this application shall have the meanings that are commonly understood by those of ordinary skill in the art. Generally, nomenclature used in connection with, and techniques of, cell and tissue culture, molecular biology, cell and cancer biology, neurobiology, neurochemistry, virology, immunology, microbiology, pharmacology, genetics and protein and nucleic acid chemistry, described herein, are those well-known and commonly used in the art.

All of the above, and any other publications, patents and published patent applications referred to in this application are specifically incorporated by reference herein. In case of conflict, the present specification, including its specific definitions, will control.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

A "patient", "subject", or "individual" are used interchangeably and refer to either a human or a non-human animal. These terms include mammals, such as humans, primates, livestock animals (e.g., bovines, porcines), companion animals (e.g., canines, felines) and rodents (e.g., mice and rats).

As used herein, a control subject refers to any individual that has not been diagnosed as having the disease or condition being assayed. The terms "normal control", "healthy control", and "not-diseased cells" likewise mean a sample (e.g., cells, serum, tissue) taken from a source (e.g., subject, control subject, cell line) that does not have the condition or disease being assayed and therefore may be used to determine the baseline for the condition or disorder being measured. It is also understood that the control subject, normal control, and healthy control, include data obtained and used as a standard, i.e. it can be used over and over again for multiple different subjects. In other words, for example, when comparing a subject sample to a control sample, the data from the control sample could have been obtained in a different set of experiments, for example, it could be an average obtained from a number of healthy subjects and not actually obtained at the time the data for the subject was obtained.

The term "diagnosis" as used herein refers to methods by which the skilled artisan can estimate and/or determine whether or not a patient is suffering from a given disease or condition. The skilled artisan often makes a diagnosis on the basis of one or more diagnostic indicators, e.g., a marker, the presence, absence, amount, or change in amount of which is indicative of the presence, severity, or absence of the condition.

The term "prognosis" as used herein refers to is used herein to refer to the likelihood of a disease or condition progression, including recurrence of a disease or condition.

The disclosure of the International Application PCT/US2009/031395 is incorporated herein by reference for all purposes.

### Description of Methods of the Invention

The present invention provides methods for diagnosing or aiding in the diagnosis of a disease or condition by comparing profiles (e.g., gene/protein/lipid/carbohydrate expression profiles, genotypes, gene copy number, gene dosage, DNA methylation, etc.) of disease or condition-associated markers (e.g., nucleic acids, proteins, lipids, carbohydrates, metabolites) between cell-free bodily fluids and non-phagocytic cells taken from the same individual, or between cell-free bodily fluids and phagocytic cells which have not yet phagocytosed cells or cellular components from the blood, i.e., phagocytic cells having a DNA content of 2n, taken from the same individual..

This invention also provides methods for assessing the risk of developing a disease or condition, prognosing said disease, monitoring said disease progression or regression, assessing the efficacy of a treatment, or identifying a compound capable of ameliorating or treating said disease or condition.

Such a subject-specific profile comparison eliminates the dependence on a population-derived average profile for a particular disease or condition, which may introduce error into the detection or diagnosis of a particular disease or condition in the subject. The methods of this invention allow detection, diagnosis, and treatment to be personalized to the individual.

The methods of this invention (i) have high specificity, sensitivity, and accuracy and are capable of detecting disease or condition-specific markers present within a bodily fluid sample, cells or tissues; and (ii) eliminate the "inequality of baseline" that is known to occur among individuals due to intrinsic (e.g., age, gender, ethnic background, health status and the like) and temporal variations in marker expression. Accordingly, in certain aspects, the invention provides non-invasive assays for the early detection of a disease or condition, i.e., before the disease can be diagnosed by conventional diagnostic techniques, e.g., imaging techniques, and, therefore, provide a foundation for improved decisionmaking relative to the needs and strategies for intervention, prevention, and treatment of individuals with such disease or condition.

The methods of this invention can be used together with any known diagnostic methods, such as physical inspection, visual inspection, biopsy, scanning, histology, radiology, imaging, ultrasound, use of a commercial kit, genetic testing, immunological testing, analysis of bodily fluids, or monitoring neural activity.

In some embodiments, the phagocytic cells are professional phagocytic cells, such as neutrophils, macrophages, monocytes, dendritic cells, foam cells, mast cells, or eosinophils. In some embodiments, the phagocytic cells are non-professional phagocytic cells, such as epithelial cells, endothelial cells, fibroblasts, or mesenchymal cells. In other embodiments, the phagocytic cells can be a mixture of different types of phagocytic cells. Non-phagocytic cells that can be used in this invention include, but are not limited to, T cells, B cells, null cells, basophils, or mixtures thereof.

As used herein, "the =2n phagocytic cells" refer to phagocytic cells that have a DNA content of 2n. According to the present invention, some phagocytic cells have not engulfed living/dying/dead diseased cells or fragments and/or cell-free disease-specific nucleic acids, proteins, lipids, and/or carbohydrates present in bodily fluids. The DNA contents of this group of phagocytic cells remain 2n.

As used herein, a "profile" of a marker of a disease or condition can broadly refer to any information concerning the marker. This information can be either qualitative (e.g., presence or absence) or quantitative (e.g., levels, copy numbers, or dosages). In some embodiments, a profile of a marker can indicate the absence of this marker. The profile can be a nucleic acid (e.g., DNA or RNA) profile, a protein profile, a lipid profile, a carbohydrate profile, a metabolite profile, or a combination thereof. A "marker" as used herein generally refers to an analyte which is differentially detectable in phagocytes and is indicative of the presence of a disease or condition. An analyte is differentially detectable if it can be distinguished quantitatively or qualitatively in phagocytes.

The methods of this invention can be applied to various diseases or conditions.

The methods of this invention can be applied to various diseases or conditions. Exemplar diseases or conditions are a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a mitochondrial disorder, a respiratory-gastrointestinal tract disease or condition, a reproductive disease or condition, an ophthalmic disease or condition, a musculo-skeletal disease or condition, or a dermal disease or condition.

As used herein, the term "cardiovascular disease or condition" refers to any condition that affects systems of heart or blood vessels (arteries and veins). Examples of cardiovascular diseases include, but are not limited to myocardial infarction, coronary artery disease, percutaneous transluminal coronary angioplasty (PTCA), coronary artery bypass surgery (CABG), restenosis, peripheral arterial disease, stroke, abdominal aorta aneurysm, intracranial aneurysm, large artery atherosclerotic stroke, cardiogenic stroke, an early onset myocardial infarction, heart failure, pulmonary embolism, acute coronary syndrome (ACS), angina, cardiac hypertrophy, arteriosclerosis, myocarditis, pancarditis, endocarditis, hypertension, congestive heart failure, atherosclerosis, cerebrovascular disease, declining cardiac health, ischemic heart disease, pericarditis, cardiogenic shock, alcoholic cardiomyopathy, congenital heart disease, ischemic cardiomyopathy, hypertensive cardiomyopathy, valvular cardiomyopathy, inflammatory cardiomyopathy, cardiomyopathy secondary to a systemic metabolic disease, dilated cardiomyopathy, hypertrophic cardiomyopathy, arrhythmogenic right ventricular cardiomyopathy, restrictive cardiomyopathy, noncompaction cardiomyopathy, valvular heart disease, hypertensive heart disease, myocardial ischemic attack, unstable angina, myocardial rupture, cardiogenic shock, embolism, deep vein thrombosis, arrhythmia, arrhythmogenic right ventricular cardiomyopathy, diabetic cardiomyopathy, mitral regurgitation, mitral valve prolapse, peripheral vascular disease, artery disease, carotid artery disease, deep vein thrombosis, venous diseases, cerebrovascular disease, arterial aneurysm, left ventricular hypertrophy, hypertensive renal disease, hypertensive retinal disease, vasculitis, left main disease, arterial vascular disease, venous vascular disease, thrombosis of the microcirculation, a transient cerebrovascular accident, limb ischemia, aneurysm, thrombosis, superficial venous thrombosis, and deep venous thrombosis.

As used herein, the term "kidney-associated disease or condition" refers to any disease or condition that affects kidney or renal system. Examples of kidney-associated disease include, but are not limited to, chronic kidney diseases, primary kidney diseases, non-diabetic kidney diseases, glomerulonephritis, interstitial nephritis, diabetic kidney diseases, diabetic nephropathy, glomerulosclerosis, rapid progressive glomerulonephritis, renal fibrosis, Alport syndrome, IDDM nephritis, mesangial proliferative glomerulonephritis, membrano proliferative glomerulonephritis, crescentic glomerulonephritis, renal insterstitial fibrosis, focal segmental glomerulosclerosis, membranous nephropathy, minimal change disease, pauci-immune rapid progressive glomerulonephritis, IgA nephropathy, polycystic kidney disease, Dent's disease, nephrocytinosis, Heymann nephritis, autosomal dominant (adult) polycystic kidney disease, autosomal recessive (childhood) polycystic kidney disease, acute kidney injury, nephrotic syndrome, renal ischemia, podocyte diseases or disorders, proteinuria, glomerular diseases, membranous glomerulonephritis, focal segmental glomerulonephritis, preeclampsia, eclampsia, kidney lesions, collagen vascular diseases, benign orthostatic (postural) proteinuria, IgM nephropathy, membranous nephropathy, sarcoidosis, diabetes mellitus, kidney damage due to drugs, Fabry's disease, aminoaciduria, Fanconi syndrome, hypertensive nephrosclerosis, interstitial nephritis, Sickle cell disease, hemoglobinuria, myoglobinuria, Wegener's Granulomatosis, Glycogen Storage Disease Type 1, chronic kidney disease, chronic renal failure, low Glomerular Filtration Rate (GFR), nephroangiosclerosis, lupus nephritis, ANCA-positive pauci-immune crescentic glomerulonephritis, chronic allograft nephropathy, nephrotoxicity, renal toxicity, kidney necrosis, kidney damage, glomerular and tubular injury, kidney dysfunction, nephritic syndrome, acute renal failure, chronic renal failure, proximal tubal dysfunction, acute kidney transplant rejection, chronic kidney transplant refection, non IgA mesangioproliferative glomerulonephritis, postinfectious glomerulonephritis, vasculitides with renal involvement of any kind, any hereditary renal disease, any interstitial nephritis, renal transplant failure, kidney cancer, kidney disease associated with other conditions (e.g., hypertension, diabetes, and autoimmune disease), Dent's disease, nephrocytinosis, Heymann nephritis, a primary kidney disease, a collapsing glomerulopathy, a dense deposit disease, a cryoglobulinemia-associated glomerulonephritis, an Henoch-Sch6nlein disease, a postinfectious glomerulonephritis, a bacterial endocarditis, a microscopic polyangitis, a Churg-Strauss syndrome, an anti-GBM-antibidy mediated glomerulonephritis, amyloidosis, a monoclonal immunoglobulin deposition disease, a fibrillary glomerulonephritis, an immunotactoid glomerulopathy, ischemic tubular injury, a medication-induced tubulo-interstitial nephritis, a toxic tubulo-interstitial nephritis, an infectious tubulo-interstitial nephritis, a bacterial pyelonephritis, a viral infectious tubulo-interstitial nephritis which results from a polyomavirus infection or an HIV infection, a metabolic-induced tubulo-interstitial disease, a mixed connective disease, a cast nephropathy, a crystal nephropathy which may results from urate or oxalate or drug-induced crystal deposition, an acute cellular tubulo-interstitial allograft rejection, a tumoral infiltrative disease which results from a lymphoma or a post-transplant lymphoproliferative disease, an obstructive disease of the kidney, vascular disease, a thrombotic microangiopathy, a nephroangiosclerosis, an atheroembolic disease, a mixed connective tissue disease, a polyarteritis nodosa, a calcineurin-inhibitor induced- vascular disease, an acute cellular vascular allograft rejection, an acute humoral allograft rejection, early renal function decline (ERFD), end stage renal disease (ESRD), renal vein thrombosis, acute tubular necrosis, acute interstitial nephritis, established chronic kidney disease, renal artery stenosis, ischemic nephropathy, uremia, drug and toxin-induced chronic tubulointerstitial nephritis, reflux nephropathy, kidney stones, Goodpasture's syndrome, and hydronephrosis.

As used herein, the term "prenatal or pregnancy-related disease or condition" refers to any disease, disorder, or condition affecting a pregnant woman, embryo, or fetus. Prenatal or pregancy-related conditions can also refer to any disease, disorder, or condition that is associated with or arises, either directly or indirectly, as a result of pregnancy. These diseases or conditions can include any and all birth defects, congenital conditions, or hereditary diseases or conditions. Examples of prenatal or pregnancy-related diseases include, but are not limited to, Rhesus disease, hemolytic disease of the newborn, beta-thalassemia, sex determination, determination of pregnancy, a hereditary Mendelian genetic disorder, chromosomal aberrations, a fetal chromosomal aneuploidy, fetal chromosomal trisomy, fetal chromosomal monosomy, trisomy 8, trisomy 13 (Patau Syndrom), trisomy 16, trisomy 18 (Edwards syndrome), trisomy 21 (Down syndrome), X-chromosome linked disorders, trisomy X (XXX syndrome), monosomy X (Turner syndrome), XXY syndrome, XYY syndrome, XYY syndrome, XXXY syndrome, XXYY syndrome, XYYY syndrome, XXXXX syndrome, XXXXY syndrome, XXXYY syndrome, XXYYY syndrome, Fragile X Syndrome, fetal growth restriction, cystic fibrosis, a hemoglobinopathy, fetal death, fetal alcohol syndrome, sickle cell anemia, hemophilia, Klinefelter syndrome, dup(17)(p11.2p1.2) syndrome, endometriosis, Pelizaeus-Merzbacher disease, dup(22)(q11.2q11.2) syndrome, cat eye syndrome, cri-du-chat syndrome, Wolf-Hirschhorn syndrome, Williams-Beuren syndrome, Charcot-Marie-Tooth disease, neuropathy with liability to pressure palsies, Smith-Magenis syndrome, neurofibromatosis, Alagille syndrome, Velocardiofacial syndrome, DiGeorge syndrome, steroid sulfatase deficiency, Prader-Willi syndrome, Kallmann syndrome, microphthalmia with linear skin defects, adrenal hypoplasia, glycerol kinase deficiency, Pelizaeus-Merzbacher disease, testis-determining factor on Y, azospermia (factor a), azospermia (factor b), azospermia (factor c), 1p36 deletion, phenylketonuria, TaySachs disease, adrenal hyperplasia, Fanconi anemia, spinal muscular atrophy, Duchenne's muscular dystrophy, Huntington's disease, myotonic dystrophy, Robertsonian translocation, Angelman syndrome, tuberous sclerosis, ataxia telangieltasia, open spina bifida, neural tube defects, ventral wall defects, small-for-gestational-age, congenital cytomegalovirus, achondroplasia, Marfan's syndrome, congenital hypothyroidism, congenital toxoplasmosis, biotinidase deficiency, galactosemia, maple syrup urine disease, homocystinuria, medium-chain acyl Co-A dehydrogenase deficiency, structural birth defects, heart defects, abnormal limbs, club foot, anencephaly, arhinencephaly/holoprosencephaly, hydrocephaly, anophthalmos/microphthalmos, anotia/microtia, transposition of great vessels, tetralogy of Fallot, hypoplastic left heart syndrome, coarctation of aorta, cleft palate without cleft lip, cleft lip with or without cleft palate, oesophageal atresia/stenosis with or without fistula, small intestine atresia/stenosis, anorectal atresia/stenosis, hypospadias, indeterminate sex, renal agenesis, cystic kidney, preaxial polydactyly, limb reduction defects, diaphragmatic hernia, blindness, cataracts, visual problems, hearing loss, deafness, X-linked adrenoleukodystrophy, Rett syndrome, lysosomal disorders, cerebral palsy, autism, aglossia, albinism, ocular albinism, oculocutaneous albinism, gestational diabetes, Arnold-Chiari malformation, CHARGE syndrome, congenital diaphragmatic hernia, brachydactlia, aniridia, cleft foot and hand, heterochromia, Dwarnian ear, Ehlers Danlos syndrome, epidermolysis bullosa, Gorham's disease, Hashimoto's syndrome, hydrops fetalis, hypotonia, Klippel-Feil syndrome, muscular dystrophy, osteogenesis imperfecta, progeria, Smith Lemli Opitz symdrom, chromatelopsia, X-linked lymphoproliferative disease, omphalocele, gastroschisis, pre-eclampsia, eclampsia, pre-term labor, premature birth, miscarriage, delayed intrauterine growth, ectopic pregnancy, hyperemesis gravidarum, morning sickness, or likelihood for successful induction of labor.

As used herein, the term "a neurological or neuropsychiatric disease or condition" refers to any disease or condition that affects nervous systems. Examples of neurological or neuropsychiatric diseases or conditions include, but are not limited to, head trauma, stroke, stroke, ischemic stroke, hemorrhagic stroke, subarachnoid hemorrhage, intra cranial hemorrhage, transient ischemic attack, vascular dementia, corticobasal ganglionic degeneration, encephalitis, epilepsy, Landau-Kleffner syndrome, hydrocephalus, pseudotumor cerebri, thalamic diseases, meningitis, myelitis, movement disorders, essential tremor, spinal cord diseases, syringomyelia, Alzheimer's disease (early onset), Alzheimer's disease (late onset), multi-infarct dementia, Pick's disease, Huntingdon's disease, Parkinson's disease, Parkinson syndromes, dementia, frontotemporal dementia, corticobasal degeneration, multiple system atrophy, progressive supranuclear palsy, Lewy body disease, Creutzfeldt-Jakob disease, Dandy-Walker syndrome, Friedreich ataxia, Machado-Joseph disease, migraine, schizophrenia, mood disorders and depression. dementia with lewy bodies (DLB), frontotemporal dementia (FTD), various forms of vascular dementia (VD), subcortical vascular dementia (Binswanger's disease), autism, developmental retardations, motor neuron diseases, amyotrophic lateral sclerosis (ALS), neuronal or brain damage, hypoxia of the brain, cerebral palsy (CP), memory disorders, movement disorders, corticalbasal ganglionic degeneration, forms of multiple system atrophy, stroke-related disorders, cerebrovascular accidents, post-irradiation encephalopathy with seizures, vascular Parkinsonism, thalamic cerebrovascular accidents, chronic inflammatory demyelinating polyneuropathy, alcohol related dementia, semantic dementia, ataxia, atypical Parkinsonism, dystonia, progressive supranuclear palsy, essential tremor, mild cognitive impairment, amyotrophic lateral sclerosis, multiple sclerosis, neuropathies, Pick's disease, congophilic amyloid angiopathy, Creutzfeldt-Jakob Disease, AIDS dementia complex, depression, anxiety disorder, phobia, Bell's Palsy, epilepsy, encephalitis, neuromuscular disorders, neurooncological disorders, brain tumors, neurovascular disorders, neuroimmunological disorders, neurootological disease, neurotrauma including spinal cord injury, pain including neuropathic pain, pediatric neurological and neuropsychiatric disorders, sleep disorders, Tourette syndrome, corticalbasal ganglionic degeneration, alcohol related dementia, semantic dementia, Alzheimer's disease combined with multi-infarct dementia, Alzheimer's disease combined with Lewy body dementia, Parkinson's disease combined with Lewy body dementia, Alzheimer's and Parkinson's disease combined with Lewy body dementia, frontotemporal dementia combined with chronic inflammatory demyelinating polyneuropathy, attention deficit hyperactivity disorder, schizophrenia, obsessive-compulsive disorder, mental retardation, autistic spectrum disorders, opsoclonus-myoclonus syndrome (OMS) seizures, articulation disorder, learning disabilities (i.e., reading or arithmetic), verbal or performance aptitude deficits, attention deficit disorder, amyloid diseases, prion diseases, Tauopathies, Alpha-Synucleinopathies, addictive states such as those caused by at least one of: cocaine, nicotine, alcohol, food, ecstasy, kat, caffeine, opium, heroin, marijuana, amphetamine, methamphetamine or gambling, and Fabry's disease.

As used herein, the term "an autoimmune or immune-related disease or condition" refers to any disease or condition that affects the function of immune systems. Examples of autoimmune or immune-related diseases or conditions include, but are not limited to, antiphospholipid syndrome, systemic lupus erythematosus, rheumatoid arthritis, autoimmune vasculitis, celiac disease, autoimmune thyroiditis, post-transfusion immunization, maternal-fetal incompatibility, transfusion reactions, immunological deficiency such IgA deficiency, common variable immunodeficiency, drug-induced lupus, diabetes mellitus, Type I diabetes, Type II diabetes, juvenile onset diabetes, juvenile rheumatoid arthritis, psoriatic arthritis, multiple sclerosis, immunodeficiency, allergies, asthma, psoriasis, atopic dermatitis, allergic contact dermatitis, chronic skin diseases, amyotrophic lateral sclerosis, chemotherapy-induced injury, graft-vs-host diseases, bone marrow transplant rejection, Ankylosing spondylitis, atopic eczema, Pemphigus, Behcet's disease, chronic fatigue syndrome fibromyalgia, chemotherapy-induced injury, myasthenia gravis, glomerulonephritis, allergic retinitis, systemic sclerosis, subacute cutaneous lupus erythematosus, cutaneous lupus erythematosus including chilblain lupus erythematosus, Sjogren's syndrome, autoimmune nephritis, autoimmune vasculitis, autoimmune hepatitis, autoimmune carditis, autoimmune encephalitis, autoimmune mediated hematological diseases, lc-SSc (limited cutaneous form of scleroderma), dc-SSc (diffused cutaneous form of scleroderma), autoimmune thyroiditis (AT), Grave's disease (GD), myasthenia gravis, multiple sclerosis (MS), ankylosing spondylitis. transplant rejection, immune aging, rheumatic/autoimmune diseases, mixed connective tissue disease, spondyloarthropathy, psoriasis, psoriatic arthritis, myositis, scleroderma, dermatomyositis, autoimmune vasculitis, mixed connective tissue disease, idiopathic thrombocytopenic purpura, Crohn's disease, human adjuvant disease, osteoarthritis, juvenile chronic arthritis, a spondyloarthropathy, an idiopathic inflammatory myopathy, systemic vasculitis, sarcoidosis, autoimmune hemolytic anemia, autoimmune thrombocytopenia, thyroiditis, immune-mediated renal disease, a demyelinating disease of the central or peripheral nervous system, idiopathic demyelinating polyneuropathy, Guillain-Barre syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease, psoriatic arthritis, psoriasis, dermatitis, polymyositis/dermatomyositis, toxic epidermal necrolysis, systemic scleroderma and sclerosis, responses associated with inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, anti-phospholipid antibody syndrome, allergic neuritis, Bechet disease, Castleman's syndrome, Goodpasture's syndrome, Lambert-Eaton Myasthenic Syndrome, Reynaud's syndrome, Sjorgen's syndrome, Stevens-Johnson syndrome, pemphigoid bullous, pemphigus, autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimmune orchitis and oophoritis, primary hypothyroidism, autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Sheehan's syndrome, autoimmune hepatitis, lymphoid interstitial pneumonitis (HIV), bronchiolitis obliterans (non-transplant) vs NSIP, Guillain-Barre' Syndrome, large vessel vasculitis (including polymyalgia rheumatica and giant cell (Takayasu's) arteritis), medium vessel vasculitis (including Kawasaki's disease and polyarteritis nodosa), ankylosing spondylitis, Berger's disease (IgA nephropathy), rapidly progressive glomerulonephritis, primary biliary cirrhosis, Celiac sprue (gluten enteropathy), cryoglobulinemia, and amyotrophic lateral sclerosis (ALS).

As used herein, the term "cancer" refers to various types of malignant neoplasms, most of which can invade surrounding tissues, and may metastasize to different sites (see, for example, PDR Medical Dictionary, 1st edition (1995), incorporated herein by reference in its entirety for all purposes). The terms "neoplasm" and "tumor" refer to an abnormal tissue that grows by cellular proliferation more rapidly than normal and continues to grow after the stimuli that initiated proliferation is removed. Id. Such abnormal tissue shows partial or complete lack of structural organization and functional coordination with the normal tissue which may be either benign (i.e., benign tumor) or malignant (i.e., malignant tumor). Examples of general categories of cancer include, but are not limited to, carcinomas (i.e., malignant tumors derived from epithelial cells such as, for example, common forms of breast, prostate, lung and colon cancer), sarcomas (i.e., malignant tumors derived from connective tissue or mesenchymal cells), lymphomas (i.e., malignancies derived from hematopoietic cells), leukemias (i.e., malignancies derived from hematopoietic cells), germ cell tumors (i.e., tumors derived from totipotent cells. In adults most often found in the testicle or ovary; in fetuses, babies and young children, most often found on the body midline, particularly at the tip of the tailbone), blastic tumors (i.e., a typically malignant tumor which resembles an immature or embryonic tissue) and the like. Examples of the types of neoplasms intended to be encompassed by the present invention include but are not limited to those neoplasms associated with cancers of neural tissue, blood forming tissue, breast, skin, bone, prostate, ovaries, uterus, cervix, liver, lung, brain, larynx, gallbladder, pancreas, rectum, parathyroid, thyroid, adrenal gland, immune system, head and neck, colon, stomach, bronchi, and/or kidneys.

As used herein, the term "infectious agent" includes, but is not limited to, pathogenic organisms such as viruses, bacteria, fungi, parasites, infectious proteins and the like.

Viruses include, but are not limited to, DNA or RNA animal viruses. As used herein, RNA viruses include, but are not limited to, virus families such as Picornaviridae (e.g., polioviruses), Reoviridae (e.g., rotaviruses), Togaviridae (e.g., encephalitis viruses, yellow fever virus, rubella virus), Orthomyxoviridae (e.g., influenza viruses), Paramyxoviridae (e.g., respiratory syncytial virus, measles virus, mumps virus, parainfluenza virus), Rhabdoviridae (e.g., rabies virus), Coronaviridae, Bunyaviridae, Flaviviridae, Filoviridae, Arenaviridae, Bunyaviridae and Retroviridae (e.g., human T cell lymphotropic viruses (HTLV), human immunodeficiency viruses (HIV)). As used herein, DNA viruses include, but are not limited to, virus families such as Papovaviridae (e.g., papilloma viruses), Adenoviridae (e.g., adenovirus), Herpesviridae (e.g., herpes simplex viruses), and Poxviridae (e.g., variola viruses).

Bacteria include, but are not limited to, gram positive bacteria, gram negative bacteria, acid-fast bacteria and the like.

As used herein, gram positive bacteria include, but are not limited to, Actinomedurae, Actinomyces israelii, Bacillus anthracis, Bacillus cereus, Clostridium botulinum, Clostridium difficile, Clostridium perfringens, Clostridium tetani, Corynebacterium, Enterococcus faecalis, Listeria monocytogenes, Nocardia, Propionibacterium acnes, Staphylococcus aureus, Staphylococcus epiderm, Streptococcus mutans, Streptococcus pneumoniae and the like.

As used herein, gram negative bacteria include, but are not limited to, Afipia felis, Bacteriodes, Bartonella bacilliformis, Bortadella pertussis, Borrelia burgdorferi, Borrelia recurrentis, Brucella, Calymmatobacterium granulomatis, Campylobacter, Escherichia coli, Francisella tularensis, Gardnerella vaginalis, Haemophilius aegyptius, Haemophilius ducreyi, Haemophilius influenziae, Heliobacter pylori, Legionella pneumophila, Leptospira interrogans, Neisseria meningitidia, Porphyromonas gingivalis, Providencia sturti, Pseudomonas aeruginosa, Salmonella enteridis, Salmonella typhi, Serratia marcescens, Shigella boydii, Streptobacillus moniliformis, Streptococcus pyogenes, Treponema pallidum, Vibrio cholerae, Yersinia enterocolitica, Yersinia pestis and the like.

As used herein, acid-fast bacteria include, but are not limited to, Myobacterium avium, Myobacterium leprae, Myobacterium tuberculosis and the like.

As used herein, other bacteria not falling into the other three categories include, but are not limited to, Bartonella henseiae, Chlamydia psittaci, Chlamydia trachomatis, Coxiella burnetii, Mycoplasma pneumoniae, Rickettsia akari, Rickettsia prowazekii, Rickettsia rickettsii, Rickettsia tsutsugamushi, Rickettsia typhi, Ureaplasma urealyticum, Diplococcus pneumoniae, Ehrlichia chafensis, Enterococcus faecium, Meningococci and the like.

As used herein, fungi include, but are not limited to, Aspergilli, Candidae, Candida albicans, Coccidioides immitis, Cryptococci, and combinations thereof.

As used herein, parasitic microbes include, but are not limited to, Balantidium coli, Cryptosporidium parvum, Cyclospora cayatanensis, Encephalitozoa, Entamoeba histolytica, Enterocytozoon bieneusi, Giardia lamblia, Leishmaniae, Plasmodii, Toxoplasma gondii, Trypanosomae, trapezoidal amoeba and the like.

As used herein, parasites include worms (e.g., helminthes), particularly parasitic worms including, but not limited to, Nematoda (roundworms, e.g., whipworms, hookworms, pinworms, ascarids, filarids and the like), Cestoda

### (e.g., tapeworms)

As used herein, "treating" a disease or condition refers to taking steps to obtain beneficial or desired results, including clinical results. Beneficial or desired clinical results include, but are not limited to, alleviation or amelioration of one or more symptoms associated with diseases or conditions.

As used herein, "administering" or "administration of" a compound or an agent to a subject can be carried out using one of a variety of methods known to those skilled in the art. For example, a compound or an agent can be administered, intravenously, arterially, intradermally, intramuscularly, intraperitonealy, intravenously, subcutaneously, ocularly, sublingually, orally (by ingestion), intranasally (by inhalation), intraspinally, intracerebrally, and transdermally (by absorbtion, e.g., through a skin duct). A compound or agent can also appropriately be introduced by rechargeable or biodegradable polymeric devices or other devices, e.g., patches and pumps, or formulations, which provide for the extended, slow, or controlled release of the compound or agent. Administering can also be performed, for example, once, a plurality of times, and/or over one or more extended periods. In some aspects, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient. In some embodiments, a compound or an agent is administered orally, e.g., to a subject by ingestion, or intravenously, e.g., to a subject by injection. In some embodiments, the orally administered compound or agent is in an extended release or slow release formulation, or administered using a device for such slow or extended release.

In certain embodiments, markers used in the methods of invention are up-regulated or activated in the cell-free bodily fluids compared to the non-phagocytic cells. In certain embodiments, markers used in the methods of invention are down-regulated or inhibited in the cell-free bodily fluids compared to the non-phagocytic cells. In certain embodiments, markers used in the methods of invention are up-regulated or activated in the cell-free bodily fluids compared to the =2n phagocytic cells. In certain embodiments, markers used in the methods of invention are down-regulated or inhibited in the cell-free bodily fluids compared to the =2n phagocytic cells. Different diseases or conditions can be associated with either up-regulation (or activation) or down-regulation (or inhibition) of different markers. As used herein, "up-regulation or up-regulated" can refer to an increase in expression levels (e.g., gene expression or protein expression), gene copy numbers, gene dosages, and other qualitative or quantitative detectable state of the markers. Similarly, "down-regulation or down-regulated" can refer to an increase in expression levels, gene copy numbers, gene dosages, and other qualitative or quantitative detectable state of the markers. As used herein, "activation or activated" can refer to an active state of the marker, e.g., a phosphorylation state, a DNA methylation state, or a DNA acetylation state. Similarly, "inhibition or inhibited" can refer to a repressed state or an inactivated state of the marker, e.g., a dephosphorylation state, a ubiquitination state, a DNA de-methylation state.

In certain embodiments, methods of this invention can also comprise extracting or enriching markers from cell-free bodily fluids. Any known extraction and enrichment methods can be used herein. In certain embodiments, methods of this invention also comprise at least one of the following steps before determination of various profiles: i) lysing the non-phagocytic orthe =2n phagocytic cells; ii) extracting cellular contents from the lysed non-phagocytic or the =2n phagocytic cells. Any known cell lysis and extraction methods can be used herein. In certain embodiments, the cell-free bodily fluidscomprise various types of materials that they have engulfed, such as, viable diseased cells, dead diseased cells, apoptotic diseased cells, circulating tumor cells, infectious agents, fetal cells, trophoblasts, or fragments thereof. In certain embodiments, at least one or more markers of a disease or condition are present in the cell-free bodily fluids. In certain embodiments, there is no marker present in the cellular contents of the non-phagocytic cells or the =2n phagocytic cells.

In certain embodiments, methods of this invention further comprise comparing the identified difference of the disease or condition-specific markers to a repository of at least one marker known in the art. Such comparison can further confirm the presence of the disease or condition. In some embodiments, the repository of the known markers can be obtained by data mining. The term "data mining", as used herein, refers to a process of finding new data patterns, relations, or correlations derived from the known data of the databases and of extracting practicable information in the future. Typically a computer-based system can be trained on data to perform the data mining, e.g., to classify the input data and then subsequently used with new input data to make decisions based on the training data. These systems include, but are not limited, expert systems, fuzzy logic, non-linear regression analysis, multivariate analysis, decision tree classifiers, and Bayesian belief networks.

In certain embodiments, the cell-free bodily fluid come from a bodily fluid sample. Exemplar bodily fluid sample can be whole blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid. In some embodiments, the cell-free bodily fluids are obtained by separating cells from the bodily fluid sample by methods known in the art, such as extraction, centrifugation, and filtration.

In some embodiments, the =2n phagocytic cells or the non-phagocytic cells are isolated from white blood cells. In certain embodiments, the =2n phagocytic cells are separated from a population of phagocytic cells.

In certain embodiments, tissue or fluid samples including cells having a DNA content of 2n are obtained post separation (e.g., via centrifugation) of non-cellular fraction of fluids obtained by puncture of a vein or artery followed by the withdrawal of blood, tissue biopsies, bronchoalveolar lavage, nasal lavage, eye lavage, peritoneal cavity lavage, vaginal lavage, bladder lavage, rectal lavage, fine needle aspiration of spinal fluid, synovial fluid aspiration, and the like. Cell free bodily fluids are obtained post separation (e.g., via centrifugation) of cellular fraction of fluids obtained by puncture of a vein or artery followed by the withdrawal of blood, tissue biopsies, bronchoalveolar lavage, nasal lavage, eye lavage, peritoneal cavity lavage, vaginal lavage, bladder lavage, rectal lavage, fine needle aspiration of spinal fluid, synovial fluid aspiration, and the like.

In the methods of this invention, cell separation/isolation/purification methods are used to isolate populations of cells from bodily fluid sample, cells, or tissues of a subject. A skilled worker can use any known cell separation/isolation/purification techniques to isolate =2n phagocytic cells or non-phagocyticcells from bodily fluids. Exemplar techniques for cell extractions/separation/isolation include, but are not limited to, using antibodies, flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, or a combination thereof.

In certain aspects of the methods described herein, analytes to be profiled include nucleic acids, proteins, lipids, carbohydrates, metabolites, or any combinations of these. In certain aspects of the methods described herein, markers include nucleic acids, proteins, lipids, carbohydrates, metabolites, or any combinations of these. As used herein, the term "nucleic acid" is intended to include DNA molecules (e.g., cDNA or genomic DNA), RNA molecules (e.g., mRNA), DNA-RNA hybrids, and analogs of the DNA or RNA generated using nucleotide analogs. The nucleic acid molecule can be a nucleotide, oligonucleotide, double-stranded DNA, single-stranded DNA, multi-stranded DNA, complementary DNA, genomic DNA, non-coding DNA, messenger RNA (mRNAs), microRNA (miRNAs), small nucleolar RNA (snoRNAs), ribosomal RNA (rRNA), transfer RNA (tRNA), small interfering RNA (siRNA), heterogeneous nuclear RNAs (hnRNA), or small hairpin RNA (shRNA).

As used herein, the term "amino acid" includes organic compounds containing both a basic amino group and an acidic carboxyl group. Included within this term are natural amino acids (e.g., L-amino acids), modified and unusual amino acids (e.g., D-amino acids and β-amino acids), as well as amino acids which are known to occur biologically in free or combined form but usually do not occur in proteins. Natural protein occurring amino acids include alanine, arginine, asparagine, aspartic acid, cysteine, glutamic acid, glutamine, glycine, histidine, isoleucine, leucine, lysine, methionine, phenylalanine, serine, threonine, tyrosine, tryptophan, proline, and valine. Natural non-protein amino acids include arginosuccinic acid, citrulline, cysteine sulfuric acid, 3,4-dihydroxyphenylalanine, homocysteine, homoserine, ornithine, 3-monoiodotyrosine, 3,5-diiodotryosine, 3, 5, 5-triiodothyronine, and 3,3',5,5'- tetraiodothyronine. Modified or unusual amino acids include D-amino acids, hydroxylysine, 4-hydroxyproline, N-Cbz-protected amino acids, 2,4-diaminobutyric acid, homoarginine, norleucine, N-methylaminobutyric acid, naphthylalanine, phenylglycine, .alpha.-phenylproline, tert-leucine, 4-aminocyclohexylalanine, N-methyl-norleucine, 3 ,4-dehydroproline, N,N-dimethylaminoglycine, N-methylaminoglycine, 4-aminopiperidine-4-carboxylic acid, 6-aminocaproic acid, trans-4-(aminomethyl)-cyclohexanecarboxylic acid, 2-, 3-, and 4-(aminomethyl)- benzoic acid, 1-aminocyclopentanecarboxylic acid, 1-aminocyclopropanecarboxylic acid, and 2-benzyl-5-aminopentanoic acid.

As used herein, the term "peptide" includes compounds that consist of two or more amino acids that are linked by means of a peptide bond. Peptides may have a molecular weight of less than 10,000 Daltons, less than 5,000 Daltons, or less than 2,500 Daltons. The term "peptide" also includes compounds containing both peptide and non-peptide components, such as pseudopeptide or peptidomimetic residues or other non-amino acid components. Such compounds containing both peptide and non-peptide components may also be referred to as a "peptide analog."

As used herein, the term "protein" includes compounds that consist of amino acids arranged in a linear chain and joined together by peptide bonds between the carboxyl and amino groups of adjacent amino acid residues. Proteins used in methods of the invention include, but are not limited to, amino acids, peptides, antibodies, antibody fragments, cytokines, lipoproteins, or glycoproteins.

As used herein, the term "antibody" includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, and antibody fragments (e.g., Fab or F(ab')2, and Fv). For the structure and properties of the different classes of antibodies, see e.g., Basic and Clinical Immunology, 8th Edition, Daniel P. Sties, Abba I. Terr and Tristram G. Parsolw (eds), Appleton & Lange, Norwalk, Conn., 1994, page 71 and Chapter 6.

As used herein, the term "cytokine" refers to a secreted protein or active fragment or mutant thereof that modulates the activity of cells of the immune system. Examples of cytokines include, without limitation, interleukins, interferons, chemokines, tumor necrosis factors, colony-stimulating factors for immune cell precursors, and the like.

As used herein, the term "lipoprotein" includes negatively charged compositions that comprise a core of hydrophobic cholesteryl esters and triglyceride surrounded by a surface layer of amphipathic phospholipids with which free cholesterol and apolipoproteins are associated. Lipoproteins may be characterized by their density (e.g. very-low-density lipoprotein (VLDL), low-density lipoprotein (LDL) and high density lipoprotein (HDL)), which is determined by their size, the relative amounts of lipid and protein. Lipoproteins may also be characterized by the presence or absence of particular modifications (e.g. oxidization, acetylation, or glycation).

As used herein, the term "glycoprotein" includes glycosides which have one or more oligo- or polysaccharides covalently attached to a peptide or protein. Exemplary glycoproteins can include, without limitation, immunoglobulins, members of the major histocompatibility complex, collagens, mucins, glycoprotein IIb/IIIa, glycoprotein-41 (gp41) and glycoprotein-120 (gp12), follicle-stimulating hormone, alpha-fetoprotein, erythropoietin, transferrins, alkaline phosphatase, and lectins.

As used herein, the term "lipid" includes synthetic or naturally-occurring compounds which are generally amphipathic and biocompatible. Lipids typically comprise a hydrophilic component and a hydrophobic component. Exemplary lipids include, but are not limited to fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, sulfatide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin,, gangliosides, plasmalogen, sulfatide, ceramide, low density lipoproteins (LDLs), very low density lipoproteins (VLDLs), high density lipoproteins (HDLs), sphingoid base-1-phosphates or derivatives thereof.

As used herein, the term "carbohydrate" includes, but is not limited to, compounds that contain oxygen, hydrogen and carbon atoms, typically (CH2O)n wherein n is an integer. Exemplary carbohydrates include, but are not limited to, monosaccharides, disaccharides, polysaccharides, or oligosaccharides.

As used herein, the term "metabolite" includes any molecule used in metabolism. Metabolites can be products, substrates, or intermediates in metabolic processes. Included within this term are primary metabolites, secondary metabolites, organic metabolites, or inorganic metabolites. Metabolites include, without limitation, amino acids, peptides, acylcarnitines, monosaccharides, lipids and phospholipids, prostaglandins, hydroxyeicosatetraenoic acids, hydroxyoctadecadienoic acids, steroids, bile acids, and glycolipids and phospholipids. Exemplary metabolites can be sphingolipids, glycosphingolipids, sphingosine, ceramide, sphingomyelin, sphingosylphosphorylcholin, dihydrosphingosine, phoshatidylcholine, phosphatidylinositol, phosphatidylserine, lysophoshatidylcholine, lysophosphatidylinositol, lysophosphatidylserine, plasmenylphoshatidylcholine, plasmanylphoshatidylcholine, proteinogenic amino acids, Alanine, Aspartic acid, Glutamic acid, Phenylalanine, Glycine, Histidine, Leucine, Isoleucine, Lysine, Methionine, Proline, Arginine, Serine, Threonine, Valine, Tryptophan, Tyrosine, asymmetrical dimethyl arginine, symmetrical dimethyl arginine, Glutamine, Asparagine, Nitrotyrosine, Hydroxyproline, Kynurenine, 3-Hydroxy kynurenine, non-proteinogenic amino acids, Ornithine, Citrulline, acylcarnitines, carnitine, free carnitine, acylcarnitine, hydroxylacylcarnitine, dicarboxylacylcarnitines, reducing monosaccharides, hexose, pentose, deoxyhexose, creatinine, creatine, spermidine spermine, putrescine, dopamine, serotonin, prostaglandins, hydoxyeicosatetraeneoic acid, Hydroxyoctadecadienoic acid, leukatrienes, thromboxanes, bile acids, sterols, cholesterols, vitamins and cofactors, drugs, and drug metabolites.

In some embodiments of the invention, profiles of at least one or more markers of a disease or condition are compared. This comparison can be quantitative or qualitative. Quantitative measurements can be taken using any of the assays described herein. For example, sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLID^{®} sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.

Quantitative comparisons can include statistical analyses such as t-test, ANOVA, Krustal-Wallis, Wilcoxon, Mann-Whitney, and odds ratio. Quantitative differences can include differences in the levels of markers between profiles or differences in the numbers of markers present between profiles, and combinations thereof. Examples of levels of the markers can be, without limitation, gene expression levels, nucleic acid levels, protein levels, lipid levels, and the like. Qualitative differences can include, but are not limited to, activation and inactivation, protein degradation, nucleic acid degradation, and covalent modifications.

In certain embodiments of the invention, the profile is a nucleic acid profile, a protein profile, a lipid profile, a carbohydrate profile, a metabolite profile, or a combination thereof. The profile can be qualitatively or quantitatively determined.

A nucleic acid profile can be, without limitation, a genotypic profile, a single nucleotide polymorphism profile, a gene mutation profile, a gene copy number profile, a DNA methylation profile, a DNA acetylation profile, a chromosome dosage profile, a gene expression profile, or a combination thereof.

The nucleic acid profile can be determined by any methods known in the art to detect genotypes, single nucleotide polymorphisms, gene mutations, gene copy numbers, DNA methylation states, DNA acetylation states, chromosome dosages. Exemplar methods include, but are not limited to, polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, methyl-binding PCR analysis, or a combination thereof.

As used herein, the term "sequencing" is used in a broad sense and refers to any technique known in the art that allows the order of at least some consecutive nucleotides in at least part of a nucleic acid to be identified, including without limitation at least part of an extension product or a vector insert. Exemplar sequencing techniques include direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{®} sequencing, MS-PET sequencing, mass spectrometry, and a combination thereof. In some embodiments, sequencing comprises an detecting the sequencing product using an instrument, for example but not limited to an ABI PRISM^{®} 377 DNA Sequencer, an ABI PRISM^{®} 310, 3100, 3100-Avant, 3730, or 373OxI Genetic Analyzer, an ABI PRISM^{®} 3700 DNA Analyzer, or an Applied Biosystems SOLiD^{™} System (all from Applied Biosystems), a Genome Sequencer 20 System (Roche Applied Science), or a mass spectrometer. In certain embodiments, sequencing comprises emulsion PCR. In certain embodiments, sequencing comprises a high throughput sequencing technique, for example but not limited to, massively parallel signature sequencing (MPSS).

In further embodiments of the invention, a protein profile can be a protein expression profile, a protein activation profile, or a combination thereof. In some embodiments, a protein activation profile can comprise determining a phosphorylation state, an ubiquitination state, a myristoylation state, or a conformational state of the protein.

A protein profile can be detected by any methods known in the art for detecting protein expression levels, protein phosphorylation state, protein ubiquitination state, protein myristoylation state, or protein conformational state. In some embodiments, a protein profile can be determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof.

In some embodiments of the invention, a lipid profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof. Further methods for analyzing lipid content in a biological sample are known in the art (See, e.g., Kang et al. (1992) Biochim. Biophys. Acta. 1128:267; Weylandt et al. (1996) Lipids 31 :977; J. Schiller et al. (1999) Anal. Biochem. 267:46; Kang et al. (2001) Proc. Natl. Acad. Sci. USA 98:4050; Schiller et al. (2004) Prog. Lipid Res. 43:499). One exemplary method of lipid analysis is to extract lipids from a biological sample (e.g. using chloroform-methanol (2:1, vol/vol) containing 0.005% butylated hydroxytoluene (BHT, as an antioxidant)), prepare fatty acid methyl esters (e.g., using 14% BF3-methanol reagent), and quantify the fatty acid methyl esters (e.g., by HPLC, TLC, by gas chromatography-mass spectroscopy using commercially available gas chromatographs, mass spectrometers, and/or combination gas chromatograph/mass spectrometers). Fatty acid mass is determined by comparing areas of various analyzed fatty acids to that of a fixed concentration of internal standard.

In some embodiments of the invention, a carbohydrate profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

In some embodiments of the invention, a metabolite profile can be determind by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

As used herein, the "difference" between different profiles detected by the methods of this invention can refer to different gene copy numbers, different DNA, RNA, protein, lipid, or carbohydrate expression levels, different DNA methylation states, different DNA acetylation states, and different protein modification states. The difference can be a difference greater than 1 fold. In some embodiments, the difference is a 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference. In some embodiments, the difference is any fold difference between 1-10, 2-10, 5-10, 10-20, or 10-100 folds.

A general principle of assays to detect markers involves preparing a sample or reaction mixture that may contain the marker (e.g., one or more of DNA, RNA, protein, polypeptide, carbohydrate, lipid, metabolite, and the like) and a probe under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one embodiment of such a method, a sample from a subject, which is to be assayed for presence and/or concentration of marker, can be anchored onto a carrier or solid phase support. In another embodiment, the reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilized through conjugation of biotin and streptavidin. Such biotinylated assay components can be prepared from biotin-NHS(N-hydroxy-succinimide) using techniques known in the art (e.g., biotinylation kit, Pierce Chemicals, Rockford, IL), and immobilized in the wells of streptavidin-coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker or probe belongs. Well known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above-mentioned approaches, the non- immobilized component is added to the solid phase upon which the second component is anchored. After the reaction is complete, uncomplexed components may be removed (e.g., by washing) under conditions such that any complexes formed will remain immobilized upon the solid phase. The detection of marker/probe complexes anchored to the solid phase can be accomplished in a number of methods outlined herein.

In certain exemplary embodiments, the probe, when it is the unanchored assay component, can be labeled for the purpose of detection and readout of the assay, either directly or indirectly, with detectable labels discussed herein and which are well-known to one skilled in the art.

It is also possible to directly detect marker/probe complex formation without further manipulation or labeling of either component (marker or probe), for example by utilizing the technique of fluorescence energy transfer (see, for example, U.S. Patent Nos. 5,631,169 and 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determination of the ability of a probe to recognize a marker can be accomplished without labeling either assay component (probe or marker) by utilizing a technology such as real-time Biomolecular Interaction Analysis (BIA) (see, e.g., Sjolander, S. and Urbaniczky, C, 1991, Anal. Chem. 63:2338 2345 and Szabo et al, 1995, Curr. Opin. Struct. Biol. 5:699 705). As used herein, "BIA" or "surface plasmon resonance" is a technology for studying biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas and Minton (1993) Trends Biochem. Sci. 18:284). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, e.g., Heegaard (1998) J. Mol. Recognit. 11 :141; Hage and Tweed (1997) J. Chromatogr. B. Biomed. Sci. Appl. 12:499). Gel electrophoresis may also be employed to separate complexed assay components from unbound components (see, e.g., Ausubel et al, ed., Current Protocols in Molecular Biology, John Wiley & Sons, New York, 1987 1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

In certain exemplary embodiments, the level of mRNA corresponding to the marker can be determined either by in situ and/or by in vitro formats in a biological sample using methods known in the art. Many expression detection methods use isolated RNA. For in vitro methods, any RNA isolation technique that does not select against the isolation of mRNA can be utilized for the purification of RNA from blood cells (see, e.g., Ausubel et al, ed., Current Protocols in Molecular Biology, John Wiley & Sons, New York 1987 1999). Additionally, large numbers of cells and/or samples can readily be processed using techniques well known to those of skill in the art, such as, for example, the single-step RNA isolation process of Chomczynski (1989, U.S. Patent No. 4,843,155).

Isolated mRNA can be used in hybridization or amplification assays that include, but are not limited to, Southern or Northern analyses, polymerase chain reaction analyses and probe arrays. In certain exemplary embodiments, a diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to an mRNA or genomic DNA encoding a marker of the present invention. Other suitable probes for use in the diagnostic assays of the invention are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in a gene chip array. A skilled artisan can readily adapt known mRNA detection methods for use in detecting the level of mRNA encoded by the markers of the present invention.

An alternative method for determining the level of mRNA corresponding to a marker of the present invention in a sample involves the process of nucleic acid amplification, e.g., by RT-PCR (the experimental embodiment set forth in U.S. Patent Nos. 4,683,195 and 4,683,202), COLD-PCR (Li et al. (2008) Nat. Med. 14:579), ligase chain reaction (Barany, 1991, Proc. Natl. Acad. Sci. USA, 88:189), self sustained sequence replication (Guatelli et al., 1990, Proc. Natl. Acad. Sci. USA 87:1874), transcriptional amplification system (Kwoh et al. (1989) Proc. Natl. Acad. Sci. USA 86:1173), Q- Beta Replicase (Lizardi et al. (1988) Bio/Technology 6:1197), rolling circle replication (U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For in situ methods, mRNA does not need to be isolated from the sample (e.g., a bodily fluid (e.g., blood cells)) prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, e.g., a housekeeping gene that is constitutively expressed. Suitable genes for normalization include housekeeping genes such as the actin gene, or epithelial cell- specific genes. This normalization allows the comparison of the expression level in a patient sample from one source to a patient sample from another source, e.g., to compare a phagocytic blood cell from an individual to a non-phagocytic blood cell from the individual.

In one embodiment of this invention, a protein or polypeptide corresponding to a marker is detected. In certain embodiments, an agent for detecting a protein or polypeptide can be an antibody capable of binding to the polypeptide, such as an antibody with a detectable label. As used herein, the term "labeled," with regard to a probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. Antibodies can be polyclonal or monoclonal. An intact antibody, or a fragment thereof (e.g., Fab or F(ab')2) can be used. In one format, antibodies, or antibody fragments, can be used in methods such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well known supports or carriers include glass, polystyrene, polypropylene, polyethylene, dextran, nylon, amylases, natural and modified celluloses, polyacrylamides, gabbros, magnetite and the like.

A variety of formats can be employed to determine whether a sample contains a protein that binds to a given antibody. Examples of such formats include, but are not limited to, competitive and non-competitive immunoassay, enzyme immunoassay (EIA), radioimmunoassay (RIA), antigen capture assays, two-antibody sandwich assays, Western blot analysis, enzyme linked immunoabsorbant assay (ELISA), a planar array, a colorimetric assay, a chemiluminescent assay, a fluorescent assay, and the like. Immunoassays, including radioimmmunoassays and enzyme- linked immunoassays, are useful in the methods of the present invention. A skilled artisan can readily adapt known protein/antibody detection methods for use in determining whether cells (e.g., bodily fluid cells such as blood cells) express a marker of the present invention.

One skilled in the art will know many other suitable carriers for binding antibody or antigen, and will be able to adapt such support for use with the present invention. For example, protein isolated from cells (e.g., bodily fluid cells such as blood cells) can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In certain exemplary embodiments, assays are provided for diagnosis, prognosis, assessing the risk of developing a disease, assessing the efficacy of a treatment, monitoring the progression or regression of a disease, and identifying a compound capable of ameliorating or treating a disease. An exemplary method for these methods involves obtaining a bodily fluid sample from a test subject and contacting the bodily fluid sample with a compound or an agent capable of detecting one or more of the markers of the disease or condition, e.g., marker nucleic acid (e.g., mRNA, genomic DNA), marker peptide (e.g., polypeptide or protein), marker lipid (e.g., cholesterol), or marker metabolite (e.g., creatinine) such that the presence of the marker is detected in the biological sample. In one embodiment, an agent for detecting marker mRNA or genomic DNA is a labeled nucleic acid probe capable of hybridizing to marker mRNA or genomic DNA. The nucleic acid probe can be, for example, a full-length marker nucleic acid or a portion thereof. Other suitable probes for use in the diagnostic assays of the invention are described herein.

As used herein, a compound capable of ameliorating or treating a disease or condition can include, without limitations, any substance that can improve symptoms or prognosis, prevent progression of the disease or condition, promote regression of the disease or condition, or eliminate the disease or condition.

The methods of the invention can also be used to detect genetic alterations in a marker gene, thereby determining if a subject with the altered gene is at risk for developing a disease and/or disorder associated with cancer and/or an infectious agent, and/or one or more other disorders described herein characterized by misregulation in a marker protein activity or nucleic acid expression, such as cancer. In certain embodiments, the methods include detecting, in a cell free bodily fluid sample from the subject, the presence or absence of a genetic alteration characterized by an alteration affecting the integrity of a gene encoding a marker peptide and/or a marker gene. For example, such genetic alterations can be detected by ascertaining the existence of at least one of: 1) a deletion of one or more nucleotides from one or more marker genes; 2) an addition of one or more nucleotides to one or more marker genes; 3) a substitution of one or more nucleotides of one or more marker genes, 4) a chromosomal rearrangement of one or more marker genes; 5) an alteration in the level of a messenger RNA transcript of one or more marker genes; 6) aberrant modification of one or more marker genes, such as of the methylation pattern of the genomic DNA; 7) the presence of a non-wild type splicing pattern of a messenger RNA transcript of one or more marker genes; 8) a non-wild type level of a one or more marker proteins; 9) allelic loss of one or more marker genes; and 10) inappropriate post-translational modification of one or more marker proteins. As described herein, there are a large number of assays known in the art which can be used for detecting alterations in one or more marker genes.

In certain embodiments, detection of the alteration involves the use of a probe/primer in a polymerase chain reaction (PCR) (see, e.g., U.S. Patent Nos. 4,683,195, 4,683,202 and 5,854,033), such as real-time PCR, COLD-PCR (Li et al. (2008) Nat. Med. 14:579), anchor PCR, recursive PCR or RACE PCR, or, alternatively, in a ligation chain reaction (LCR) (see, e.g., Landegran et al. (1988) Science 241:1077; Prodromou and Pearl (1992) Protein Eng. 5:827; and Nakazawa et al. (1994) Proc. Natl. Acad. Sci. USA 91:360), the latter of which can be particularly useful for detecting point mutations in a marker gene (see Abravaya et al. (1995) Nucleic Acids Res. 23:675). This method can include the steps of collecting a sample of cell free bodily fluid from a subject, isolating nucleic acid (e.g., genomic, mRNA or both) from the sample, contacting the nucleic acid sample with one or more primers which specifically hybridize to a marker gene under conditions such that hybridization and amplification of the marker gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al., (1990) Proc. Natl. Acad. Sci. USA 87: 1874), transcriptional amplification system (Kwoh et al., (1989) Proc. Natl. Acad. Sci. USA 86:1173), Q Beta Replicase (Lizardi et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in one or more marker genes from a sample can be identified by alterations in restriction enzyme cleavage patterns. For example, sample and control DNA is isolated, optionally amplified, digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Pat. No. 5,498,531) can be used to score for the presence of specific mutations by development or loss of a ribozyme cleavage site.

In other embodiments, genetic mutations in one or more of the markers described herein can be identified by hybridizing a sample and control nucleic acids, e.g., DNA or RNA, to high density arrays containing hundreds or thousands of oligonucleotides probes (Cronin et al. (1996) Human Mutation 7: 244; Kozal et al. (1996) Nature Medicine 2:753). For example, genetic mutations in a marker nucleic acid can be identified in two dimensional arrays containing light-generated DNA probes as described in Cronin, M. T. et al. supra. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence a marker gene and detect mutations by comparing the sequence of the sample marker gene with the corresponding wild-type (control) sequence. Examples of sequencing reactions include those based on techniques developed by Maxam and Gilbert ((1977) Proc. Natl. Acad. Sci. USA 74:560) or Sanger ((1977) Proc. Natl. Acad. Sci. USA 74:5463). It is also contemplated that any of a variety of automated sequencing procedures can be utilized when performing the diagnostic assays ((1995) Biotechniques 19:448), including sequencing by mass spectrometry (see, e.g., PCT International Publication No. WO 94/16101; Cohen et al. (1996) Adv. Chromatogr. 36:127-162; and Griffin et al. (1993) Appl. Biochem. Biotechnol. 38:147).

Other methods for detecting mutations in a marker gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes formed by hybridizing (labeled) RNA or DNA containing the wild-type marker sequence with potentially mutant RNA or DNA obtained from a tissue sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl. Acad. Sci. USA 85:4397; Saleeba et al. (1992) Methods Enzymol. 217:286. In one embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in marker cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657). According to an exemplary embodiment, a probe based on a marker sequence, e.g., a wild-type marker sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in marker genes. For example, single strand conformation polymorphism (SSCP) may be used to detect differences in electrophoretic mobility between mutant and wild type nucleic acids (Orita et al. (1989) Proc. Natl. Acad. Sci. USA 86:2766, see also Cotton (1993) Mutat. Res. 285:125; and Hayashi (1992) Genet. Anal. Tech. Appl. 9:73). Single-stranded DNA fragments of sample and control marker nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility (Keen et al. (1991) Trends Genet. 7:5).

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to ensure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys. Chem. 265:12753).

Examples of other techniques for detecting point mutations include, but are not limited to, selective oligonucleotide hybridization, selective amplification or selective primer extension. For example, oligonucleotide primers may be prepared in which the known mutation is placed centrally and then hybridized to target DNA under conditions which permit hybridization only if a perfect match is found (Saiki et al. (1986) Nature 324:163; Saiki et al. (1989) Proc. Natl. Acad. Sci. USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucl. Acids Res. 17:2437) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci. USA 88:189). In such cases, ligation will occur only if there is a perfect match at the 3' end of the 5' sequence making it possible to detect the presence of a known mutation at a specific site by looking for the presence or absence of amplification.

In one aspect, this invention provides a method for identifying one or more markers for a disease or condition comprising: a) determining a first profile of analytes from a cell-free bodily fluid sample from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from a cell-free bodily fluid sample from a control subject not having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. Optionally, this method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; determining a second profile of analytes from a cell-free bodily fluid sample from a control subject not having said disease or condition; identifying a first set of differences between the first and second profiles, wherein the first set of differences is specific to the first profile relative to the second profile; b) determining a third profile of analytes from non-phagocytic cells from the subject having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; obtaining a second profile of analytes from a cell-free bodily fluid sample from a control subject not having said disease or condition by data mining; identifying a first set of differences between the first and second profiles, wherein the first set of differences is specific to the first profile relative to the second profile; b) determining a third profile of analytes from non-phagocytic cells from the subject having said disease or condition; obtaining a fourth profile of analytes from non-phagocytic cells from a control subject not having said disease or condition by data mining; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition by data mining; obtaining a sixth profile of analytes from cells or tissues not affected by said disease or condition by data mining; identifying a third set of differences between the fifth and sixth profiles, wherein the third set of differences is specific to the fifth profile relative to the sixth profile; and e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from cells or tissues affected by said disease or condition from the subject having said disease or condition; determining a fourth profile of analytes from cells or tissues not affected by said disease or condition from the subject having said disease or condition; identifying a second set of differences between the third and fourth profiles, wherein the second set of differences is specific to the third profile relative to the fourth profile; c) identifying one or more analytes present in both the first set of differences and the second set of differences, the identified analytes being markers of said disease or condition. And optionally, the method further comprises d) determining a fifth profile of analytes from a cell-free bodily fluid sample from a control subject not having said disease or condition; identifying a third set of differences between the first and fifth profiles, wherein the third set of differences is specific to the first profile relative to the fifth profile; e) identifying at least one of the one or more markers of c) present in the third set of differences.

In yet another aspect, this invention provides a method for identifying one or more markers of a disease or condition comprising: a) determining a first profile of analytes from a cell-free bodily fluid sample from a subject having said disease or condition; determining a second profile of analytes from =2n phagocytic cells from the subject having said disease or condition; identifying a first set of differences between the first and second profiles, wherein the first set of differences is specific to the first profile relative to the second profile; b) determining a third profile of analytes from a cell-free bodily fluid sample from a control subject not having said disease or condition; determining a fourth profile of analytes from =2n phagocytic cells from the control subject not having said disease or condition; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises: d) obtaining a fifth profile of analytes from cells or tissues affected by said disease or condition from the subject having said disease or condition; obtaining a sixth profile of analytes from cells or tissues not affected by said disease or condition from the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

An exemplary method for detecting the presence or absence of an analyte (e.g., DNA, RNA, protein, polypeptide, carbohydrate, lipid or the like) corresponding to a marker of the invention in a biological sample involves obtaining a bodily fluid sample (e.g., blood) from a test subject and contacting the bodily fluid sample with a compound or an agent capable of detecting one or more markers. Detection methods described herein can be used to detect one or more markers in a biological sample in vitro as well as in vivo. For example, in vitro techniques for detection of mRNA include Northern hybridizations and in situ hybridizations. In vitro techniques for detection of a polypeptide corresponding to a marker of the invention include enzyme linked immunosorbent assays (ELISAs), Western blots, immunoprecipitations and immunofluorescence. In vitro techniques for detection of genomic DNA include Southern hybridizations. Furthermore, in vivo techniques for detection of a polypeptide corresponding to a marker of the invention include introducing into a subject a labeled antibody directed against the polypeptide. For example, the antibody can be labeled with a radioactive marker whose presence and location in a subject can be detected by standard imaging techniques. Because each marker is also an analyte, any method described herein to detect the presence or absence of a marker can also be used to detect the presence or absence of an analyte.

The marker that is useful in the methods of the invention can include any mutation in any one of the above-identified markers. Mutation sites and sequences can be identified, for example, by databases or repositories of such information, e.g., The Human Gene Mutation Database (www.hgmd.cf.ac.uk), the Single Nucleotide Polymorphism Database (dbSNP, www.ncbi.nlm.nih.gov/projects/SNP), and the Online Mendelian Inheritance in Man (OMIM) website (www.ncbi.nlm.nih.gov/omim).

The marker that is useful in the methods of the invention can include any marker that is known to be associated with a disease or condition.

According to certain embodiments, white blood cell (WBC) subpopulations (isolated for example from blood, urine, and saliva) such as non-phagocytic WBCs, phagocytic WBCs that have not phagocytosed/internalized live, dying, or dead prokaryotic and eukaryotic cells and fragments thereof, and WBCs with a DNA content of 2n (i.e., DNA Index = 1) are useful for reporting and excluding the intrinsic genomic, proteomic, metabolomic, glycomic, glycoproteomic, lipidomic, and/or lipoproteomic profile(s) of the individual being evaluated. Such identification of patient-specific signatures that are unrelated to the disease, pathology, and/or condition being diagnosed enables the identification and detection of tumor-/other disease-/condition-specific signatures within the cell-free bodily fluids (such as whole blood, plasma, serum, urine, saliva, cerebrospinal fluid, amniotic fluid, intraocular fluid, nasal fluid, lung lavage fluid, peritoneal fluid, stool, lymph and the like) of an individual suspected of having cancer or other diseases or disorders or conditions. Therefore, the comparison of profiles of disease or condition specific markers - present in cell-free bodily fluids (e.g., whole blood, serum, plasma, urine, saliva, cerebrospinal fluid, amniotic fluid, intraocular fluid, nasal fluid, lung lavage fluid, peritoneal fluid, stool, lymph) - with those profiles in any non-phagocytic WBC or cells that can be obtained noninvasively (e.g., scrapped from the cheek pouch) or any phagocytic and/or non-phagocytic WBCs or cells that can be obtained noninvasively (e.g., scrapped from the cheek pouch) with a DNA Index of 1 will lead to the identification of patient-specific and tumor specific, disease specific or condition specific signatures that are not expressed, under-expressed in the non-phagocytic cell or in any WBCs and/or other bodily cell whose DNA content equals 2 (i.e., with a DNA Index = 1), or expressed in the cells not as a consequence of the disease or condition being diagnosed or detected, i.e., are related to the intrinsic genomic, proteomic, and epigenetic profiles of the individual. Likewise, protein expression profiles of cell-free bodily fluids and non-phagocytic WBC (DNA content of 2n), any WBCs whose DNA content equals 2 (i.e., with a DNA Index = 1), or any other mammalian cell that can be obtained noninvasively with a DNA content of 2n, will lead to the identification and detection of tumor specific, disease specific, or condition specific protein signatures within the fluids that are not expressed, under-expressed in the non-phagocytic cell or any WBCs and/or other bodily cell whose DNA content equals 2 (i.e., with a DNA Index = 1), or expressed in the cells not as a consequence of the disease or condition being diagnosed or detected, i.e., are related to the intrinsic genomic, proteomic, and epigenetic profiles of the individual. Furthermore, lipid profiles of cell-free bodily fluids and non-phagocytic WBC (DNA content of 2n), any WBCs whose DNA content equals 2 (i.e., with a DNA Index = 1), or any other mammalian cell that can be obtained noninvasively with a DNA content of 2n, will lead to the identification and detection of tumor specific, disease specific, or condition specific lipid signatures within the fluids that are not expressed, under-expressed in the non-phagocytic cell or any WBCs and/or other bodily cell whose DNA content equals 2 (i.e., with a DNA Index = 1), or expressed in the cells not as a consequence of the disease or condition being diagnosed or detected, i.e., are related to the intrinsic genomic, proteomic, and epigenetic profiles of the individual.

The marker that is useful in the methods of the invention can include any marker that is known to be associated with a disease or condition. Markers that can be used in this invention can be any marker that has been well-characterized as associated with a specific disease or condition, or any markers that have beeN identified by the methods of this invention.

In some embodiments, the markers comprise at least one gene selected from the group consisting of AKT2, BAK1, EGFR, ERBB2, ETS2, FOS, JUN, MAP2K1, MMP2, PDGFB, RB1, SERPINB2, SNCG, and SPP1. In some embodiments, the one or more markers comprise at least one gene selected from the group consisting of AKT1, AKT2, BAK2, CDC25A, E2F1, EGFR, ERBB2, FOS, JUN, MAP2K1, MMP2, NFKB1, PDGFB, PIK3R1, PNN, RB1, SERPINB2, SERPINB5, SNCG, SPP1, TERT, TIMP3, and TP53. In some embodiments, the one or more markers comprise at least one gene selected from the group consisting of CASP8, CASP9, COL18A1, ETS2, HTATIP2, MMP9, SRC, and TWIST1. In some embodiments, the one or more markers comprise at least one gene selected from the group consisting of AKT1, APAF1, ATM, CDC25A, CDKN1A, ETS2, FOS, IL8, ITGA4, ITGA6, ITGAV, JUN, MAP2K1, NFKBIA, PLAU, PLAUR, RAF1, SERPINB2, SYK, TIMP1, TNF, TNFRSF10B, and TNFRSF1A. In some embodiments, the markers comprise at least one gene selected from the group consisting of ACP2, AK2, AKT3, ARL5B, ATP2B3, BGN, BRAF, BTG2, CAMKK2, CAPG, CAPN12, CPLX2, DENNDSA, DNA2, FAM104A, FNIP1, GFRA4, GLUD1, GNAQ, GP1BB, HNRPLL, HOXA2, HPS3, INPP4A, ITGAV, KLHL23, LANCL2, LYPD6, MAPKAPK3, MEF2A (includes, EG:4205), MEF2C, NVL, PCYT1A, PGLYRP4, PLOD1, PPP1CB, PRKAB2, PROS1, PTPRE, RASA4 (includes,EG:10156), RBMS2, RBPJ, STAT5B, THBS1, TRIB1, TRIM2, TSPAN6, and ZDHHC21. In some embodiments, the markers comprise at least one gene selected from the group consisting of B4GALT5, BOP1, CCL2, CCL3, CCL3L1, CCRL2, CD83, CLEC4G, CLIC4, CTSC, CTSO, CXCL10, FCGR3A, FPR3, HBA1, HBB, LRMP, MAP1LC3B2, MS4A4A, MSR1, MYADML, NID1, PF4, PION, RNF217, SAMD9L, SERPING1, and SPARC. In some embodiments, the markers comprise at least one gene selected from the group consisting of ACOT9, AMPD2, ARHGAP15, BATF2, C3AR1, C5orf41, CCL3, CCL3L1, CD63, CHST11, CHSY1, CLEC4G, CTSZ, CXorf21, CYTH4, CYTIP, DLEU2, DNAJA1, DOCK8, DTX3L, DUSP6, EPSTI1, ERF, F2RL1, FYB, GABRB2, GBP5, GLRX, GNB4, ICAM1, IFI35, IFIH1, IFNAR2, IL1R1, IRF1, ITGA5, LAP3, LAPTM5, LCP2, MAP1LC3B, MAP1LC3B2, MICAL2, MT1DP, MT1JP, MT1M, MT2A, MYADML, NEK6, NINJ2, NNMT, NT5C3L, NUB1, PDE4B, PLOD1, PML, PRKCB, PSMB9, RCN3, RGS4, RNASE6, RTP4, SAMD9L, SEL1L, SERPING1, SETX, SIGLEC10, SKIL, SLC7A7, SNORA21, SP100, SP110, SP140, SSFA2, STAT2, STK17B, STK3, TDRD7, TMCC1, TMPRSS11E2, TNFRSF1B, TPM1, TRIM21,TXNDC4, UBE2L6, UBE2W, USP18, VAV1, WARS, WIPF1, and WIPI1. In some embodiments, the markers comprise at least one gene selected from the group consisting of ADAR, ADM, ALAS1, ANKRD22, ARHGAP27, B3GNT5, BCL10, C12orf35, C15orf29, C2orf59, CD177, CEACAM1, CPEB2, DDX58, F2RL1, GDPD3, GNAI3, HIST2H3A, HIST2H3D, HIST2H4A, HMGCR, HSPA6, HSPC159, IL4R, IMPA2, KPNB1, KREMEN1, KRT23, LDLR, LOC100130904, LTB4R, MAEA, MARK2, MBOAT2, MPZL3, N4BP1, NBEAL2, NMI, NPEPPS, PARP14, PGM2, PPIF, PXN, RALBP1, ROD1, RPS6KA1, S100P, SERTAD2, SLC9A1, SLPI, SP110, SPINT1, ST14, TBC1D3, TNFRSF9, TRIM21, UPP1, VPS24, ZBTB34, and ZNF256.

In some embodiments, the marker that is useful in the methods of the invention for prenatal or pregnancy-related diseases or conditions include those disclosed in, for example, United States Patents 7,655,399, 7,651,838, 6,660,477, 6,172,198, 5,594,637, 5,514,598, 6,258,540, 6,664,056, 7,235,359, and 7,645,576, United States Patent Application Publications 20090162842, 20090155776, 20070207466, 20060019278, 20040086864, 20020045176, 20010051341, 20020192642, 20040009518, 20040203037, 20050282185, 20060252071, 20070275402, 20080153090, 20090170102, 20090061425, 20020045176, 20040137452, 20050164241, 20060019278, 20060252068, 20060252071, 20060257901, 20070141625, 20070218469, 20070275402, 20090155776, 20090162842, 20090170102, 20090317797, 20100120056, 20100120076, and 20100137263 and International Patent Application Publications WO/2006/026020, WO/2002/068685, WO/2005/111626, WO/2009/055487, WO/2009/001392, and WO/2008/014516.

In some embodiments, the marker that is useful in the methods of the invention for neurological or neuropsychiatric diseases or conditions include those disclosed in, for example in United States Patents 7,723,117, 6,867,236, United States Patent Application Publications 20060115854, 20060115855, 20060166283, 20060234301, 20060259990, 20060259991, 20070162983, 20070264197, 20080026405, 20080038730, 20080051334, 20080152589, 20080220013, 20080261226, 20080269103, 20080286263, 20090041862, 20090239241, 20090275046, 20090318354, 20090324611, 20100009352, 20100021929, 20100028356, 20100055722, 20100062463, 20100075891, 20100105623, 20100124756, 20100159486, 20100167937, 20100169988, 20100167320, 20100112587, 20100098705, 20100068705, 20100009356, 20090305265, 20100124746, 20100092983, 20070148661, 20070141625, 20100120050, 20090155230, 20090274709, International Patent Application Publications WO/2004/040016, WO/2004/071269, WO/2005/033341, WO/2005/052592, WO/2005/103712, WO/2005/114222, WO/2006/020269, WO/2006/048778, WO/2006/050475, WO/2006/061609, WO/2006/105907, WO/2006/133423, WO/2006/134390, WO/2007/098585, WO/2007/119179, WO/2008/010660, WO/2008/014314, WO/2008/028257, WO/2008/046509, WO/2008/046510, WO/2008/046511, WO/2008/046512, WO/2008/063369, WO/2008/085035, WO/2008/095261, WO/2008/100596, WO/2008/120684, WO/2008/125651, WO/2008/127317, WO/2008/132464, WO/2009/000520, WO/2009/001392, WO/2009/068591, WO/2009/074331, WO/2009/100131, WO/2010/005750, WO/2010/011506, WO/2010/019553, WO/2010/059242, WO/2010/061283, WO/2010/063009, WO/2010/066000, WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/07556.

In some embodiments, the marker that is useful in the methods of the invention for cardiovascular diseases or conditions include those disclosed in, for example in United States Patents 7,670,769, 7,445,886, 7,432,107, 7,157,235, and 7,009,038, United States Patent Application Publications 20100167320, 20100112587, 20100098705, 20100068705, 20100009356, 20090305265, 20100124746, 20100092983, 20070148661, 20070141625, 20100120050, 20090155230, and 20090274709, and International Patent Application Publications WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/075566.

In some embodiments, the marker that is useful in the methods of the invention for kidney-associated diseases or conditions include those disclosed in, for example in United States Patents 7,488,584, 7,459,280, 7,294,465, and 7,662,578, United States Patent Application Publications 20100143951, 20100124746, 20100120056, 20100120041, 20100081142, 20090155230, and 20090239242, International Patent Application Publications WO/2010/059996, WO/2010/054389, WO/2010/048347, WO/2010/048497, WO/2010/054167, WO/2010/048346, WO/2010/046137, WO/2010/025434, WO/2010/018185, WO/2010/012306, WO/2009/122387, WO/2009/083950, WO/2009/080780, WO/2009/060035, WO/2009/059259, WO/2008/154238, WO/2008/089936, WO/2008/084331, WO/2008/042012, WO/2007/131345, WO/2005/012907, WO/2004/024098, WO/2003/019193, WO/2007/112999, WO/2007/082733, WO/2006/073941, WO/2010/068686, WO/2010/022210, and WO/2009/127644.

In some embodiments, the marker that is useful in the methods of the invention for autoimmune or immune-related diseases or conditions include those disclosed in, for example 7,604,948, 7,670,764, 6,986,995, and 6,631,330, United States Patent Application Publication 20070141625, 20090263474, 20100075891, 20100104579, 20100105086, 20100131286, 20090176217, 20090202469, 20020119118, 20090258025, 20100137393, 20100120629, 20090318392, 20090196927, 20090023166, 20080227709, 20080039402, 20080026378, 20070224638, 20070218519, 20060210562, 20050266432, 20050164233, 20050130245, 20090130683, 20090110667, 20090054321, 20090023166, and 20080274118, and International Patent Application Publication WO/2009/043848, WO/2010/053587, WO/2010/046503, WO/2010/039714, WO/2009/100342, WO/2009/053537, WO/2009/017444, WO/2008/156867, WO/2008/147938, WO/2008/129296, WO/2008/137835, WO/2008/082519, WO/2008/064336, WO/2008/043782, WO/2008/043725, WO/2007/047907, WO/2006/125117, WO/2006/114661, WO/2006/020899, WO/2005/114222, WO/2005/007836, WO/2004/076639, WO/2004/050704, and WO/2001/014881.

The present invention also provides kits that comprise marker detection agents that detect at least one or more of the markers identified by the methods of this invention. This present invention also provides methods of treating or preventing a disease or condition in a subject comprising administering to said subject an agent that modulates the activity or expression of at least one or more of the markers identified by the methods of this invention.

It is to be understood that the embodiments of the present invention which have been described are merely illustrative of some of the applications of the principles of the present invention. Numerous modifications may be made by those skilled in the art based upon the teachings presented herein without departing from the true spirit and scope of the invention.

The following examples are set forth as being representative of the present invention. These examples are not to be construed as limiting the scope of the invention as these and other equivalent embodiments will be apparent in view of the present disclosure, figures, and accompanying claims.

### EXAMPLE 1

### Representative Method I for the Separation of Phagocytic Cells with DNA Content of 2n from Non-Phagocytic Cells and the Analysis of Expression Profiles

1. Separate blood sample into plasma and buffy coat including WBC sample. Coat plates to receive WBC sample with avidin.
2. Add biotinylated antibody to non-phagocytic blood cell (e.g., T cells) to the wells, incubate for 30 min at RT, wash wells.
3. Add magnetic beads.
4. Add WBC blood sample.
5. Incubate at 37 °C (30 minutes - 1 hour).
6. Following phagocytosis of beads by phagocytic cells and binding of avidin-biotin-antibody to non-phagocytic cells, place plate on top of magnet and wash (the phagocytic cells that internalized the magnetic beads and the non-phagocytic cells bound to the antibody will stay; all other cells will be washed away).
7. Remove magnet and collect phagocytic cells and separate into phagocytic cells with DNA equal to 2n and DNA greater than 2n. Non-phagocytes and phagocytes having DNA equal to 2n are referred to as cells having DNA equal to 2n.
8. Isolate RNA from plasma, Isolate RNA from phagocytic cells with DNA equal to 2n and of non-phagocytic cells, prepare cDNA, cRNA and use to differentiate genetic profile(s) (e.g., whole gene arrays and/or cancer gene arrays) of plasma versus genetic profile(s) of phagocytic and/or non-phagocytic cells.
9. Isolate DNA from plasma and cells having DNA equal to 2n and identify tumor-DNA signatures selectively present in plasma (i.e., absent in cells having DNA of 2n such as non-phagocytes); compare the profiles (e.g., whole gene arrays, DNA mutations and/or SNPs obtained in phagocytic and non-phagocytic cells).
10. Isolate protein from plasma and cells having DNA equal to 2n, run Western blots using antibodies to known proteins overexpressed by human tumors (e.g., PSA and PSMA in prostate cancer; CEA in colon cancer; and CA125 in ovarian cancer), and compare the profiles obtained in plasma and cells having DNA equal to 2n. Alternatively, use mass spectroscopy to identify the proteins.
11. Isolate lipids from plasma and cells having DNA equal to 2n and compare quantity and quality, for example using HPLC.

### EXAMPLE 2

### Representative Method II for the Separation of Phagocytic Cells from Non-Phagocytic Cells and the Analysis of Expression Profiles

1. Separate blood sample into plasma and buffy coat including WBC sample.
2. Cytospin WBC on glass slides.
3. Fix cells in acetone/methanol (-20 °C for 5 minutes).
4. Stain with hematoxylin and eosin stain and anti-T cell antibody.
5. Isolate T cells (non-phagocytic) and macrophages (phagocytic) using laser capture microscopy (LCM). Separate into phagocytic cells with DNA equal to 2n and DNA greater than 2n. Non-phagocytes and phagocytes having DNA equal to 2n are referred to as cells having DNA equal to 2n.
6. Isolate RNA from plasma, Isolate RNA from phagocytic cells with DNA equal to 2n and of non-phagocytic cells, prepare cDNA, cRNA and use to differentiate genetic profile(s) (e.g., whole gene arrays and/or cancer gene arrays) of plasma versus genetic profile(s) of phagocytic and/or non-phagocytic cells.
7. Isolate DNA from plasma and cells having DNA equal to 2n and identify tumor-DNA signatures selectively present in plasma (i.e., absent in cells having DNA of 2n such as non-phagocytes); compare the profiles (e.g., whole gene arrays, DNA mutations and/or SNPs obtained in phagocytic and non-phagocytic cells).
8. Isolate protein from plasma and cells having DNA equal to 2n, run Western blots using antibodies to known proteins overexpressed by human tumors (e.g., PSA and PSMA in prostate cancer; CEA in colon cancer; and CA125 in ovarian cancer), and compare the profiles obtained in plasma and cells having DNA equal to 2n. Alternatively, use mass spectroscopy to identify the proteins.
9. Isolate lipids from plasma and cells having DNA equal to 2n and compare quantity and quality, for example using HPLC.

### EXAMPLE 3

### Representative Method III for the Separation of Phagocytic Cells from Non-Phagocytic Cells and the Analysis of Expression Profiles

1. Separate plasma from whole blood.
2. Use magnetic antibody-conjugated beads to isolate non-phagocytic (e.g., T cells) and phagocytic cells (e.g., neutrophils and/or macrophages and/or monocytes) from whole blood. Separate into phagocytic cells with DNA equal to 2n and DNA greater than 2n. Non-phagocytes and phagocytes having DNA equal to 2n are referred to as cells having DNA equal to 2n.
3. Isolate RNA from plasma, Isolate RNA from phagocytic cells with DNA equal to 2n and of non-phagocytic cells, prepare cDNA, cRNA and use to differentiate genetic profile(s) (e.g., whole gene arrays and/or cancer gene arrays) of plasma versus genetic profile(s) of phagocytic and/or non-phagocytic cells.
4. Isolate DNA from plasma and cells having DNA equal to 2n and identify tumor-DNA signatures selectively present in plasma (i.e., absent in cells having DNA of 2n such as non-phagocytes); compare the profiles (e.g., whole gene arrays, DNA mutations and/or SNPs obtained in phagocytic and non-phagocytic cells).
5. Isolate protein from plasma and cells having DNA equal to 2n, run Western blots using antibodies to known proteins overexpressed by human tumors (e.g., PSA and PSMA in prostate cancer; CEA in colon cancer; and CA125 in ovarian cancer), and compare the profiles obtained in plasma and cells having DNA equal to 2n. Alternatively, use mass spectroscopy to identify the proteins.
6. Isolate lipids from plasma and cells having DNA equal to 2n and compare quantity and quality, for example using HPLC.

### EXAMPLE 4

### Representative Method IV for the Separation of Phagocytic Cells from Non-Phagocytic Cells and the Analysis of Expression Profiles

1. Separate blood sample into plasma and buffy coat including WBC sample. Stain WBC with fluorescent antibodies specific against a particular cell subpopulation (e.g., neutrophils, macrophages, monocytes, T cells and the like) and a DNA stain, (e.g., Hoechst 33342, Propidium iodide).
2. Sort the cells (e.g., by FACS).
3. Isolate RNA from plasma, Isolate RNA from phagocytic cells with DNA equal to 2n and of non-phagocytic cells, preparecDNA, cRNA and use to differentiate genetic profile(s) (e.g., whole gene arrays and/or cancer gene arrays) of plasma versus genetic profile(s) of phagocytic and/or non-phagocytic cells.
4. Isolate DNA from plasma and cells having DNA equal to 2n and identify tumor-DNA signatures selectively present in plasma (i.e., absent in cells having DNA of 2n such as non-phagocytes); compare the profiles (e.g., whole gene arrays, DNA mutations and/or SNPs obtained in phagocytic and non-phagocytic cells).
5. Isolate protein from plasma and cells having DNA equal to 2n, run Western blots using antibodies to known proteins overexpressed by human tumors (e.g., PSA and PSMA in prostate cancer; CEA in colon cancer; and CA125 in ovarian cancer), and compare the profiles obtained in plasma and cells having DNA equal to 2n. Alternatively, use mass spectroscopy to identify the proteins.
6. Isolate lipids from plasma and cells having DNA equal to 2n and compare quantity and quality, for example using HPLC.

### EXAMPLE 5

### Detection of Tumor-Specific Gene Signatures in Cell Free Bodily Fluids Obtained from Tumor-Bearing Mice

According to embodiments of the present invention, methods are provided to differentiate between "normal non-specific noise" and "tumor-specific" and/or "disease-specific" and/or "condition-specific" signatures in cell free bodily fluids. The gene-expression profiles of cell free bodily fluids from tumor-bearing mice are compared with that of non-phagocytic T cells from the same donor mice to identify tumor-specific signatures within the cell free bodily fluids that are either not expressed or significantly differentially expressed in non-phagocytic cells from the same tumor-bearing mice and from non-tumor-bearing animals.

### Human Prostate LNCaP Cancer Cells

Athymic nude mice (*n* = 5) are injected subcutaneously (s.c.) with human prostate LNCaP cancer cells. Twenty-seven days later (tumor size = ~0.4 cm), the mice are bled by cardiac puncture (~1 mL/mouse) into EDTA-containing tubes that are then centrifuged. The plasma is isolated. The buffy coat is isolated and washed, and neutrophils, macrophages, and T cells are separated using, respectively, anti-mouse neutrophil-, macrophage-, and T cell-immunomagnetic DynaBeads. RNA is isolated from T cells (Triazol^{®}). The RNA quality is determined. cDNA and biotinylated cRNA (cRNA-B) is prepared. Biotinylated cRNA (cRNA-B) is prepared from the plasma. The cRNA-B sample from the plasma and from the T cells are separately incubated with cancer-gene human microarrays (Oligo GEArray^{®} Human Cancer PathwayFinder Microarray - OHS-033 - SuperArray Bioscience). Following hybridization, the membranes are washed and stained with avidin-alkaline phosphatase, and the genes are detected using chemiluminescence (X-ray film). The genetic signatures are compared between that obtained from the T cells and the plasma. The genetic signature from the T cells is subtracted from the genetic signature from the plasma to provide a patient specific signature one or more markers associated with prostate cancer.
Human LS174T Colon Adenocarcinoma Tumors,
LLC1 Carcinoma Cells, B16F10 Mouse Melanoma Cells

Similar experiments are carried out with plasma and cells isolated from athymic nude mice (*n* = 5) injected s.c. with human LS174T colon adenocarcinoma tumors (tumor size = ~0.3 cm), C57Bl mice (*n* = 5) injected s.c. with Lewis lung mouse LLC1 carcinoma cells (tumor size = ~0.6 cm), and C57Bl mice (*n* = 5) injected intravenously with 10⁶ B16F10 mouse melanoma cells (when the tumor cells are of mouse origin, the cRNA-B samples from plasma and from T cells are hybridized with the Oligo GEArray^{®} Mouse Cancer PathwayFinder Microarray - OMM-033 - SuperArray Bioscience). RNA is also isolated from exponentially growing LS174T, LLC1, B16F10, and LNCaP cells in culture and from plasma and T cells isolated from non-tumor-bearing C57Bl and nude mice, and their cancer-related gene profiles are determined.

According to aspects of the present invention, plasma obtained from mice injected with human prostate or colon tumor cells and from mice bearing mouse lung cancer or melanoma will have various cancer-related gene signatures that can also be found in their respective tumor cells. According to further aspects, cancer-related genes are not expressed or are minimally expressed by (i) non-phagocytic T cells isolated from tumor-bearing mice; and (ii) phagocytic neutrophils and macrophages obtained from non-tumor-bearing mice.

### EXAMPLE 6

### Detection of Tumor-Specific Gene Signatures in Cell Free Bodily Fluid Obtained from Cancer Patients

According to certain embodiments of the present invention, the gene-expression profiles of cell free bodily fluids from cancer patients were compared with that of non-phagocytic T cells from the same donor individuals to identify tumor-specific signatures within the cell free bodily fluids that were either not expressed or significantly differentially expressed in non-phagocytic cells.

### Patients with Head and Neck Tumors

Ten milliliters of venous blood is obtained (into an EDTA-containing tube) from patients known to have squamous cell carcinoma of the neck. Following centrifugation at 2,000 rpm for 5 minutes at room temperature, the plasma is retained and the buffy coat is transferred to a tube and washed with PBS.

The cells are separated employing T cell-, neutrophil-, and macrophage/monocyte-rat anti-human immunomagnetic DynaBeads^{®} from INVITROGEN^{™}, Carlsbad, CA. In essence, the beads are added consecutively to the WBC sample and following individual 4 °C, 30 minute incubations, the T cells-, neutrophils-, and macrophages/monocytes-bound beads are isolated using a magnet and washed with PBS three times.

RNA is then isolated from T cells (using TRIZOL^{®}, INVITROGEN^{™}, Carlsbad, CA). The RNA quantity and quality is determined and cDNA and biotinylated cRNA (cRNA-B) is prepared. Biotinylated cRNA (cRNA-B) is prepared from the plasma. The cRNA-B samples from the plasma and the T cells are each incubated (60°C overnight) with cancer-gene human microarrays (Oligo GEArray^{®} Human Cancer PathwayFinder Microarray - OHS-033-SuperArray Bioscience, Frederick, MD). Following hybridization, the membranes are washed and stained with avidin-alkaline phosphatase, and the genes are detected using chemiluminescence (X-ray film). The genetic signatures are compared between that obtained from the T cells and the plasma. The genetic signature from the T cells is subtracted from the genetic signature from the plasma to provide a patient specific signature one or more markers associated with head and neck cancer.

According to aspects of the present invention, plasma obtained from head and neck cancer patients will have various cancer-related gene signatures that are also found in their respective tumor cells. The following genes can be identified: E26 viral oncogene homolog (ETS2), HIV-1 Tat interactive protein (HTAT1P2), IL8 (neutrophil activation and chemotaxis), Jun oncogene (JUN), and matrix metalloproteinase 9 (MMP9).

According to an additional aspect, these cancer-related genes are not expressed or are minimally expressed by non-phagocytic T cells.

### Ovarian Cancer Patients

Similar experiments are carried out with plasma and cells isolated from a patient with ovarian cancer. Plasma can include many cancer-related genes that are not expressed or are minimally expressed by non-phagocytic T cells. Such cancer genes can include one or more of AKT1, APAF1, ATM, CDC25A, CDKN1A, ETS2, FOS, IL8, ITGA4, ITGA6, ITGAV, JLTN, MAP2K1, NFKBIA, PLAU, PLAUR, RAF1, SERPINB2, SYK, TIMP1, TNF, TNFRSF10B, or TNFRSF1A.

### EXAMPLE 7

### Detection of Tumor-Specific Protein Signatures in Cell Free Bodily Fluids Obtained from Mice Bearing Human Prostate LNCaP Tumors and Human Colon LS174T Tumors

A protein purification kit (Norgen, Incorporated, Product # 23500) is used to isolate and purify proteins from mouse WBCs, T cells, and macrophages. The assay is very simple and fast (approximately 30 minutes) and the isolated proteins, can be used in a number of downstream applications, such as SDS-PAGE analysis and Western blots.

Protein samples are isolated from plasma and non-phagocytic (T-lymphocytes) cells obtained from mice bearing LNCaP and LS174T tumors since the former cell line expresses PSA (Denmeade et al. (2001) Prostate 48:1; Lin et al. (2001) J. Urol. 166:1943) and the latter exhibits a tumor-specific glycoprotein (TAG-72), a high molecular weight mucin (Colcher et al. (1981) Proc. Natl. Acad. Sci. USA 78:3199); Colcher et al. (1984) Cancer Res. 44:5744; Kassis et al. (1996) J. Nucl. Med. 37:343. Western blot analysis is carried out with 16 µg of the purified protein samples from each of the plasma and T cells. In essence, each sample is mixed with two volumes of SDS loading buffer and run on 10% SDS-PAGE along with unstained precision plus protein standards (Biorad) in Tris-glycine-SDS buffer (pH 8.4) at 200 volts. The proteins are transferred to a nitrocellulose membrane (overnight at 4°C) using a Mini Trans-Blot (Biorad) apparatus and a transfer buffer containing 25 mM Tris, pH 8.4, 192 mM glycine, and 20% methanol. The membrane is blocked with 5% nonfat dry milk (60 min at room temperature (RT)) and incubated (1 hour, RT) with either B72.3, a mouse monoclonal antibody against human TAG-72, or ER-PR8, a mouse monoclonal antibody against human PSA. The blots are washed and then incubated with Immun-Star Goat Anti Mouse-HRP conjugate (Biorad), a secondary antibody specific to mouse IgG, and developed by incubation (5 min, RT) with a 1:1 mixture of luminol solution and peroxide buffer (Biorad), followed by autoradiography.

According to one aspect of the present invention, plasma from LNCaP tumor-bearing mice can be positive for PSA, whereas this protein is not detected in non-phagocytic T cells from the same animals. Similarly, TAG-72 is expressed by monocytes/macrophages obtained from LS174T tumor-bearing mice and is not detected in T cells from the same animals.

### EXAMPLE 8

### Profiling Experiments

### Isolation of Blood Phagocytic Cells

A sample of blood is obtained from a patient. The blood (~5 mL) will be transferred to a 50-mL tube containing 50 µL0.5 M EDTA (final EDTA concentration = ~4.8 mM). The tube will be vortexed gently and 25 mL RBC Lysis Buffer (Norgen, Incorporated) will be added. The tube will be vortexed gently again, incubated at room temperature until the color of the solution changes to bright red (3-5 min), and centrifuged at 2,000 rpm for 3 min. Following careful aspiration of the supernatant, the WBCs will be washed with 40 mL Ca/Mg-free 0.1 M PBS (containing 2% FBS, 2 mM EDTA, and 20 mM glucose), and the cells (106/mL) will then be incubated (30 min, 4 oC, in the dark) with a cell-staining solution containing (i) the DNA, viable cell-permeable stain Hoechst 33342 (4 µg/mL; Em = 483 nm), (ii) the anti-human monocytes/macrophages monoclonal antibody (Alexa Fluor^{®} 647-conjugate; Em =668 nm), which recognizes the human F4/80 antigen expressed by circulating monocytes/macrophages, and (iii) the anti-human neutrophil monoclonal antibody (RPE-conjugate; Em = 578 nm), which recognizes human circulating neutrophils. The cells will then be washed and sorted (BD FACSAria) into neutrophils (Nn=2), neutrophils (Nn>2), monocytes/macrophages (M/Mn=2), and monocytes/macrophages (M/Mn>2). According to aspects of the present invention, the content of cells having a DNA content of 2n are compared with the content of plasma obtained from the same individual.

### Gene Profiling

Human whole-genome gene profiling will be performed. For RNA samples obtained from plasma or human tumor cells or neutrophils (Nn=2, Nn>2) and monocytes/macrophages (M/Mn=2, M/Mn>2), the GeneChip^{®} Human Genome U133Plus 2.0 Array by Affymetrix, Incorporated will be used. This array analyzes the expression level of over 47,000 transcripts and variants, including 38,500 well-characterized human genes. In general, the extracted RNA will be used to determine the expression profiles of human genes using the above-mentioned array. To ensure array reproducibility, each sample will be profiled in triplicate and the experiment repeated once. The microarray data will be filtered for cancer-induction-related genes as described below and validated using quantitative real-time, reverse transcriptase, polymerase chain reaction (RT-PCR).

### Upregulation/Downregulation of Cancer-Induction-Related Genes

RNA will be isolated using Triazol (Invitrogen, Incorporated) and purified using the cartridges provided in the kit. The RNA quality and quantity will be assessed with the Bioanalyzer 2100 (Agilent Technologies, Incorporated, Palo Alto, CA) and Degradometer software version 1.41 (Worldwide Web: dnaarrays.org). These experimental results will help in distinguishing the molecular pathways perturbed consequent to the presence of tumors.

### Analysis of Microarray Experiments

The analysis of the large scale/high throughput molecular expression data generated will rely heavily on the ability to (i) identify genes differentially expressed in plasma, (ii) annotate the identified genes, and (iii) assign the annotated genes to those specifically expressed by a specific tumor. Statistical analysis of the microarray data can be done, for example, using the dChip package which easily accommodates this type of gene list construction in its "Analysis/Compare Samples" menu. When using Affymetrix GeneChips, one or more Gene Chips and associated methods will be applied to ascertain the quality of the raw microarray data (Gautier et al. (2004) Bioinformatics 20:307). Furthermore, various background correction and normalization procedures will be utilized to arrive at an optimal protocol for normalization and summarization of the probe sets (to produce expression values) (Huber et al. (2002) Bioinformatics 18(Suppl. 1):S96; Wu et al. (2004) Journal of the American Statistical Association 99:909; Seo and Hoffman (2006) BioMed Central Bioinformatics 7:395). In a two-step filtration approach, we will compare the gene profiles of Pn=2 to those of Pn>2 and construct a list of expressed genes and then compare these genes to the tumor-specific genes identified for each tumor cell line - post filtration of Pn=2 gene profile. For example, (i) blood will be obtained from breast cancer patients and separated into plasma and buffy coat from which T cells are isolated; (ii) the gene profiles will be determined in triplicate for each of the plasma and the T cells; (iii) the mean (from the 3 samples) of each identified gene and its respective standard error (SE) will be calculated for each group (Nn>2 and Nn=2); (iv) the gene expression profiles of the two groups will then be compared and a list (L-1) of expressed genes identified on the basis of an absolute ≥2-fold log change (Nn>2/Nn=2), according to the Welch modified two-sample t-test; (v) the gene expression profiles of Nn=2 and that of breast cancer (obtained from tumor and normal breast tissue biopsies) will be compared and a list (L-2) of expressed genes identified; and (vi) breast-cancer-specific gene signatures that have been acquired/expressed by Nn>2 will be identified by comparing the genes in L-1and L-2 ("Analysis/Compare Samples/Combine Comparisons," dChip) and filtering common genes.

### Protein Profiling

Fifty to one hundred micrograms of the total protein from each of the plasma and the T cells will be denatured and reduced with tris-(2-carboxyethyl)phosphinetrypsin (1 mM) and 0.02% sodium dodecyl sulfate at 60 °C for 1 hour. Cysteines are subsequently blocked and total protein is digested with trypsin at 37 °C for 12-16 hours. The resulting peptides will be iTRAQ-labeled (with tags 113-119 and 121) for 1 hour (4-plex or 8-plex depending on the number of cell types to be compared). Following labeling, the separately tagged samples are combined and injected into an Agilent 1200 Series HPLC system equipped with a strong cation exchange column (Applied Biosystems 4.6 x 100 Porous). The 96 collected fractions are then pooled into 14 fractions, and each fraction is injected into the LC Packings Ultimate HPLC System for a second round of fractionation under reverse-phase conditions (LC Packings 15 cm x 75 µm analytical column). The reverse-phase fractions are spotted directly onto the target plate using an LC Packings Probot and are analyzed with mass spectrometry (Applied Biosystems 4800 Plus Proteomics Analyzer). Following data acquisition, the spectra are processed using the ProteinPilot software package (Applied Biosystems MDS Sciex), and the individual proteins in each of the plasma and T cell samples with their relative expression levels are identified using the ProteinPilotTM software and the analysis and identification of cancer-associated proteomic signatures will be carried out.

## Claims

1. A method for diagnosing or aiding in the diagnosis of a disease or condition, or assessing the risk of developing the disease or condition, or prognosing or aiding in the prognosis of the disease or condition, in a subject comprising:
a) determining a first profile of one or more markers of the disease or condition from a cell-free bodily fluid sample;
b) determining a second profile of at least one of the one or more markers from a population of phagocytic cells having a DNA content of 2n (=2n phagocytic cells) or a population of non-phagocytic cells; and
c) identifying a difference between the first and second profiles of at least one or more of said markers, wherein the difference is indicative of the presence of said disease or condition, or the risk of developing said disease or condition, or the prognosis of said disease or condition, in the subject.

2. A method for assessing the efficacy of a treatment for a disease or condition, or monitoring the progression or regression of the disease or condition, or identifying a compound capable of ameliorating or treating the disease or condition, in a subject comprising:
a) determining a first profile of one or more markers of the disease or condition from a cell-free bodily fluid sample from the subject at a first time point; determining a second profile of at least one of the one or more markers from a population of =2n phagocytic cells or a population of non-phagocytic cells from the subject at the first time point; identifying a first difference between the first and second profiles of at least one or more of said markers;
b) determining a third profile of the one or , or at a second time point, or after administration of the compound, respectively more markers from a cell-free bodily fluid sample from the subject at a second time point; determining a fourth profile of at least one of the one or more markers from a population of =2n phagocytic cells or a population of non-phagocytic cells from the subject at the second time point; identifying a second difference between the third and fourth profiles of at least one or more of said markers; and
c) identifying a difference between the first difference and the second difference, wherein the identified difference is indicative of the efficacy of the treatment for said disease or condition, or the progression or regression of said disease or condition, or indicates that the compound is capable of ameliorating or treating said disease or condition, in the subject.
